(19)

Europäisches
Patentamt
European
Patent Office
Office européen
des brevets

(11) **EP 4 574 977 A1**

(12) **EUROPEAN PATENT APPLICATION**
published in accordance with Art. 153(4) EPC

(43) Date of publication:
**25.06.2025 Bulletin 2025/26**

(21) Application number: **23854935.6**

(22) Date of filing: **17.08.2023**

(51) International Patent Classification (IPC):
**C12N 15/113** (2010.01)      **C12Q 1/6883** (2018.01)
**G01N 33/53** (2006.01)

(52) Cooperative Patent Classification (CPC):
**C12N 15/113; C12Q 1/6883; G01N 33/53**

(86) International application number:
**PCT/JP2023/029768**

(87) International publication number:
**WO 2024/038901 (22.02.2024 Gazette 2024/08)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **17.08.2022 JP 2022130277**

(71) Applicants:
• **Tokyo Metropolitan Institute of Medical Science
Tokyo 156-8506 (JP)**
• **Kagoshima University
Kagoshima-shi, Kagoshima 890-8580 (JP)**
• **National Hospital Organization
Tokyo 152-8621 (JP)**
• **TOKYO MEDICAL UNIVERSITY
Tokyo
160-8402 (JP)**

(72) Inventors:
• **KOHARA, Michinori
Tokyo 156-8506 (JP)**
• **KOHARA, Kyoko
Kagoshima 890-8580 (JP)**
• **YATSUHASHI, Hiroshi
Nagasaki 856-8562 (JP)**
• **OCHIYA, Takahiro
Tokyo 160-8402 (JP)**

(74) Representative: **J A Kemp LLP
80 Turnmill Street
London EC1M 5QU (GB)**

Remarks:
The complete document including Reference Table(s) and the Sequence Listing(s) can be downloaded from the EPO website

(54) **LIVER DISEASE NONINVASIVE BIOMARKER AND METHOD FOR DETECTING LIVER DISEASE USING SAME**

(57)    The present invention provides: a biomarker for non-invasively or minimally invasively detecting liver disease with high accuracy; a kit, method and program using this biomarker for non-invasively or minimally invasively detecting liver disease with high accuracy; a data processing method for detecting liver disease with high accuracy using this liver disease non-invasive marker; and a data processing device for use in this data processing. More specifically, provided is a biomarker selected from the group consisting of SEQ ID NOs: 1 to 125. Moreover provided is a detection kit for liver disease which includes a nucleic acid capable of binding specifically to a specific miRNA. Further provided are: a method for assessing the presence or absence of liver disease or the risk or degree of progression thereof in a subject, and/or the degree of success of a procedure performed for treatment, the method including measuring a level of a biomarker in a biological sample from the subject, and comparing a measurement value of the biomarker with a reference value, where the biomarker is one or more of the biomarkers selected from the group consisting of SEQ ID NOs: 1 to 125; a program; a data processing method for detecting liver disease with high accuracy using this liver disease non-invasive marker; and a data processing device for use in this data processing.

**EP 4 574 977 A1**

**Description**

Technical Field

The present invention relates to a liver disease non-invasive biomarker. The present invention moreover relates to a kit, a method and a program for detecting liver disease with high accuracy using this liver disease non-invasive marker, as well as a data processing method for detecting liver disease with high accuracy using this liver disease non-invasive marker, and a data processing device for use in this data processing.

Background Art

The liver biopsy, as a method for analyzing the degree of improvement of fibrogenesis, is still currently widely used as a highly accurate method of evaluation. However, since hepatic cirrhosis patients have a tendency towards haemorrhaging, it is highly probable that a grave complication would be brought on by a liver biopsy. Moreover, since only one portion of liver tissue is sampled in a liver biopsy, there is always a problem, as a sampling error, as to whether the result of the liver biopsy reflects the state of the entire liver.

It is known that with a disease such as cancer etc., an abnormality occurs in the function of miRNA, which could become a cause of such disease. Although several markers which can non-invasively or minimally invasively evaluate liver disease are used at the present time, a highly accurate marker has not yet been established (refer e.g. to Non Patent Literature 1). Accordingly, the development of a non-invasive or minimally invasive surrogate marker which can diagnose correctly is still strongly desired. The practical application of such a highly accurate and non-invasive or minimally invasive surrogate marker would contribute not only to the improvement of diagnosis, but also to the improvement of the patient's quality of life (QOL).

Citation List

Non Patent Literature

Non Patent Literature 1: Serum Wisteria floribunda agglutinin-positive Mac-2-binding protein for patients with chronic hepatitis B and C: a comparative study, H Nishikawa, et. al., J Viral Hepat. 2016 Dec; 23(12): 977-984. doi: 10.1111/jvh.12575. Epub 2016 Jul 31.

Summary of Invention

Technical Problem

The present invention provides a biomarker for non-invasively or minimally invasively detecting liver disease with high accuracy. The present invention moreover provides a kit, method and program using this biomarker for non-invasively or minimally invasively detecting liver disease with high accuracy, as well as a data processing method for detecting liver disease with high accuracy using this liver disease non-invasive marker, and a data processing device for use in this data processing.

Solution to Problem

As a result of keen investigation on the aforementioned problem to be solved, the present inventors found out that multiple miRNAs from blood, which can be sampled with low invasion, can be used as a detection marker of liver disease; they also found out that liver disease can be significantly detected by using a nucleic acid capable of binding specifically to these miRNAs, and hence they attained the perfection of the present invention.

The present invention includes the aspects below.

<1-1> A detection kit for liver disease, which includes a nucleic acid capable of binding specifically to one or more polynucleotides, which are biomarkers, selected from the group consisting of miR-4454 (SEQ ID NO: 1), miR-3138 (SEQ ID NO: 2), miR-3679 (SEQ ID NO: 3), miR-4521 (SEQ ID NO: 4), miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21), miR-320b-2 (SEQ ID NO: 22), miR-451a (SEQ ID NO: 23), miR-548d-3p (SEQ ID NO: 24), miR-320b (SEQ ID NO: 25), miR-1470 (SEQ ID NO: 26), miR-4258 (SEQ ID NO: 27), miR-4673 (SEQ ID NO: 28), miR-4748 (SEQ ID NO: 29), miR-4787-3p (SEQ ID NO: 30), miR-204-3p (SEQ ID NO: 31), miR-6131 (SEQ ID NO: 32), miR-6752-3p (SEQ ID NO: 33), miR-4648 (SEQ ID NO: 34), miR-6126 (SEQ ID NO: 35), miR-1228-3p (SEQ ID NO: 36), miR-1231 (SEQ ID NO: 37), miR-4286 (SEQ ID NO: 38), miR-3935 (SEQ ID NO: 39), miR-2467-3p (SEQ ID NO: 40), miR-1185-2-3p (SEQ ID NO: 41), miR-6729-3p (SEQ ID NO: 42), miR-6798-3p (SEQ ID NO: 43), miR-379-5p (SEQ ID NO: 44), miR-323a-5p (SEQ ID NO: 45), miR-665 (SEQ ID NO: 46), miR-4279 (SEQ ID NO: 47), miR-3605-5p (SEQ ID NO: 48), miR-4684-3p (SEQ ID NO: 49), miR-365b-5p (SEQ ID NO: 50), miR-6782-5p (SEQ ID NO: 51), miR-6880-3p (SEQ ID NO: 52), miR-6887-3p (SEQ ID NO: 53), miR-4433b-5p (SEQ ID NO: 54), miR-10396a-3p (SEQ ID NO: 55), miR-373-5p (SEQ ID NO: 56), miR-4539 (SEQ ID NO: 57), miR-4687-3p (SEQ ID NO: 58), miR-4763-5p (SEQ ID NO: 59), miR-4482-3p (SEQ ID NO: 60), miR-6721-5p (SEQ ID NO: 61), miR-6812-3p (SEQ ID NO: 62), miR-6815-5p (SEQ ID NO: 63), miR-6871-5p (SEQ ID NO: 64), miR-7975 (SEQ ID NO: 65), miR-11181-3p (SEQ ID NO: 66), miR-2278 (SEQ ID NO: 67), miR-3192-5p (SEQ ID NO: 68), miR-4722-3p (SEQ ID NO: 69), miR-4750-3p (SEQ ID NO: 70), miR-6804-5p (SEQ ID NO: 71), miR-6828-5p (SEQ ID NO: 72), miR-614 (SEQ ID NO: 73), miR-320c (SEQ ID NO: 74), miR-2110 (SEQ ID NO: 75), miR-3177-3p (SEQ ID NO: 76), miR-4497 (SEQ ID NO: 77), miR-210-5p (SEQ ID NO: 78), miR-6778-5p (SEQ ID NO: 79), miR-6780a-5p (SEQ ID NO: 80), miR-6801-3p (SEQ ID NO: 81), miR-8059 (SEQ ID NO: 82), miR-657 (SEQ ID NO: 83), miR-921 (SEQ ID NO: 84), miR-4451 (SEQ ID NO: 85), miR-3059-3p (SEQ ID NO: 86), miR-3619-3p (SEQ ID NO: 87), miR-4646-5p (SEQ ID NO: 88), miR-525-5p (SEQ ID NO: 89), miR-4444 (SEQ ID NO: 90), miR-4458 (SEQ ID NO: 91), miR-4746-3p (SEQ ID NO: 92), miR-1292-3p (SEQ ID NO: 93), miR-6133 (SEQ ID NO: 94), miR-6754-3p (SEQ ID NO: 95), miR-365a-3p (SEQ ID NO: 96), miR-365b-3p (SEQ ID NO: 97), miR-6748-3p (SEQ ID NO: 98), miR-6892-3p (SEQ ID NO: 99), miR-8083 (SEQ ID NO: 100), miR-4724-5p (SEQ ID NO: 101), miR-6884-5p (SEQ ID NO: 102), miR-7156-3p (SEQ ID NO: 103), miR-19b-3p (SEQ ID NO: 104), miR-518e-3p (SEQ ID NO: 105), miR-646 (SEQ ID NO: 106), miR-298 (SEQ ID NO: 107), miR-1234-3p (SEQ ID NO: 108), miR-2115-5p (SEQ ID NO: 109), miR-3142 (SEQ ID NO: 110), miR-3179 (SEQ ID NO: 111), miR-3190-5p (SEQ ID NO: 112), miR-3675-3p (SEQ ID NO: 113), miR-4441 (SEQ ID NO: 114), miR-4475 (SEQ ID NO: 115), miR-6718-5p (SEQ ID NO: 116), miR-6743-3p (SEQ ID NO: 117), miR-6894-3p (SEQ ID NO: 118), miR-7112-5p (SEQ ID NO: 119), miR-12116 (SEQ ID NO: 120), miR-518d-3p (SEQ ID NO: 121), miR-145-5p (SEQ ID NO: 122), miR-6801-5p (SEQ ID NO: 123), miR-127-3p (SEQ ID NO: 124), and miR-4731-3p (SEQ ID NO: 125), or to a complementary strand of these polynucleotides.

<1-2> The detection kit for liver disease described in <1-1>, which includes a nucleic acid capable of binding specifically to one or more polynucleotides, which are biomarkers, selected from the group consisting of miR-4454 (SEQ ID NO: 1), miR-3138 (SEQ ID NO: 2), miR-3679 (SEQ ID NO: 3), miR-4521 (SEQ ID NO: 4), miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21), miR-320b-2 (SEQ ID NO: 22), miR-451a (SEQ ID NO: 23), miR-548d-3p (SEQ ID NO: 24), miR-320b (SEQ ID NO: 25), miR-1470 (SEQ ID NO: 26), miR-4258 (SEQ ID NO: 27), miR-4673 (SEQ ID NO: 28), miR-4748 (SEQ ID NO: 29), miR-4787-3p (SEQ ID NO: 30), miR-204-3p (SEQ ID NO: 31), miR-6131 (SEQ ID NO: 32), miR-6752-3p (SEQ ID NO: 33), miR-4648 (SEQ ID NO: 34), miR-6126 (SEQ ID NO: 35), miR-1228-3p (SEQ ID NO: 36), miR-1231 (SEQ ID NO: 37), miR-4286 (SEQ ID NO: 38), miR-3935 (SEQ ID NO: 39), miR-2467-3p (SEQ ID NO: 40), miR-1185-2-3p (SEQ ID NO: 41), miR-6729-3p (SEQ ID NO: 42), miR-6798-3p (SEQ ID NO: 43), miR-379-5p (SEQ ID NO: 44), miR-323a-5p (SEQ ID NO: 45), miR-665 (SEQ ID NO: 46), miR-4279 (SEQ ID NO: 47), miR-3605-5p (SEQ ID NO: 48), miR-4684-3p (SEQ ID NO: 49), miR-365b-5p (SEQ ID NO: 50), miR-6782-5p (SEQ ID NO: 51), miR-6880-3p (SEQ ID NO: 52), miR-6887-3p (SEQ ID NO: 53), miR-4433b-5p (SEQ ID NO: 54), miR-10396a-3p (SEQ ID NO: 55), miR-373-5p (SEQ ID NO: 56), miR-4539 (SEQ ID NO: 57), miR-4687-3p (SEQ ID NO: 58), miR-4763-5p (SEQ ID NO: 59), miR-4482-3p (SEQ ID NO: 60), miR-6721-5p (SEQ ID NO: 61), miR-6812-3p (SEQ ID NO: 62), miR-6815-5p (SEQ ID NO: 63), miR-6871-5p (SEQ ID NO: 64), miR-7975 (SEQ ID NO: 65), miR-11181-3p (SEQ ID NO: 66), miR-2278 (SEQ ID NO: 67), miR-3192-5p (SEQ ID NO: 68), miR-4722-3p (SEQ ID NO: 69), miR-4750-3p (SEQ ID NO: 70), miR-6804-5p (SEQ ID NO: 71), miR-6828-5p (SEQ ID NO: 72), miR-614 (SEQ ID NO: 73), miR-320c (SEQ ID NO: 74), miR-2110 (SEQ ID NO: 75), miR-3177-3p (SEQ ID NO: 76), miR-4497 (SEQ ID NO: 77), miR-210-5p (SEQ ID NO: 78), miR-6778-5p (SEQ ID NO: 79), miR-6780a-5p (SEQ ID NO: 80), miR-6801-3p (SEQ ID NO: 81), and miR-8059 (SEQ ID NO: 82), or to a complementary strand of these polynucleotides.

<1-3> The detection kit for liver disease described in <1-2>, which includes a nucleic acid capable of binding specifically to a polynucleotide, which is a biomarker, selected from the group consisting of miR-4454 (SEQ ID NO: 1), miR-3138 (SEQ ID NO: 2), miR-3679 (SEQ ID NO: 3), and miR-4521 (SEQ ID NO: 4), or to a complementary strand of these polynucleotides.

<1-4> The detection kit for liver disease described in <1-3>, where the kit further includes at least one nucleic acid capable of binding specifically to a polynucleotide which is another biomarker.

# EP 4 574 977 A1

<1-5> The detection kit for liver disease described in <1-4>, where the other biomarker is one or more of the biomarkers selected from the group consisting of miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21), and miR-320b-2 (SEQ ID NO: 22).

<1-6> The detection kit for liver disease described in <1-1>, which includes a nucleic acid capable of binding specifically to one or more polynucleotides, which are biomarkers, selected from the group consisting of miR-451a (SEQ ID NO: 23), miR-4787-3p (SEQ ID NO: 30), miR-6126 (SEQ ID NO: 35), miR-1228-3p (SEQ ID NO: 36), miR-6798-3p (SEQ ID NO: 43), miR-7975 (SEQ ID NO: 65), miR-614 (SEQ ID NO: 73), miR-6801-3p (SEQ ID NO: 81), miR-657 (SEQ ID NO: 83), miR-921 (SEQ ID NO: 84), miR-4451 (SEQ ID NO: 85), miR-3059-3p (SEQ ID NO: 86), miR-3619-3p (SEQ ID NO: 87), miR-4646-5p (SEQ ID NO: 88), miR-525-5p (SEQ ID NO: 89), miR-4444 (SEQ ID NO: 90), miR-4458 (SEQ ID NO: 91), miR-4746-3p (SEQ ID NO: 92), miR-1292-3p (SEQ ID NO: 93), miR-6133 (SEQ ID NO: 94), miR-6754-3p (SEQ ID NO: 95), miR-365a-3p (SEQ ID NO: 96), miR-365b-3p (SEQ ID NO: 97), miR-6748-3p (SEQ ID NO: 98), miR-6892-3p (SEQ ID NO: 99), miR-8083 (SEQ ID NO: 100), miR-4724-5p (SEQ ID NO: 101), miR-6884-5p (SEQ ID NO: 102), miR-7156-3p (SEQ ID NO: 103), miR-19b-3p (SEQ ID NO: 104), miR-518e-3p (SEQ ID NO: 105), miR-646 (SEQ ID NO: 106), miR-298 (SEQ ID NO: 107), miR-1234-3p (SEQ ID NO: 108), miR-2115-5p (SEQ ID NO: 109), miR-3142 (SEQ ID NO: 110), miR-3179 (SEQ ID NO: 111), miR-3190-5p (SEQ ID NO: 112), miR-3675-3p (SEQ ID NO: 113), miR-4441 (SEQ ID NO: 114), miR-4475 (SEQ ID NO: 115), miR-6718-5p (SEQ ID NO: 116), miR-6743-3p (SEQ ID NO: 117), miR-6894-3p (SEQ ID NO: 118), miR-7112-5p (SEQ ID NO: 119), miR-12116 (SEQ ID NO: 120), miR-518d-3p (SEQ ID NO: 121), miR-145-5p (SEQ ID NO: 122), miR-6801-5p (SEQ ID NO: 123), miR-127-3p (SEQ ID NO: 124), and miR-4731-3p (SEQ ID NO: 125), or to a complementary strand of these polynucleotides.

<1-7> The detection kit for liver disease described in <1-1>, where the liver disease is selected from the group consisting of hepatitis, hepatic fibrosis, fatty liver, hepatic cirrhosis and liver cancer.

<1-8> The detection kit for liver disease described in <1-1>, where the liver disease is hepatic cirrhosis.

<1-9> The detection kit for liver disease described in <1-2>, where the liver disease is a viral liver disease, and is preferably hepatitis C (HCV) or hepatitis B (HBV).

<1-10> The detection kit for liver disease described in <1-6>, where the liver disease is non-alcoholic steatohepatitis (NASH).

<2-1> The detection kit for liver disease described in <1-1>, where the nucleic acid is selected from a polynucleotide or a fragment thereof listed in any of the (a) to (c) below:

(a)

(a-1) a polynucleotide or a fragment thereof containing 15 or more continuous bases, the polynucleotide consisting of a nucleotide sequence complementary to a nucleotide sequence of any of SEQ ID NOs: 1 to 125, or to a nucleotide sequence where u is t in this nucleotide sequence,

(a-2) a polynucleotide or a fragment thereof containing a deletion, substitution, addition or insertion of one or more bases in a nucleotide sequence of a polynucleotide or a fragment thereof in (a-1),

(a-3) a polynucleotide of (a-1) or (a-2) or a fragment thereof containing a modified nucleic acid and/or a modified nucleotide,

(b) a polynucleotide or a fragment thereof containing 15 or more continuous bases, the polynucleotide containing a nucleotide sequence complementary to a nucleotide sequence of any of SEQ ID NOs: 1 to 125, or to a nucleotide sequence where u is t in this nucleotide sequence, and

(c) a polynucleotide that hybridizes with a polynucleotide or a fragment thereof listed in any of the (c-1) to (c-4) below under a high stringent condition.

(c-1) a polynucleotide or a fragment thereof containing 15 or more continuous bases, the polynucleotide consisting of a nucleotide sequence of any of SEQ ID NOs: 1 to 125, or a nucleotide sequence where u is t in this nucleotide sequence,

(c-2) a polynucleotide or a fragment thereof containing a deletion, substitution, addition or insertion of one or more bases in a nucleotide sequence of a polynucleotide or a fragment thereof in (c-1),

(c-3) a polynucleotide of (c-1) or (c-2) or a fragment thereof containing a modified nucleic acid and/or a modified nucleotide,

(c-4) a polynucleotide or a fragment thereof containing 15 or more continuous bases, the polynucleotide containing a nucleotide sequence of any of SEQ ID NOs: 1 to 125, or a nucleotide sequence where u is t in this nucleotide sequence

<2-2> The detection kit for liver disease described in <2-1>, where the nucleic acid is selected from a polynucleotide or a fragment thereof listed in any of the (a) to (c) below:

(a)

(a-1) a polynucleotide or a fragment thereof containing 15 or more continuous bases, the polynucleotide consisting of a nucleotide sequence complementary to a nucleotide sequence of any of SEQ ID NOs: 1 to 4, or to a nucleotide sequence where u is t in this nucleotide sequence,
(a-2) a polynucleotide or a fragment thereof containing a deletion, substitution, addition or insertion of one or more bases in a nucleotide sequence of a polynucleotide or a fragment thereof in (a-1),
(a-3) a polynucleotide of (a-1) or (a-2) or a fragment thereof containing a modified nucleic acid and/or a modified nucleotide,

(b) a polynucleotide or a fragment thereof containing 15 or more continuous bases, the polynucleotide containing a nucleotide sequence complementary to a nucleotide sequence of any of SEQ ID NOs: 1 to 4, or to a nucleotide sequence where u is t in this nucleotide sequence, and
(c) a polynucleotide that hybridizes with a polynucleotide or a fragment thereof listed in any of the (c-1) to (c-4) below under a high stringent condition.

(c-1) a polynucleotide or a fragment thereof containing 15 or more continuous bases, the polynucleotide consisting of a nucleotide sequence of any of SEQ ID NOs: 1 to 4, or a nucleotide sequence where u is t in this nucleotide sequence,
(c-2) a polynucleotide or a fragment thereof containing a deletion, substitution, addition or insertion of one or more bases in a nucleotide sequence of a polynucleotide or a fragment thereof in (c-1),
(c-3) a polynucleotide of (c-1) or (c-2) or a fragment thereof containing a modified nucleic acid and/or a modified nucleotide,
(c-4) a polynucleotide or a fragment thereof containing 15 or more continuous bases, the polynucleotide containing a nucleotide sequence of any of SEQ ID NOs: 1 to 4, or a nucleotide sequence where u is t in this nucleotide sequence

<2-3> The detection kit for liver disease described in <2-2>, where the kit further includes at least one nucleic acid capable of binding specifically to a polynucleotide which is another biomarker, and
the nucleic acid capable of binding specifically to a polynucleotide which is the other biomarker, is selected from a polynucleotide or a fragment thereof listed in any of the (d) to (f) below:

(d)

(d-1) a polynucleotide or a fragment thereof containing 15 or more continuous bases, the polynucleotide consisting of a nucleotide sequence complementary to a nucleotide sequence of any of SEQ ID NOs: 5 to 21, or to a nucleotide sequence where u is t in this nucleotide sequence,
(d-2) a polynucleotide or a fragment thereof containing a deletion, substitution, addition or insertion of one or more bases in a nucleotide sequence of a polynucleotide or a fragment thereof in (d-1),
(d-3) a polynucleotide of (d-1) or (d-2) or a fragment thereof containing a modified nucleic acid and/or a modified nucleotide,

(e) a polynucleotide or a fragment thereof containing 15 or more continuous bases, the polynucleotide containing a nucleotide sequence complementary to a nucleotide sequence of any of SEQ ID NOs: 5 to 21, or to a nucleotide sequence where u is t in this nucleotide sequence, and
(f) a polynucleotide that hybridizes with a polynucleotide or a fragment thereof listed in any of the (f-1) to (f-4) below under a high stringent condition.

(f-1) a polynucleotide or a fragment thereof containing 15 or more continuous bases, the polynucleotide consisting of a nucleotide sequence of any of SEQ ID NOs: 5 to 21, or a nucleotide sequence where u is t in this nucleotide sequence,
(f-2) a polynucleotide or a fragment thereof containing a deletion, substitution, addition or insertion of one or more bases in a nucleotide sequence of a polynucleotide or a fragment thereof in (f-1),

(f-3) a polynucleotide of (f-1) or (f-2) or a fragment thereof containing a modified nucleic acid and/or a modified nucleotide,

(f-4) a polynucleotide or a fragment thereof containing 15 or more continuous bases, the polynucleotide containing a nucleotide sequence of any of SEQ ID NOs: 5 to 21, or a nucleotide sequence where u is t in this nucleotide sequence

<2-4> The detection kit for liver disease described in <2-1>, where the polynucleotide of any of the above (a) to (c) is selected from polynucleotides of any of SEQ ID NOs: 1 to 82.

<2-5> The detection kit for liver disease described in <2-1>, where the polynucleotide of any of the above (a) to (c) is selected from polynucleotides of any of SEQ ID NOs: 23, 30, 35, 36, 43, 65, 73, 81, 83 to 125.

<2-6> The detection kit for liver disease described in <2-1>, where the liver disease is selected from the group consisting of hepatitis, hepatic fibrosis, fatty liver, hepatic cirrhosis and liver cancer.

<2-7> The detection kit for liver disease described in <2-1>, where the liver disease is hepatic cirrhosis.

<2-8> The detection kit for liver disease described in <2-4>, where the liver disease is a viral liver disease, and is preferably hepatitis C (HCV) or hepatitis B (HBV).

<2-9> The detection kit for liver disease described in <2-5>, where the liver disease is non-alcoholic steatohepatitis (NASH).

<3-1> A biomarker, selected from polynucleotides of any of SEQ ID NOs: 1 to 125.

<3-2> The biomarker described in <3-1>, selected from polynucleotides of any of SEQ ID NOs: 1 to 82.

<3-3> The biomarker described in <3-2>, selected from the group consisting of miR-4454 (SEQ ID NO: 1), miR-3138 (SEQ ID NO: 2), miR-3679 (SEQ ID NO: 3) and miR-4521 (SEQ ID NO: 4).

<3-4> The biomarker described in <3-1>, selected from polynucleotides of any of SEQ ID NOs: 23, 30, 35, 36, 43, 65, 73, 81, 83 to 125.

<3-5> The biomarker described in <3-1>, where the biomarker is obtainable from a biological sample selected from the group consisting of blood, serum, plasma, saliva, sweat, tears, faeces, urine and organ specimens.

<3-6> The biomarker described in <3-1>, for use in diagnosing the presence or absence of liver disease or the risk thereof.

<3-7> The biomarker described in <3-1>, for use in assessing the degree of progression of liver disease.

<3-8> The biomarker described in <3-1>, where the liver disease is selected from the group consisting of hepatitis, hepatic fibrosis, fatty liver, hepatic cirrhosis and liver cancer.

<3-9> The biomarker described in <3-1>, where the liver disease is hepatic cirrhosis.

<3-10> The biomarker described in <3-2>, where the liver disease is a viral liver disease, and is preferably hepatitis C (HCV) or hepatitis B (HBV).

<3-11> The biomarker described in <3-4>, where the liver disease is non-alcoholic steatohepatitis (NASH).

<4-1> A method for assessing the presence or absence of liver disease or the risk or degree of progression thereof in a subject, and/or the degree of success of a procedure performed for treatment, the method including

measuring a level of a biomarker in a biological sample from the subject, and
comparing a measurement value of the biomarker with a reference value, where
the biomarker is one or more of the biomarkers selected from polynucleotides of SEQ ID NOs: 1 to 125.

<4-2> The method described in <4-1>, where the biomarker is selected from polynucleotides of any of SEQ ID NOs: 1 to 82.

<4-3> The method described in <4-2>, where the biomarker is one or more of the biomarkers selected from the group consisting of miR-4454 (SEQ ID NO: 1), miR-3138 (SEQ ID NO: 2), miR-3679 (SEQ ID NO: 3) and miR-4521 (SEQ ID NO: 4).

<4-4> The method described in <4-3>, which further includes

measuring a level of an additional biomarker in a biological sample from a subject, and
comparing a measurement value of the additional biomarker with a reference value.

<4-5> The method described in <4-4>, where the additional biomarker is one or more of the biomarkers selected from the group consisting of miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21) and miR-320b-2 (SEQ ID NO: 22).

<4-6> The method described in <4-1>, where the biomarker is selected from polynucleotides of any of SEQ ID NOs:

23, 30, 35, 36, 43, 65, 73, 81, 83 to 125.

<4-7> The method described in <4-1>, where the biological sample is selected from the group consisting of blood, serum, plasma, saliva, sweat, tears, faeces, urine and organ specimens.

<4-8> The method described in <4-1>, where the liver disease is selected from the group consisting of hepatitis, hepatic fibrosis, fatty liver, hepatic cirrhosis and liver cancer.

<4-9> The method described in <4-1>, where the liver disease is hepatic cirrhosis.

<4-10> The method described in <4-2>, where the liver disease is a viral liver disease, and is preferably hepatitis C (HCV) or hepatitis B (HBV).

<4-11> The method described in <4-6>, where the liver disease is non-alcoholic steatohepatitis (NASH).

<5-1> A method for screening a candidate compound of a therapeutic drug for liver disease in a subject, which includes

> measuring a level of a biomarker in a biological sample from the subject administered with a candidate compound of a therapeutic drug for liver disease, and
> comparing a measurement value of the biomarker with a reference value, where
> the biomarker is one or more of the biomarkers selected from polynucleotides of SEQ ID NOs: 1 to 125.

<5-2> The method described in <5-1>, where the biomarker is selected from polynucleotides of any of SEQ ID NOs: 1 to 82.

<5-3> The method described in <5-2>, where the biomarker is one or more of the biomarkers selected from the group consisting of miR-4454 (SEQ ID NO: 1), miR-3138 (SEQ ID NO: 2), miR-3679 (SEQ ID NO: 3) and miR-4521 (SEQ ID NO: 4).

<5-4> The method described in <5-3>, which further includes

> measuring a level of an additional biomarker in a biological sample from a subject, and
> comparing a measurement value of the additional biomarker with a reference value.

<5-5> The method described in <5-4>, where the additional biomarker is one or more of the biomarkers selected from the group consisting of miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21) and miR-320b-2 (SEQ ID NO: 22).

<5-6> The method described in <5-1>, where the biomarker is selected from polynucleotides of any of SEQ ID NOs: 23, 30, 35, 36, 43, 65, 73, 81, 83 to 125.

<5-7> The method described in <5-1>, where the biological sample is selected from the group consisting of blood, serum, plasma, saliva, sweat, tears, faeces, urine and organ specimens.

<5-8> The method described in <5-1>, where the liver disease is selected from the group consisting of hepatitis, hepatic fibrosis, fatty liver, hepatic cirrhosis and liver cancer.

<5-9> The method described in <5-1>, where the liver disease is hepatic cirrhosis.

<5-10> The method described in <5-2>, where the liver disease is a viral liver disease, and is preferably hepatitis C (HCV) or hepatitis B (HBV).

<5-11> The method described in <5-6>, where the liver disease is non-alcoholic steatohepatitis (NASH).

<6-1> A method for treating liver disease in a subject, which includes

> measuring a level of a biomarker in a biological sample from the subject, and
> comparing a measurement value of the biomarker with a reference value, where
> the biomarker is one or more of the biomarkers selected from polynucleotides of SEQ ID NOs: 1 to 125.

<6-2> The method described in <6-1>, where the biomarker is selected from polynucleotides of any of SEQ ID NOs: 1 to 82.

<6-3> The method described in <6-2>, where the biomarker is one or more of the biomarkers selected from the group consisting of miR-4454 (SEQ ID NO: 1), miR-3138 (SEQ ID NO: 2), miR-3679 (SEQ ID NO: 3) and miR-4521 (SEQ ID NO: 4).

<6-4> The method described in <6-3>, which further includes

> measuring a level of an additional biomarker in a biological sample from a subject, and
> comparing a measurement value of the additional biomarker with a reference value.

<6-5> The method described in <6-4>, where the additional biomarker is one or more of the biomarkers selected from the group consisting of miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21) and miR-320b-2 (SEQ ID NO: 22).

<6-6> The method described in <6-1>, where the biomarker is selected from polynucleotides of any of SEQ ID NOs: 23, 30, 35, 36, 43, 65, 73, 81, 83 to 125.

<6-7> The method described in <6-1>, where the biological sample is selected from the group consisting of blood, serum, plasma, saliva, sweat, tears, faeces, urine and organ specimens.

<6-8> The method described in <6-1>, where the liver disease is selected from the group consisting of hepatitis, hepatic fibrosis, fatty liver, hepatic cirrhosis and liver cancer.

<6-9> The method described in <6-1>, where the liver disease is hepatic cirrhosis.

<6-10> The method described in <6-2>, where the liver disease is a viral liver disease, and is preferably hepatitis C (HCV) or hepatitis B (HBV).

<6-11> The method described in <6-6>, where the liver disease is non-alcoholic steatohepatitis (NASH).

<7-1> A method for detecting liver disease with high accuracy, which includes:

   measuring a level of a biomarker 1 in a biological sample from a subject;
   comparing a measurement value of the biomarker 1 with a reference value to obtain a parameter 1;
   measuring a level of at least one additional biomarker N (where N is an integer of 2 or more) in the biological sample from the subject;
   comparing a measurement value of the additional biomarker N with a reference value to obtain a parameter N; and
   obtaining a result in the form of one set of at least two parameters made up of a combination of the parameter 1 and one or more of the parameters N; and

   the result in the form of one set of at least two parameters indicating the presence or absence of liver disease or the risk or degree of progression thereof in the subject, and/or the degree of success of a procedure performed for treatment.

<7-2> The method described in <7-1>, where the result in the form of one set of at least two parameters is evaluated using a computer program.

<7-3> The method described in <7-1>, where the biomarker 1 and the at least one additional biomarker are selected from polynucleotides of SEQ ID NOs: 1 to 125.

<7-4> The method described in <7-3>, where the biomarker 1 and the at least one additional biomarker are selected from polynucleotides of any of SEQ ID NOs: 1 to 82.

<7-5> The method described in <7-4>, where the biomarker 1 and the at least one additional biomarker are selected from the group consisting of miR-4454 (SEQ ID NO: 1), miR-3138 (SEQ ID NO: 2), miR-3679 (SEQ ID NO: 3), miR-4521 (SEQ ID NO: 4), miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21) and miR-320b-2 (SEQ ID NO: 22).

<7-6> The method described in <7-3>, where the biomarker 1 and the at least one additional biomarker are selected from polynucleotides of any of SEQ ID NOs: 23, 30, 35, 36, 43, 65, 73, 81, 83 to 125.

<7-7> The method described in <7-1>, where the biological sample is selected from the group consisting of blood, serum, plasma, saliva, sweat, tears, faeces, urine and organ specimens.

<7-8> The method described in <7-1>, where the liver disease is selected from the group consisting of hepatitis, hepatic fibrosis, fatty liver, hepatic cirrhosis and liver cancer.

<7-9> The method described in <7-1>, where the liver disease is hepatic cirrhosis.

<7-10> The method described in <7-4>, where the liver disease is a viral liver disease, and is preferably hepatitis C (HCV) or hepatitis B (HBV).

<7-11> The method described in <7-6>, where the liver disease is non-alcoholic steatohepatitis (NASH).

<8-1> A diagnostic support system for liver disease, provided with:

   a unit for measuring a level of one or more or at least two biomarkers in a biological sample from a subject;
   a unit for comparing a measurement value of the one or more or at least two biomarkers with respective reference values thereof to obtain a plurality of parameters; and
   a unit for calculating, from the plurality of parameters, the presence or absence of liver disease or the risk or degree of progression thereof in the subject, and/or the degree of success of a procedure performed for treatment.

<8-2> The diagnostic support system for liver disease described in <8-1>, where the one or more or at least two biomarkers are selected from polynucleotides of SEQ ID NOs: 1 to 125.

<8-3> The diagnostic support system for liver disease described in <8-2>, where the one or more or at least two biomarkers are selected from polynucleotides of any of SEQ ID NOs: 1 to 82.

<8-4> The diagnostic support system for liver disease described in <8-3>, where the one or more or at least two biomarkers are selected from the group consisting of miR-4454 (SEQ ID NO: 1), miR-3138 (SEQ ID NO: 2), miR-3679 (SEQ ID NO: 3), miR-4521 (SEQ ID NO: 4), miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21) and miR-320b-2 (SEQ ID NO: 22).

<8-5> The diagnostic support system for liver disease described in <8-2>, where the one or more or at least two biomarkers are selected from polynucleotides of any of SEQ ID NOs: 23, 30, 35, 36, 43, 65, 73, 81, 83 to 125.

<8-6> The diagnostic support system for liver disease described in <8-1>, where the biological sample is selected from the group consisting of blood, serum, plasma, saliva, sweat, tears, faeces, urine and organ specimens.

<8-7> The diagnostic support system for liver disease described in <8-1>, where the liver disease is selected from the group consisting of hepatitis, hepatic fibrosis, fatty liver, hepatic cirrhosis and liver cancer.

<8-8> The diagnostic support system for liver disease described in <8-1>, where the liver disease is hepatic cirrhosis.

<8-9> The diagnostic support system for liver disease described in <8-3>, where the liver disease is a viral liver disease, and is preferably hepatitis C (HCV) or hepatitis B (HBV).

<8-10> The diagnostic support system for liver disease described in <8-5>, where the liver disease is non-alcoholic steatohepatitis (NASH).

<9-1> A method for performing data processing for staging the pathology of liver disease of a subject, the method executed by a computer, the computer provided with:

a computer processor; and
a computer readable medium storing software that gives instructions for staging the pathology of liver disease of the subject; and

where the method includes:

incorporating data of a plurality of parameters into a computer readable medium, the data of a plurality of parameters obtained by comparing a measurement value of one or more or at least two biomarkers in a biological sample obtained from the subject with respective reference values thereof; and
outputting an assessment result of a pathology of liver disease of the subject as information by the software that gives instructions for staging the pathology of liver disease of the subject, the assessment performed by the computer that executes the instructions using the computer processor to process the data of the plurality of parameters; and

where the staging includes one or more selected from the group consisting of a hepatic fibrogenesis stage, Child-Pugh classification, MELD score, MELD Na score, PELD score, ALBI score, mALBI (modified ALBI) score, FibroScan score, new Inuyama classification, and new European classification.

<9-2> The method described in <9-1>, where the assessment result is a combination of results respectively assessed by processing the data of the plurality of parameters.

<9-3> The method described in <9-1>, where the one or more or at least two biomarkers are selected from polynucleotides of SEQ ID NOs: 1 to 125.

<9-4> The method described in <9-3>, where the one or more or at least two biomarkers are selected from polynucleotides of any of SEQ ID NOs: 1 to 82.

<9-5> The method described in <9-4>, where the one or more or at least two biomarkers are selected from the group consisting of miR-4454 (SEQ ID NO: 1), miR-3138 (SEQ ID NO: 2), miR-3679 (SEQ ID NO: 3), miR-4521 (SEQ ID NO: 4), miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21) and miR-320b-2 (SEQ ID NO: 22).

<9-6> The method described in <9-3>, where the one or more or at least two biomarkers are selected from polynucleotides of any of SEQ ID NOs: 23, 30, 35, 36, 43, 65, 73, 81, 83 to 125.

<9-7> The method described in <9-1>, where the biological sample is selected from the group consisting of blood, serum, plasma, saliva, sweat, tears, faeces, urine and organ specimens.

<9-8> The method described in <9-1>, where the liver disease is selected from the group consisting of hepatitis, hepatic fibrosis, fatty liver, hepatic cirrhosis and liver cancer.

<9-9> The method described in <9-1>, where the liver disease is hepatic cirrhosis.

<9-10> The method described in <9-4>, where the liver disease is a viral liver disease, and is preferably hepatitis C (HCV) or hepatitis B (HBV).

<9-11> The method described in <9-6>, where the liver disease is non-alcoholic steatohepatitis (NASH).

<9-12> The method described in <9-1>, where the subject is a mammalian subject.

<9-13> The method described in <9-11>, where the subject is a human subject.

<10-1> A program for executing an assessment of the presence or absence of liver disease or the risk or degree of progression thereof in a subject and/or an assessment of the degree of success of a procedure performed for a treatment on a computer, the program executing:

a measurement value acquisition step of respectively acquiring a measurement value of a level of one or more or at least two biomarkers obtained from a biological sample from the subject;

a parameter data acquisition step of acquiring data of a plurality of parameters by comparing a measurement value of the one or more or at least two biomarkers with respective reference values; and

an assessment step of processing the data of the plurality of parameters, and assessing the presence or absence of liver disease or the risk or degree of progression thereof in the subject, and/or the degree of success of a procedure performed for treatment; and

the biomarker being selected from a polynucleotide of SEQ ID NOs: 1 to 125.

<10-2> The program described in <10-1>, where the biomarker is selected from polynucleotides of any of SEQ ID NOs: 1 to 82.

<10-3> The program described in <10-2>, where the biomarker is selected from the group consisting of miR-4454 (SEQ ID NO: 1), miR-3138 (SEQ ID NO: 2), miR-3679 (SEQ ID NO: 3) and miR-4521 (SEQ ID NO: 4).

<10-4> The program described in <10-1>, where the biomarker is selected from polynucleotides of any of SEQ ID NOs: 23, 30, 35, 36, 43, 65, 73, 81, 83 to 125.

<10-5> The program described in <10-1>, where the biological sample is selected from the group consisting of blood, serum, plasma, saliva, sweat, tears, faeces, urine and organ specimens.

<10-6> The program described in <10-1>, where the liver disease is selected from the group consisting of hepatitis, hepatic fibrosis, fatty liver, hepatic cirrhosis and liver cancer.

<10-7> The program described in <10-1>, where the liver disease is hepatic cirrhosis.

<10-8> The program described in <10-2>, where the liver disease is a viral liver disease, and is preferably hepatitis C (HCV) or hepatitis B (HBV).

<10-9> The program described in <10-4>, where the liver disease is non-alcoholic steatohepatitis (NASH).

<11-1> A data processing device to perform data processing for detecting liver disease, provided with:

a missing value supplement unit to obtain missing value supplemental data by supplementing a missing value in miRNA expression level data;

a logarithmic transformation unit to obtain logarithmic transformation data by logarithmically transforming the missing value supplemental data;

a regression transformation unit to obtain regression transformation data by substituting the logarithmic transformation data in a regression model and executing a regression transformation;

a training data acquisition unit to acquire training data; and

a learnt model creation unit to create a learnt model using the regression transformation data as the training data, and

the miRNA is one or more selected from polynucleotides of SEQ ID NOs: 1 to 125.

<11-2> The data processing device to perform data processing for detecting liver disease described in <11-1>, where the regression transformation includes:

multiplying a regression coefficient;

obtaining a logit value showing a predictive result of a fibrogenesis stage by adding an intercept value at each fibrogenesis stage;

calculating a predictive probability of each fibrogenesis stage by performing an inverse transformation of a logistic transformation on the logit value and transforming it to a probability value of between 0 and 1; and
configuring the fibrogenesis stage with the highest probability amongst the predictive probabilities of each fibrogenesis stage, to be a predictive value.

<11-3> The data processing device to perform data processing for detecting liver disease described in <11-2>, where the regression model is selected from the group consisting of a multinominal LASSO model, gaussian LASSO (Least Absolute Shrinkage and Selection Operator) model, and ordinal logistic regression model.

<11-4> The data processing device to perform data processing for detecting liver disease described in <11-1>, where the training data are the training data for showing a relationship between a blood miRNA concentration for learning, and a fibrogenesis stage corresponding to the blood miRNA concentration for learning.

<11-5> The data processing device to perform data processing for detecting liver disease described in <11-1>, where the data processing for detecting liver disease further includes a training data acquisition step for acquiring training data, and a learning data creation step for creating learning data utilizing the training data, and where the training data are training data for showing a relationship between a blood miRNA concentration for learning and a fibrogenesis stage corresponding to the blood miRNA concentration for learning.

<11-6> The data processing device to perform data processing for detecting liver disease described in <11-1>, where the data processing for detecting liver disease further includes a pre-processing step for obtaining data for analysis, and the pre-processing step includes:

obtaining a low expression miRNA list by listing low expression miRNA;
obtaining a missing value supplemental data by supplementing a missing value in the data of the miRNA list;
obtaining data for transformation by excluding low expression miRNA included in the low expression miRNA list from an analysis target; and
logarithmically transforming the data for transformation.

<11-7> The data processing device to perform data processing for detecting liver disease described in <11-1>, where the biomarker is one or more of the biomarkers selected from polynucleotides of any of SEQ ID NOs: 1 to 82.

<11-8> The data processing device to perform data processing for detecting liver disease described in <11-1>, where the biomarker is one or more of the biomarkers selected from polynucleotides of any of SEQ ID NOs: 23, 30, 35, 36, 43, 65, 73, 81, 83 to 125.

<11-9> The data processing device to perform data processing for detecting liver disease described in <11-1>, where the biological sample is selected from the group consisting of blood, serum, plasma, saliva, sweat, tears, faeces, urine and organ specimens.

<11-10> The data processing device to perform data processing for detecting liver disease described in <11-1>, where the liver disease is selected from the group consisting of hepatitis, hepatic fibrosis, fatty liver, hepatic cirrhosis and liver cancer.

<11-11> The data processing device to perform data processing for detecting liver disease described in <11-1>, where the liver disease is hepatic cirrhosis.

<11-12> The data processing device to perform data processing for detecting liver disease described in <11-7>, where the liver disease is a viral liver disease, and is preferably hepatitis C (HCV) or hepatitis B (HBV).

<11-13> The data processing device to perform data processing for detecting liver disease described in <11-8>, where the liver disease is non-alcoholic steatohepatitis (NASH).

Advantageous Effects of Invention

Liver disease can be detected non-invasively or minimally invasively with high accuracy by using: the liver disease non-invasive biomarker; the kit, method and program using this biomarker; the data processing method for detecting liver disease with high accuracy using this liver disease non-invasive marker; and the data processing device for use in this data processing, of the present invention.

Brief Description of Drawings

[Fig. 1] shows an analysis of the relationship between a variety of miRNA expression levels and fibrogenesis stages for subjects with a fibrogenesis stage of 0, and subjects with a fibrogenesis stage of 1 to 4.
[Fig. 2] shows analyses of the relationships between a variety of miRNA expression levels and fibrogenesis stages (extent of fibrogenesis) for normal subjects, subjects with a fibrogenesis stage of 0, subjects with a fibrogenesis stage of 1, and subjects with a fibrogenesis stage of 4.

[Fig. 3] shows an analysis of the relationship between a variety of miRNA expression levels and Child-Pugh scores for subjects with a Child-Pugh score of 0, and subjects with a Child-Pugh score of 6 or higher.

[Fig. 4] shows analyses of relationships between a variety of miRNA expression levels and Child-Pugh scores for subjects with a Child-Pugh score of 0 and subjects with a Child-Pugh score of 6 to 11.

[Fig. 5] is a flowchart showing the flow of data processing for predicting the fibrogenesis stage of a viral liver disease.

[Fig. 6] is a flowchart showing the flow of data processing for predicting the fibrogenesis stage of non-alcoholic steatohepatitis (NASH).

[Fig. 7] is a flowchart showing the flow of data processing for predicting the Brunt classification of non-alcoholic steatohepatitis (NASH).

Description of Embodiments

The present invention will be explained in detail as follows. However, the below explanation is not intended to limit the scope of the present invention.

< Biomarker >

In one embodiment of the present invention, provided is a biomarker.

In the present invention, a biomarker is selected from the group consisting of miR-4454 (SEQ ID NO: 1), miR-3138 (SEQ ID NO: 2), miR-3679 (SEQ ID NO: 3), miR-4521 (SEQ ID NO: 4), miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21), miR-320b-2 (SEQ ID NO: 22), miR-451a (SEQ ID NO: 23), miR-548d-3p (SEQ ID NO: 24), miR-320b (SEQ ID NO: 25), miR-1470 (SEQ ID NO: 26), miR-4258 (SEQ ID NO: 27), miR-4673 (SEQ ID NO: 28), miR-4748 (SEQ ID NO: 29), miR-4787-3p (SEQ ID NO: 30), miR-204-3p (SEQ ID NO: 31), miR-6131 (SEQ ID NO: 32), miR-6752-3p (SEQ ID NO: 33), miR-4648 (SEQ ID NO: 34), miR-6126 (SEQ ID NO: 35), miR-1228-3p (SEQ ID NO: 36), miR-1231 (SEQ ID NO: 37), miR-4286 (SEQ ID NO: 38), miR-3935 (SEQ ID NO: 39), miR-2467-3p (SEQ ID NO: 40), miR-1185-2-3p (SEQ ID NO: 41), miR-6729-3p (SEQ ID NO: 42), miR-6798-3p (SEQ ID NO: 43), miR-379-5p (SEQ ID NO: 44), miR-323a-5p (SEQ ID NO: 45), miR-665 (SEQ ID NO: 46), miR-4279 (SEQ ID NO: 47), miR-3605-5p (SEQ ID NO: 48), miR-4684-3p (SEQ ID NO: 49), miR-365b-5p (SEQ ID NO: 50), miR-6782-5p (SEQ ID NO: 51), miR-6880-3p (SEQ ID NO: 52), miR-6887-3p (SEQ ID NO: 53), miR-4433b-5p (SEQ ID NO: 54), miR-10396a-3p (SEQ ID NO: 55), miR-373-5p (SEQ ID NO: 56), miR-4539 (SEQ ID NO: 57), miR-4687-3p (SEQ ID NO: 58), miR-4763-5p (SEQ ID NO: 59), miR-4482-3p (SEQ ID NO: 60), miR-6721-5p (SEQ ID NO: 61), miR-6812-3p (SEQ ID NO: 62), miR-6815-5p (SEQ ID NO: 63), miR-6871-5p (SEQ ID NO: 64), miR-7975 (SEQ ID NO: 65), miR-11181-3p (SEQ ID NO: 66), miR-2278 (SEQ ID NO: 67), miR-3192-5p (SEQ ID NO: 68), miR-4722-3p (SEQ ID NO: 69), miR-4750-3p (SEQ ID NO: 70), miR-6804-5p (SEQ ID NO: 71), miR-6828-5p (SEQ ID NO: 72), miR-614 (SEQ ID NO: 73), miR-320c (SEQ ID NO: 74), miR-2110 (SEQ ID NO: 75), miR-3177-3p (SEQ ID NO: 76), miR-4497 (SEQ ID NO: 77), miR-210-5p (SEQ ID NO: 78), miR-6778-5p (SEQ ID NO: 79), miR-6780a-5p (SEQ ID NO: 80), miR-6801-3p (SEQ ID NO: 81), miR-8059 (SEQ ID NO: 82), miR-657 (SEQ ID NO: 83), miR-921 (SEQ ID NO: 84), miR-4451 (SEQ ID NO: 85), miR-3059-3p (SEQ ID NO: 86), miR-3619-3p (SEQ ID NO: 87), miR-4646-5p (SEQ ID NO: 88), miR-525-5p (SEQ ID NO: 89), miR-4444 (SEQ ID NO: 90), miR-4458 (SEQ ID NO: 91), miR-4746-3p (SEQ ID NO: 92), miR-1292-3p (SEQ ID NO: 93), miR-6133 (SEQ ID NO: 94), miR-6754-3p (SEQ ID NO: 95), miR-365a-3p (SEQ ID NO: 96), miR-365b-3p (SEQ ID NO: 97), miR-6748-3p (SEQ ID NO: 98), miR-6892-3p (SEQ ID NO: 99), miR-8083 (SEQ ID NO: 100), miR-4724-5p (SEQ ID NO: 101), miR-6884-5p (SEQ ID NO: 102), miR-7156-3p (SEQ ID NO: 103), miR-19b-3p (SEQ ID NO: 104), miR-518e-3p (SEQ ID NO: 105), miR-646 (SEQ ID NO: 106), miR-298 (SEQ ID NO: 107), miR-1234-3p (SEQ ID NO: 108), miR-2115-5p (SEQ ID NO: 109), miR-3142 (SEQ ID NO: 110), miR-3179 (SEQ ID NO: 111), miR-3190-5p (SEQ ID NO: 112), miR-3675-3p (SEQ ID NO: 113), miR-4441 (SEQ ID NO: 114), miR-4475 (SEQ ID NO: 115), miR-6718-5p (SEQ ID NO: 116), miR-6743-3p (SEQ ID NO: 117), miR-6894-3p (SEQ ID NO: 118), miR-7112-5p (SEQ ID NO: 119), miR-12116 (SEQ ID NO: 120), miR-518d-3p (SEQ ID NO: 121), miR-145-5p (SEQ ID NO: 122), miR-6801-5p (SEQ ID NO: 123), miR-127-3p (SEQ ID NO: 124) and miR-4731-3p (SEQ ID NO: 125).

In one embodiment of the present invention, a biomarker is selected from the group consisting of miR-4454 (SEQ ID NO: 1), miR-3138 (SEQ ID NO: 2), miR-3679 (SEQ ID NO: 3), miR-4521 (SEQ ID NO: 4), miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21), miR-320b-2 (SEQ ID NO: 22), miR-451a (SEQ ID NO: 23), miR-548d-3p (SEQ ID NO: 24), miR-320b (SEQ ID NO: 25), miR-1470 (SEQ ID NO: 26), miR-4258 (SEQ ID NO: 27), miR-4673 (SEQ ID NO: 28), miR-4748 (SEQ ID NO: 29), miR-4787-3p (SEQ ID NO: 30), miR-204-3p (SEQ ID NO: 31), miR-6131 (SEQ ID NO: 32), miR-6752-3p (SEQ ID NO: 33), miR-4648 (SEQ ID NO: 34), miR-6126 (SEQ ID NO: 35), miR-1228-3p (SEQ ID NO: 36), miR-1231 (SEQ ID NO: 37), miR-4286 (SEQ ID NO: 38), miR-3935 (SEQ ID NO: 39), miR-2467-3p (SEQ ID NO: 40), miR-1185-2-3p (SEQ ID NO: 41), miR-6729-3p (SEQ ID NO: 42), miR-6798-3p (SEQ ID NO: 43), miR-379-5p (SEQ ID NO: 44), miR-323a-5p (SEQ ID NO: 45), miR-665 (SEQ ID NO: 46), miR-4279 (SEQ ID NO: 47), miR-3605-5p (SEQ ID NO: 48), miR-4684-3p (SEQ ID NO: 49), miR-365b-5p (SEQ ID NO: 50), miR-6782-5p (SEQ ID NO: 51), miR-6880-3p (SEQ ID NO: 52), miR-6887-3p (SEQ ID NO: 53), miR-4433b-5p (SEQ ID NO: 54), miR-10396a-3p (SEQ ID NO: 55), miR-373-5p (SEQ ID NO: 56), miR-4539 (SEQ ID NO: 57), miR-4687-3p (SEQ ID NO: 58), miR-4763-5p (SEQ ID NO: 59), miR-4482-3p (SEQ ID NO: 60), miR-6721-5p (SEQ ID NO: 61), miR-6812-3p (SEQ ID NO: 62), miR-6815-5p (SEQ ID NO: 63), miR-6871-5p (SEQ ID NO: 64), miR-7975 (SEQ ID NO: 65), miR-11181-3p (SEQ ID NO: 66), miR-2278 (SEQ ID NO: 67), miR-3192-5p (SEQ ID NO: 68), miR-4722-3p (SEQ ID NO: 69), miR-4750-3p (SEQ ID NO: 70), miR-6804-5p (SEQ ID NO: 71), miR-6828-5p (SEQ ID NO: 72), miR-614 (SEQ ID NO: 73), miR-320c (SEQ ID NO: 74), miR-2110 (SEQ ID NO: 75), miR-3177-3p (SEQ ID NO: 76), miR-4497 (SEQ ID NO: 77), miR-210-5p (SEQ ID NO: 78), miR-6778-5p (SEQ ID NO: 79), miR-6780a-5p (SEQ ID NO: 80), miR-6801-3p (SEQ ID NO: 81), and miR-8059 (SEQ ID NO: 82).

In one embodiment of the present invention, a biomarker may include miR-4454 (SEQ ID NO: 1), miR-3138 (SEQ ID NO: 2), miR-3679 (SEQ ID NO: 3), miR-4521 (SEQ ID NO: 4), miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21), and miR-320b-2 (SEQ ID NO: 22); however, there are no limitations on this. In one embodiment of the present invention, a biomarker is selected from the group consisting of miR-4454 (SEQ ID NO: 1), miR-3138 (SEQ ID NO: 2), miR-3679 (SEQ ID NO: 3), miR-4521 (SEQ ID NO: 4), miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21) and miR-320b-2 (SEQ ID NO: 22).

In one embodiment of the present invention, a biomarker may be selected from the group consisting of miR-4454 (SEQ ID NO: 1), miR-3138 (SEQ ID NO: 2), miR-3679 (SEQ ID NO: 3) and miR-4521 (SEQ ID NO: 4); however, there are no limitations on this. In an embodiment of the present invention, provided is a biomarker selected from the group consisting of miR-4454 (SEQ ID NO: 1), miR-3138 (SEQ ID NO: 2), miR-3679 (SEQ ID NO: 3) and miR-4521 (SEQ ID NO: 4). In a preferred embodiment of the present invention, a biomarker is one or more of the biomarkers selected from the group consisting of miR-4454 (SEQ ID NO: 1), miR-3138 (SEQ ID NO: 2), miR-3679 (SEQ ID NO: 3), and miR-4521 (SEQ ID NO: 4). In one embodiment of the present invention, a biomarker is miR-4454 (SEQ ID NO: 1), miR-3138 (SEQ ID NO: 2), miR-3679 (SEQ ID NO: 3), or miR-4521 (SEQ ID NO: 4), or a combination thereof.

In an embodiment of the present invention, in addition to a biomarker being selected from the group consisting of miR-4454 (SEQ ID NO: 1), miR-3138 (SEQ ID NO: 2), miR-3679 (SEQ ID NO: 3) and miR-4521 (SEQ ID NO: 4), an additional biomarker may be used, where the additional biomarker is one or more of the biomarkers selected from the group consisting of miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21), and miR-320b-2 (SEQ ID NO: 22).

The sequences of miR-4454 (SEQ ID NO: 1), miR-3138 (SEQ ID NO: 2), miR-3679 (SEQ ID NO: 3), miR-4521 (SEQ ID NO: 4), miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21) and miR-320b-2 (SEQ ID NO: 22) are as follows.

[Table 1-1]

| SEQ ID NO | Name of miR | Sequence |
|---|---|---|
| 1 | miR-4454 | ccggatccga gtcacggcac caaatttcat gcgtgtccgt gtgaagagac cacca |

(continued)

| SEQ ID NO | Name of miR | Sequence |
|---|---|---|
| 2 | miR-3138 | ccctcctcgg cacttccccc acctcactgc ccgggtgccc acaagactgt ggacagtgag gtagagggag tgccgaggag gg |
| 3 | miR-3679 | cgtggtgagg atatggcagg gaaggggagt ttccctctat tcccttcccc ccagtaatct tcatcatg |
| 4 | miR-4521 | tcgctaagg aagtcctgtg ctcagttttg tagcatcaaa actaggattt ctcttgttac |
| 5 | miR-320A | ctcccctccg ccttctcttc ccggttcttc ccggagtcgg gaaaagctgg gttgagaggg cgaaaaagga tg |
| 6 | miR-483 | gaggggggaag acgggaggaa agaagggagt ggttccatca cgcctcctca ctcctctcct cccgtcttct cctctc |
| 7 | miR-122 | ccttagcaga gctgtggagt gtgacaatgg tgtttgtgtc taaactatca aacgccatta tcacactaaa tagctactgc taggc |
| 8 | miR-125B-1 | tgcgctcctc tcagtccctg agaccctaac ttgtgatgtt taccgtttaa atccacgggt taggctcttg ggagctgcga gtcgtgct |
| 9 | miR-125B-2 | accagacttt tcctagtccc tgagaccta acttgtgagg tatttagta acatcacaag tcaggctctt gggacctagg cggagggga |
| 10 | miR-194-1 | atggtgttat caagtgtaac agcaactcca tgtggactgt gtaccaattt ccagtggaga tgctgttact tttgatggtt accaa |
| 11 | miR-194-2 | tggttcccgc cccctgtaac agcaactcca tgtggaagtg cccactggtt ccagtggggc tgctgttatc tggggcgagg gccag |
| 12 | miR-99a | cccattggca taaaccgta gatccgatct tgtggtgaag tggaccgcac aagctcgctt ctatgggtct gtgtcagtgt g |
| 13 | miR-223 | cctggcctcc tgcagtgcca cgctccgtgt atttgacaag ctgagttgga cactccatgt ggtagagtgt cagtttgtca aataccccaa gtgcggcaca tgcttaccag |
| 14 | miR-378a | agggctcctg actccaggtc ctgtgtgtta cctagaaata gcactggact tggagtcaga aggcct |
| 15 | miR-34a | ggccagctgt gagtgtttct ttggcagtgt cttagctggt tgttgtgagc aatagtaagg aagcaatcag caagtatact gccctagaag tgctgcacgt tgtggggccc |
| 16 | miR-320c-1 | aaaaatgagg ccttctcttc ccagttcttc ccagagtcag gaaaagctgg gttgagaggg tagaaaaaaa at |
| 17 | miR-320b-1 | ataaattaat ccctctcttt ctagttcttc ctagagtgag gaaaagctgg gttgagaggg caaacaaatt aa |
| 18 | miR-192 | gccgagaccg agtgcacagg gctctgacct atgaattgac agccagtgct ctcgtctccc tctggctgc caattccata ggtcacaggt atgttcgcct caatgccagc |
| 19 | miR-148a | gaggcaaagt tctgagacac tccgactctg agtatgatag aagtcagtgc actacagaac tttgtctc |
| 20 | miR-100 | cctgttgcca caaacccgta gatccgaact tgtggtatta gtccgcacaa gcttgtatct ataggtatgt gtctgttagg |

(continued)

| SEQ ID NO | Name of miR | Sequence |
|---|---|---|
| 21 | miR-144 | tggggccctg gctgggatat catcatatac tgtaagtttg cgatgagaca ctacagtata gatgatgtac tagtccgggc accccc |
| 22 | miR-320b-2 | gtctcttagg ctttctcttc ccagatttcc caaagttggg aaaagctggg ttgagagggc aaaaggaaaa a |

The sequences of miR-451a (SEQ ID NO: 23), miR-548d-3p (SEQ ID NO: 24), miR-320b (SEQ ID NO: 25), miR-1470 (SEQ ID NO: 26), miR-4258 (SEQ ID NO: 27), miR-4673 (SEQ ID NO: 28), miR-4748 (SEQ ID NO: 29), miR-4787-3p (SEQ ID NO: 30), miR-204-3p (SEQ ID NO: 31), miR-6131 (SEQ ID NO: 32), miR-6752-3p (SEQ ID NO: 33), miR-4648 (SEQ ID NO: 34), miR-6126 (SEQ ID NO: 35), miR-1228-3p (SEQ ID NO: 36), miR-1231 (SEQ ID NO: 37), miR-4286 (SEQ ID NO: 38), miR-3935 (SEQ ID NO: 39), miR-2467-3p (SEQ ID NO: 40), miR-1185-2-3p (SEQ ID NO: 41), miR-6729-3p (SEQ ID NO: 42), miR-6798-3p (SEQ ID NO: 43), miR-379-5p (SEQ ID NO: 44), miR-323a-5p (SEQ ID NO: 45), miR-665 (SEQ ID NO: 46), miR-4279 (SEQ ID NO: 47), miR-3605-5p (SEQ ID NO: 48), miR-4684-3p (SEQ ID NO: 49), miR-365b-5p (SEQ ID NO: 50), miR-6782-5p (SEQ ID NO: 51), miR-6880-3p (SEQ ID NO: 52), miR-6887-3p (SEQ ID NO: 53), miR-4433b-5p (SEQ ID NO: 54), miR-10396a-3p (SEQ ID NO: 55), miR-373-5p (SEQ ID NO: 56), miR-4539 (SEQ ID NO: 57), miR-4687-3p (SEQ ID NO: 58), miR-4763-5p (SEQ ID NO: 59), miR-4482-3p (SEQ ID NO: 60), miR-6721-5p (SEQ ID NO: 61), miR-6812-3p (SEQ ID NO: 62), miR-6815-5p (SEQ ID NO: 63), miR-6871-5p (SEQ ID NO: 64), miR-7975 (SEQ ID NO: 65), miR-11181-3p (SEQ ID NO: 66), miR-2278 (SEQ ID NO: 67), miR-3192-5p (SEQ ID NO: 68), miR-4722-3p (SEQ ID NO: 69), miR-4750-3p (SEQ ID NO: 70), miR-6804-5p (SEQ ID NO: 71), miR-6828-5p (SEQ ID NO: 72), miR-614 (SEQ ID NO: 73), miR-320c (SEQ ID NO: 74), miR-2110 (SEQ ID NO: 75), miR-3177-3p (SEQ ID NO: 76), miR-4497 (SEQ ID NO: 77), miR-210-5p (SEQ ID NO: 78), miR-6778-5p (SEQ ID NO: 79), miR-6780a-5p (SEQ ID NO: 80), miR-6801-3p (SEQ ID NO: 81) and miR-8059 (SEQ ID NO: 82) are as follows.

[Table 1-2-1]

| SEQ ID NO | Name of miR | Sequence |
|---|---|---|
| 23 | miR-451a | cttgggaatg gcaaggaaac cgttaccatt actgagttta gtaatggtaa tggttctctt gctataccca ga |
| 24 | miR-548d-3p | gagagggaag atttaggttg gtgcaaaagt aattgtggtt tttgccattg aaagtaatgg caaaaaccac agtttctttt gcaccaacct aataaaa |
| 25 | miR-320b | ataaattaat ccctctcttt ctagttcttc ctagagtgag gaaaagctgg gttgagaggg caaacaaatt aa |
| 26 | miR-1470 | gccctccgcc cgtgcacccc ggggcaggag accccgcggg acgcgccgag gtagggggga c |
| 27 | miR-4258 | acgcccccg ccccgccacc gccttggagg ctgacctctt actttcggtc ggtcttcttc cctgggcttg gtttgggggc ggggagtgt c |
| 28 | miR-4673 | gtccaggcag gagccggact ggacctcagg gaagaggctg accggccc tcttgcggc |
| 29 | miR-4748 | tggctggctg aggtttgggg aggatttgct ggtgctagag aggaaagcag accctacccca accccacgcc ctactacagc ca |
| 30 | miR-4787-3p | cggtccagac gtggcggggg tggcggcggc atcccggacg gcctgtgagg gatgcgccgc ccactgcccc gcgccgcctg accg |
| 31 | miR-204-3p | ggctacagtc tttcttcatg tgactcgtgg acttcccttt gtcatcctat gcctgagaat atatgaagga ggctgggaag gcaaagggac gttcaattgt catcactggc |
| 32 | miR-6131 | tcccgcattc cctctgcttt ggtcaggtgg tgccctcctt ccatgggtag agccagagat ggtgggttct ggctggtcag atgggagtgg acagagaccc ggggtcctc |

(continued)

| SEQ ID NO | Name of miR | Sequence |
|---|---|---|
| 33 | miR-6752-3p | atggaggggg gtgtggagcc agggggccca ggtctacagc ttctccccgc tccctgcccc catactccca g |
| 34 | miR-4648 | tgtgggactg caaatgggag ctcagcacct gcctgccacc cacgcagacc agcccctgct ctgttcccac ag |
| 35 | miR-6126 | agcctgtggg aaagagaaga gcagggcagg gtgaaggccc ggcggagaca ctctgcccac cccacaccct gcctatgggc cacacagct |
| 36 | miR-1228-3p | gtgggcgggg gcaggtgtgt ggtgggtggt ggcctgcggt gagcagggcc ctcacacctg cctcgccccc cag |
| 37 | miR-1231 | gtcagtgtct gggcggacag ctgcaggaaa gggaagacca aggcttgctg tctgtccagt ctgccaccct accctgtctg ttcttgccac ag |
| 38 | miR-4286 | tacttatggc accccactcc tggtaccata gtcataagtt aggagatgtt agagctgtga gtaccatgac ttaagtgtgg tggcttaaac atg |
| 39 | miR-3935 | ggatgtgttc ctgtcccaga aggagctgat ggttgtatct atgaaggtaa gcatttttgt agatacgagc accagccacc ctaagcaaag gcagagaatg ctta |
| 40 | miR-2467-3p | ggacaggcac ctgaggctct gttagccttg gctctgggtc ctgctcctta gagcagaggc agagaggctc agggtctgtc t |
| 41 | miR-1L85-2-3p | tttggtactt aaagagagga tacccttgt atgttcactt gattaatggc gaatatacag ggggagactc tcatttgcgt atcaaa |
| 42 | miR-6729-3p | gagggtgggc gagggcggct gagcggctcc atcccccggc ctgctcatcc ccctcgccct ctcag |
| 43 | miR-6798-3p | ggcagccagg gggatgggcg agcttgggcc cattcctttc cttaccctac cccccatccc cctgtag |
| 44 | miR-379-5p | agagatggta gactatggaa cgtaggcgtt atgatttctg acctatgtaa catggtccac taactct |
| 45 | miR-323a-5p | ttggtacttg gagagaggtg gtccgtggcg cgttcgcttt atttatggcg cacattacac ggtcgacctc tttgcagtat ctaatc |

[Table 1-2-2]

| 46 | miR-665 | tctcctcgag gggtctctgc ctctacccag gactctttca tgaccaggag gctgaggccc ctcacaggcg gc |
|---|---|---|
| 47 | miR-4279 | tgctctgtgg agctgaggag cagattctct ctctctcctc ccggcttcac ctcctgag |
| 48 | miR-3605-5p | actttatacg tgtaattgtg atgaggatgg atagcaagga agccgctccc acctgaccct cacggcctcc gtgttacctg tcctctaggt gggacgctcg |
| 49 | miR-4684-3p | gcaccagggg tacctctcta ctgacttgca acatacattt gtcttggtgt gttgcaagtc ggtggagacg tacccttggt gc |
| 50 | miR-365b-5p | agagtgttca aggacagcaa gaaaaatgag ggactttcag gggcagctgt gttttctgac tcagtcataa tgcccctaaa aatccttatt gttcttgcag tgtgcatcgg g |
| 51 | miR-6782-5p | tggggtaggg gtgggggaat tcaggggtgt cgaactcatg gctgccacct ttgtgtcccc atcctgcag |

(continued)

| 52 | miR-6880-3p | gagggtggtg gaggaagagg gcagctccca tgactgcctg accgccttct ctcctccccag |
| 33 | miR-6887-3p | gagaatgggg ggacagatgg agaggacaca ggctggcact gaggtcccct ccactttcct cctag |
| 54 | miR-4433b-5p | tgtgttccct atcctcctta tgtcccaccc ccactcctgt ttgaatattt caccagaaac aggagtgggg ggtgggacgt aaggaggatg ggggaaagaa ca |
| 55 | miR-10396a-3p | ggcggggctc ggagccgggc ttcggccggg ccccgggccc tcgaccggg |
| 56 | miR-373-5p | gggatactca aaatgggggc gctttccttt ttgtctgtac tgggaagtgc ttcgattttg gggtgtccc |
| 57 | miR-4539 | tgagctgagc tctgctgtgc tgtgctgagc agggctgagc tgaactgggc tgagctgggc |
| 58 | miR-4687-3p | acctgaggag ccagccctcc tcccgcaccc aaacttggag cacttgacct ttggctgttg gaggggcag gctcgcgggt |
| 59 | miR-4763-5p | aggcaggggc tggtgctggg cggg |
| 60 | miR-4482-3p | agtgagcaac ccagtgggct atggaaatgt gtggaagatg gcatttctat ttctcagtgg ggctcttacc |
| 61 | miR-6721-5p | ccctcatctc tgggcagggg cttattgtag gagtctctga agagagctgt ggactgacct gctttaaccc ttccccaggt tcccatt |
| 62 | miR-6812-3p | tgaggatggg gtgagatggg gaggagcagc cagtcctgtc tcaccgctct tcccctgacc ccag |
| 63 | miR-6815-5p | cactgtaggt ggcgccggag gagtcatttc ccatcactaa tggcttctct tgcacacccag |
| 64 | miR-6871-5p | ctcctcatgg gagttcgggg tggttgctgg aggtcagcac cctgtggctc ccacag |
| 65 | miR-7975 | gtgcaaagag caggaggaca ggggatttat ctcccaaggg aggtcccctg atcctagtca cggcacca |
| 66 | miR-11181-3p | gtctgaccaa cctcctcccg caaactgttt tggcctctga aggaggagga ggtcaggcat ggt |
| 67 | miR-2278 | gtgctgcagg tgttggagag cagtgtgtgt tgcctgggga ctgtgtggac tggtatcacc cagacagctt gcactgactc cagaccctgc cgtcat |
| 68 | miR-3192-5p | ggaagggatt ctgggaggtt gtagcagtgg aaaaagttct tttcttcctc tgatcgccct ctcagctctt ccttct |
| 69 | miR-4722-3p | ggcaggaggg ctgtgccagg ttggctgggc caggcctgac ctgccagcac ctccctgcag |
| 70 | miR-4750-3p | cgctcgggcg gaggtggttg agtgccgact ggcgcctgac ccacccctc ccgcag |
| 71 | miR-6804-5p | ggatgtgagg gtgtcagcag gtgacggtgg gggccacgct gacagccgca cctgcctctc acccacag |
| 72 | miR 6828-5p | ggctcaggaa gcaagagaac cctgtggtct aacctttccc atctgctctc ttgttcccag |

[Table 1-2-3]

| 73 | miR-614 | tctaagaaac gcagtggtct ctgaagcctg caggggcagg ccagccctgc actgaacgcc tgttcttgcc aggtggcaga aggttgctgc |
|---|---|---|
| 74 | miR-320c | aggagcactg ggtatgtaca aataaaatgg catggcaggc cttctctttc cagttcttcc cagaattggg aaaagctggg ttgacagggt aaaaaaaaga aaaacaaat |
| 75 | miR-2110 | caggggtttg gggaaacggc cgctgagtga ggcgtcggct gtgtttctca ccgcggtcttttcctcccac tcttg |
| 76 | miR-3177-3p | ccacgtgcca tgtgtacaca cgtgccaggc gctgtcttga gacattcgcg cagtgcacgg cactggggac acgtggcact gg |
| 77 | miR-4497 | acctccggga cggctgggcg ccggcggccg ggagatccgc gcttcctgaa tcccggccgg cccgcccggc gcccgtccgc ccgcgggtc |
| 78 | miR-210-5p | acccggcagt gcctccaggc gcaggggcagc ccctgcccac cgcacactgc gctgccccag acccactgtg cgtgtgacag cggctgatct gtgcctgggc agcgcgaccc |
| 79 | miR-6778-5p | gttcaagtgg gaggacagga ggcaggtgtg gttggaggaa gcagcctgaa cctgcctccc tgacattcca cag |
| 80 | miR-6780a-5p | gacacttggg agggaagaca gctggagagt atggtcacag cagcatcctc ctctgttttc tttcctag |
| | | |
| 81 | miR-6801-3p | tggcctggtc agaggcagca ggaaatgaga gttagccagg agctttgcat actcacccct gccactcact ggccccccag |
| 82 | miR-8059 | tacaggtgca ggggaactgt agatgaaaag gcttggcact tgagggaaag cctcagttca ttctcattt gctcacctgt t |

In one embodiment of the present invention, a biomarker is selected from the group consisting of miR-451a (SEQ ID NO: 23), miR-4787-3p (SEQ ID NO: 30), miR-6126 (SEQ ID NO: 35), miR-1228-3p (SEQ ID NO: 36), miR-6798-3p (SEQ ID NO: 43), miR-7975 (SEQ ID NO: 65), miR-614 (SEQ ID NO: 73), miR-6801-3p (SEQ ID NO: 81), miR-657 (SEQ ID NO: 83), miR-921 (SEQ ID NO: 84), miR-4451 (SEQ ID NO: 85), miR-3059-3p (SEQ ID NO: 86), miR-3619-3p (SEQ ID NO: 87), miR-4646-5p (SEQ ID NO: 88), miR-525-5p (SEQ ID NO: 89), miR-4444 (SEQ ID NO: 90), miR-4458 (SEQ ID NO: 91), miR-4746-3p (SEQ ID NO: 92), miR-1292-3p (SEQ ID NO: 93), miR-6133 (SEQ ID NO: 94), miR-6754-3p (SEQ ID NO: 95), miR-365a-3p (SEQ ID NO: 96), miR-365b-3p (SEQ ID NO: 97), miR-6748-3p (SEQ ID NO: 98), miR-6892-3p (SEQ ID NO: 99), miR-8083 (SEQ ID NO: 100), miR-4724-5p (SEQ ID NO: 101), miR-6884-5p (SEQ ID NO: 102), miR-7156-3p (SEQ ID NO: 103), miR-19b-3p (SEQ ID NO: 104), miR-518e-3p (SEQ ID NO: 105), miR-646 (SEQ ID NO: 106), miR-298 (SEQ ID NO: 107), miR-1234-3p (SEQ ID NO: 108), miR-2115-5p (SEQ ID NO: 109), miR-3142 (SEQ ID NO: 110), miR-3179 (SEQ ID NO: 111), miR-3190-5p (SEQ ID NO: 112), miR-3675-3p (SEQ ID NO: 113), miR-4441 (SEQ ID NO: 114), miR-4475 (SEQ ID NO: 115), miR-6718-5p (SEQ ID NO: 116), miR-6743-3p (SEQ ID NO: 117), miR-6894-3p (SEQ ID NO: 118), miR-7112-5p (SEQ ID NO: 119), miR-12116 (SEQ ID NO: 120), miR-518d-3p (SEQ ID NO: 121), miR-145-5p (SEQ ID NO: 122), miR-6801-5p (SEQ ID NO: 123), miR-127-3p (SEQ ID NO: 124) and miR-4731-3p (SEQ ID NO: 125).

The sequences of miR-451a (SEQ ID NO: 23), miR-4787-3p (SEQ ID NO: 30), miR-6126 (SEQ ID NO: 35), miR-1228-3p (SEQ ID NO: 36), miR-6798-3p (SEQ ID NO: 43), miR-7975 (SEQ ID NO: 65), miR-614 (SEQ ID NO: 73), miR-6801-3p (SEQ ID NO: 81), miR-657 (SEQ ID NO: 83), miR-921 (SEQ ID NO: 84), miR-4451 (SEQ ID NO: 85), miR-3059-3p (SEQ ID NO: 86), miR-3619-3p (SEQ ID NO: 87), miR-4646-5p (SEQ ID NO: 88), miR-525-5p (SEQ ID NO: 89), miR-4444 (SEQ ID NO: 90), miR-4458 (SEQ ID NO: 91), miR-4746-3p (SEQ ID NO: 92), miR-1292-3p (SEQ ID NO: 93), miR-6133 (SEQ ID NO: 94), miR-6754-3p (SEQ ID NO: 95), miR-365a-3p (SEQ ID NO: 96), miR-365b-3p (SEQ ID NO: 97), miR-6748-3p (SEQ ID NO: 98), miR-6892-3p (SEQ ID NO: 99), miR-8083 (SEQ ID NO: 100), miR-4724-5p (SEQ ID NO: 101), miR-6884-5p (SEQ ID NO: 102), miR-7156-3p (SEQ ID NO: 103), miR-19b-3p (SEQ ID NO: 104), miR-518e-3p (SEQ ID NO: 105), miR-646 (SEQ ID NO: 106), miR-298 (SEQ ID NO: 107), miR-1234-3p (SEQ ID NO: 108), miR-2115-5p (SEQ ID NO: 109), miR-3142 (SEQ ID NO: 110), miR-3179 (SEQ ID NO: 111), miR-3190-5p (SEQ ID NO: 112), miR-3675-3p (SEQ ID NO: 113), miR-4441 (SEQ ID NO: 114), miR-4475 (SEQ ID NO: 115), miR-6718-5p (SEQ ID NO: 116), miR-6743-3p (SEQ ID NO: 117), miR-6894-3p (SEQ ID NO: 118), miR-7112-5p (SEQ ID NO: 119), miR-12116 (SEQ ID NO: 120), miR-518d-3p (SEQ ID NO: 121), miR-145-5p (SEQ ID NO: 122), miR-6801-5p (SEQ ID NO: 123), miR-127-3p (SEQ ID NO: 124) and miR-4731-3p (SEQ ID NO: 125) are as follows.

[Table 1-3-1]

| SEQ ID NO | Name of miR | Sequence |
|---|---|---|
| 23 | miR-451a | cttgggaatg gcaaggaaac cgttaccatt actgagttta gtaatggtaa tggttctctt gctataccca ga |
| 30 | miR-4787-3p | cggtccagac gtggcggggg tggcggcggc atcccggacg gcctgtgagg gatgcgccgc ccactgcccc gcgccgcctg accg |
| 35 | miR-6126 | agcctgtggg aaagagaaga gcagggcagg gtgaaggccc ggcggagaca ctctgcccac cccacaccct gcctatgggc cacacagct |
| 36 | miR-1228-3p | gtgggcgggg gcaggtgtgt ggtgggtggt ggcctgcggt gagcagggcc ctcacacctg cctcgccccc cag |
| 43 | miR-6798-3p | ggcagccagg gggatgggcg agcttgggcc cattcctttc cttaccctac cccccatccc cctgtag |
| 65 | miR-7975 | gtgcaaagag caggaggaca ggggatttat ctcccaaggg aggtccctg atcctagtca cggcacca |
| 73 | miR-614 | tctaagaaac gcagtggtct ctgaagcctg caggggcagg ccagccctgc actgaacgcc tgttcttgcc aggtggcaga aggttgctgc |
| 81 | miR-6801-3p | tggcctggtc agaggcagca ggaaatgaga gttagccagg agctttgcat actcacccct gccactcact ggcccccag |
| 83 | miR-657 | gtgtagtaga gctaggagga gagggtcctg gagaagcgtg gaccggtccg ggtgggttcc ggcaggttct caccctctct aggccccatt ctcctctg |
| 84 | miR-921 | actagtgagg gacagaacca ggattcagac tcaggtccat gggcctggat cactgg |
| 85 | miR-4451 | tctgtacctc agctttgctc ccaaccaacc acttccacat gttttgctgg tagagctgag gacagc |
| 86 | miR-3059-3p | aggtggtaca cccttcctc tctgccccat agggtgtagc tctaactacc ctctagggaa gagaaggttg ggtgaacagc ct |
| 87 | miR-3619-3p | acggcatctt tgcactcagc aggcaggctg gtgcagcccg tggtggggga ccatcctgcc tgctgtgggg taaggacggc tgt |
| 88 | miR-4646-5p | actgggaaga ggagctgagg gacattgcgg agagggtctc acattgtccc tctcccttcc cag |
| 89 | miR-525-5p | ctcaagctgt gactctccag agggatgcac tttctcttat gtgaaaaaaa agaaggcgct tccctttaga gcgttacggt ttggg |
| 90 | miR-4444 | gtgacgactg gccccgcctc ttcctctcgg tcccatattg aactcgagtt ggaagaggcg agtccggtct caaa |
| 91 | miR-4458 | gagcgcacag aggtaggtgt ggaagaaagt gaaacactat tttaggtttt agttacactc tgctgtggtg tgctg |
| 92 | miR-4746-3p | gtgtctgtgc cggtcccagg agaacctgca gaggcatcgg gtcagcggtg ctcctgcggg ccgacactca c |

(continued)

| SEQ ID NO | Name of miR | Sequence |
|---|---|---|
| 93 | miR-1292-3p | cctgggaacg ggttccggca gacgctgagg ttgcgttgac gctcgcgccc cggctcccgt tccagg |
| 94 | miR-6133 | ggaatgtcac ctgtgtgttt tctctgcatg ccctcttcat tgttctgctg aagactggtc tcttcatgtg tgagggagga ggttgggtat tgagggaaaa caggggc |
| 95 | miR-6754-3p | ggctgccagg gaggctggtt tggaggagtc tggtggcctg ttctcttcac ctgcctctgc ctgcag |
| 96 | miR-365a-3p | accgcaggga aaatgaggga cttttggggg cagatgtgtt tccattccac tatcataatg cccctaaaaa tccttattgc tcttgca |
| 97 | miR-365b-3p | agagtgttca aggacagcaa gaaaaatgag ggactttcag gggcagctgt gttttctgac tcagtcataa tgcccctaaa aatccttatt gttcttgcag tgtgcatcgg g |
| 98 | miR-6748-3p | tggtgtgtgg gtgggaagga ctggatttga aatggtccca ctcctgacga tcctgtccct gtctcctaca g |

[Table 1-3-2]

| 99 | miR-6892-3p | gtaagggacc ggagagtagg aaaagcaggg ctcagggcca gagagactgg gcatagaact aaggaggatg gtgtcctcct gactgcatct ctcttccctc tcccacccct tgcag |
|---|---|---|
| 100 | miR-8083 | cgaggggggtg caggacttga cggctgcaac tgtgcggccg gccacaccgt cacaccgggt gcagcgtctg tcattctgta ccctcatct |
| 101 | miR-4724-5p | acgcaaaatg aactgaacca ggagtgagct tcgtgtacat tatctattag aaaatgaagt accttctggt tcagctagtc cctgtgcgt |
| 102 | miR 6884-5p | cccgcagagg ctgagaaggt gatgttggct caagaaaggg agatagatgg tagcccatca cctttccgtc tcccctag |
| 103 | miR-7156-3p | ttgttctcaa actggctgtc agagtgtgca tggcaggctg cagccacttg gggaactggt cactgttcta tggttagttt tgcaggtttg catccagctg tgtgatattc tgctgtgcaa atccatgcaa aactgactgt ggtagtg |
| 104 | miR-19b-3p | cactgttcta tggttagttt tgcaggtttg catccagctg tgtgatattc tgctgtgcaa atccatgcaa aactgactgt ggtagtg |
| 105 | miR-518e-3p | tctcaggctg tgaccctcta gagggaagcg cttctgttg gctaaaagaa aagaaagcgc ttcccttcag agtgttaacg ctttgaga |
| 106 | miR-646 | gatcaggagt ctgccagtgg agtcagcaca cctgctttc acctgtgatc ccaggagagg aagcagctgc ctctgaggcc tcaggctcag tggc |
| 107 | miR-298 | tcaggtcttc agcagaagca gggaggttct cccagtggtt ttccttgact gtgaggaact agcctgctgc tttgctcagg agtgagct |
| 108 | miR-1234-3p | gtgagtgtgg ggtggctggg gcggggggggg cccggggacg gcttgggcct gcctagtcgg cctgaccacc caccccacag |
| 109 | miR-2115-5p | actgtcatcc cactgcttcc agcttccatg actcctgatg gaggaatcac atgaattcat cagaattcat ggaggctaga agcagtatga ggatcattta |
| 110 | miR-3142 | ttcagaaagg cctttctgaa ccttcagaaa ggctgctgaa tcttcagaaa ggcctttctg aaccttcaga aaggctgctg aa |

(continued)

| 111 | miR-3179 | agaaggggtg aaatttaaac gt |
| 112 | miR-3190-5p | ctggggtcac ctgtctggcc agctacgtcc ccacggccct tgtcagtgtg gaaggtagac ggccagagag gtgaccccgg |
| 113 | miR-3675-3p | ggatgataag ttatgggggct tctgtagaga tttctatgag aacatctcta aggaactccc ccaaactgaa ttc |
| 114 | miR-4441 | cagagtctcc ttcgtgtaca gggaggagac tgtacgtgag agatagtcag atccgcatgt tagagcagag tctccttcgt gtacagggag gagattgtac |
| 115 | miR-4475 | atctcaatga gtgtgtggtt ctaaatgact catagtcaag ggaccaagca ttcattatgaa |
| 116 | miR-6718-5p | agtgaatccc tagtggtcag agggcttatg atatattgtg agagccatgt cataagcctt ttggccacta gggattcaat |
| 117 | miR-6743-3p | gggtaaaggg gcagggacgg gtggccccag gaagaagggc ctggtggagc cgctcttctc cctgcccaca g |
| 118 | miR-6894-3p | caagaaggag gatggagagc tgggccagac atgctcttgc ctgccctctt cctccag |
| 119 | miR-7112-5p | acgggcaggg cagtgcaccc tgcaggtgag agcgggaaca cctgcatcac agcctttggc cctag |
| 120 | miR-12116 | gcctttggtt cttcttaggc ttccccctcc tcctgccccc tggcctgctt gcctccaggc caggcggcag gaggaggggg aagcctaaga agaacccaga gc |
| 121 | miR-518d-3p | tcccatgctg tgaccctcta gagggaagca ctttctgttg tctgaaagaa accaaagcgc ttccctttgg agcgttacgg tttgaga |
| 122 | miR-145-5p | caccttgtcc tcacggtcca gtttttcccag gaatcccttia gatgctaaga tgggggattcc |
| 123 | miR-6801-5p | tggcctggtc agaggcagca ggaaatgaga gttagccagg agctttgcat actcacccct gccactcact ggcccccag |
| 124 | miR-127-3p | tgtgatcact gtctccagcc tgctgaagct cagagggctc tgattcagaa agatcatcgg atccgtctga gcttggctgg tcggaagtct catcatc |
| 125 | miR-4731-3p | ccctgccagt gctggggggcc acatgagtgt gcagtcatcc acacacaagt ggcccccaac actggcaggg |

An isoform or variant may be present in miRNA. For example, a portion of the bases in the sequences of the biomarkers exemplified in the aforementioned, for example 1 or more bases, 1 to 5 bases, 1 to 3 bases, 1 to 2 bases, or 1 base may be substituted or deleted; or a base containing no biomarker (for example, 1 or more bases, 1 to 5 bases, 1 to 3 bases, 1 to 2 bases, or 1 base) may be added or inserted. Moreover, a base may also have 90% or more, 91% or more, 92% or more, 93% or more, 94% or more, 95% or more, 96% or more, 97% or more, 98% or more, or about 99% or more identity with a biomarker. The identity can be assessed by means of e.g. BLAST and the like. A biomarker described in the present specification may also include mutants and variants thereof, except for the case where such understanding is particularly inconsistent.

In the present invention, the biomarkers exemplified in the aforementioned can be obtained from a biological sample of a subject. Examples of biological samples of which a biomarker of the present invention is obtainable include blood, serum, plasma, saliva, sweat, tears, faeces, urine, an organ specimen, skin, tissue exudate, hair etc.; however, there are no limitations on this. In one embodiment of the present invention, a biological sample of which a biomarker of the present invention is obtainable is selected from blood, serum, plasma, saliva, sweat, tears, faeces, urine, and an organ specimen. In an embodiment of the present invention, provided is a biomarker obtainable from a biological sample selected from the group consisting of blood, serum, plasma, whole blood, a hemolysate of corpuscles, fractionated materials or processed materials of blood, saliva, sweat, tears, faeces, urine, and an organ specimen. In one embodiment of the present invention, the biological sample is blood. In one embodiment of the present invention, the biological sample is serum. In one embodiment of the present invention, the biological sample is plasma. In one embodiment of the present invention, the biological sample is saliva. In one embodiment of the present invention, the biological sample is sweat. In one embodiment of the present invention, the biological sample is tears. In one embodiment of the present invention, the biological sample is faeces. In one embodiment of the present invention, the biological sample is urine. In one embodiment of the present invention, the biological sample is an organ specimen.

In an embodiment of the present invention, the aforementioned biomarkers are biomarkers for liver disease. In one embodiment of the present invention, the aforementioned biomarkers are used in the detection of liver disease, e.g. may be used in a diagnosis of the presence or absence of liver disease or the risk thereof, an assessment or monitoring of the degree of progression of liver disease, and/or an assessment of the degree of success of a procedure performed for treatment, or a monitoring of therapeutic effects etc.; however, there are no limitations on this. In one embodiment of the present invention, the aforementioned biomarker is used in the detection of liver disease. In one preferred embodiment of the present invention, the aforementioned biomarker is used in a diagnosis of the presence or absence of liver disease or the risk thereof. In one preferred embodiment of the present invention, the aforementioned biomarker is used in an assessment of the degree of progression of liver disease. In one preferred embodiment of the present invention, the aforementioned biomarker is used in an assessment of the degree of success of a procedure performed for treatment. The assessment of the degree of success of a procedure performed for treatment includes an assessment for, e.g. whether a candidate compound of a therapeutic drug has achieved a preferable therapeutic effect, the presence or absence of a therapeutic response, and the tolerance; however, there are no limitations on this.

In one embodiment of the present invention, provided is a biomarker used in the detection of liver disease. In one preferred embodiment of the present invention, provided is a biomarker for use in diagnosing the presence or absence of liver disease or the risk thereof. In one preferred embodiment of the present invention, provided is a biomarker for use in assessing the degree of progression of liver disease. In one preferred embodiment of the present invention, provided is a biomarker for use in assessing the degree of success of a procedure performed for treatment. The staging in relation to the degree of progression of liver disease is mentioned below.

Liver diseases configured as subjects of the liver disease non-invasive biomarker of the present invention include hepatitis, hepatic fibrosis, fatty liver, hepatic cirrhosis and liver cancer; however, there are no limitations on this. In one embodiment of the present invention, a liver disease configured as a subject of the liver disease non-invasive biomarker is selected from the group consisting of hepatitis, hepatic fibrosis, fatty liver, hepatic cirrhosis and liver cancer. In a preferred embodiment of the present invention, a liver disease configured as a subject of the liver disease non-invasive biomarker is hepatic cirrhosis. In a preferred embodiment of the present invention, a liver disease configured as a subject of the liver disease non-invasive biomarker is a viral liver disease, e.g. hepatitis C and hepatitis B.

In another preferred embodiment of the present invention, a liver disease configured as a subject of the liver disease non-invasive biomarker is non-alcoholic steatohepatitis (NASH).

In one embodiment of the present invention, provided is a biomarker where a liver disease configured as a subject of the liver disease non-invasive biomarker is selected from the group consisting of hepatitis, hepatic fibrosis, fatty liver, hepatic cirrhosis and liver cancer. In a preferred embodiment of the present invention, provided is a biomarker where a liver disease configured as a subject of the liver disease non-invasive biomarker is hepatic cirrhosis. In a preferred embodiment of the present invention, provided is a biomarker where a liver disease configured as a subject of the liver disease non-invasive biomarker is a viral liver disease, e.g. hepatitis C and hepatitis B.

In another preferred embodiment of the present invention, provided is a biomarker where a liver disease configured as a subject of the liver disease non-invasive biomarker is non-alcoholic steatohepatitis (NASH).

< Method for detecting liver disease >

The present invention provides a method for detecting liver disease, where the method includes measuring a level of a biomarker in a biological sample from a subject, and comparing a measurement value of the biomarker with a reference value. In one embodiment of the present invention, the aforementioned method for detecting liver disease includes, e.g. a diagnosis of the presence or absence of liver disease or the risk thereof, an assessment of the degree of progression of liver disease, and/or an assessment of the degree of success of a procedure performed for treatment etc.; however, there are no limitations on this. In one embodiment of the present invention, the aforementioned method for detecting liver disease may be used in a diagnosis of the presence or absence of liver disease or the risk thereof, an assessment of the degree of progression of liver disease, and/or an assessment of the degree of success of a procedure performed for treatment. In one embodiment of the present invention, the aforementioned method for detecting liver disease is used in a diagnosis of the presence or absence of liver disease or the risk thereof. In one embodiment of the present invention, the aforementioned method for detecting liver disease is used in an assessment of the degree of progression of liver disease. In one embodiment of the present invention, the aforementioned method for detecting liver disease is used in an assessment of the degree of success of a procedure performed for treatment. The assessment of the degree of success of a procedure performed for treatment includes an assessment for, e.g. whether a candidate compound of a therapeutic drug has achieved a preferable therapeutic effect, the presence or absence of a therapeutic response, and the tolerance; however, there are no limitations on this.

In one embodiment of the present invention, a biomarker used in the aforementioned method for detecting liver disease is one or more biomarkers selected from the group consisting of SEQ ID NOs: 1 to 125. In one embodiment of the present invention, provided is a method for assessing the presence or absence of liver disease or the risk or degree of progression thereof in a subject, and/or the degree of success of a procedure performed for treatment, the method including measuring a level of a biomarker in a biological sample from the subject, and comparing a measurement value of the biomarker with a reference value, where the biomarker is one or more of the biomarkers selected from the group consisting of SEQ ID NOs: 1 to 125.

In one embodiment of the present invention, the biomarker is one or more of the biomarkers selected from the group consisting of SEQ ID NOs: 1 to 82. In one embodiment of the present invention, provided is a method for assessing the presence or absence of liver disease or the risk or degree of progression thereof in a subject, and/or the degree of success of a procedure performed for treatment, the method including measuring a level of a biomarker in a biological sample from the subject, and comparing a measurement value of the biomarker with a reference value, where the biomarker is one or more of the biomarkers selected from the group consisting of SEQ ID NOs: 1 to 82.

In one embodiment of the present invention, the biomarker may be selected from the group consisting of miR-4454 (SEQ ID NO: 1), miR-3138 (SEQ ID NO: 2), miR-3679 (SEQ ID NO: 3) and miR-4521 (SEQ ID NO: 4); however, there are no limitations on this. In a preferred embodiment of the present invention, a biomarker used in the aforementioned method for detecting liver disease is one or more of the biomarkers selected from the group consisting of miR-4454 (SEQ ID NO: 1), miR-3138 (SEQ ID NO: 2), miR-3679 (SEQ ID NO: 3) and miR-4521 (SEQ ID NO: 4). In one embodiment of the present invention, a biomarker used in the aforementioned method for detecting liver disease is miR-4454 (SEQ ID NO: 1), miR-3138 (SEQ ID NO: 2), miR-3679 (SEQ ID NO: 3), or miR-4521 (SEQ ID NO: 4), or a combination thereof.

In one embodiment of the present invention,

provided is a method for assessing the presence or absence of liver disease or the risk or degree of progression thereof in a subject, and/or the degree of success of a procedure performed for treatment, the method including
measuring a level of a biomarker in a biological sample from the subject, and
comparing a measurement value of the biomarker with a reference value, where
the biomarker is one or more of the biomarkers selected from the group consisting of miR-4454 (SEQ ID NO: 1), miR-3138 (SEQ ID NO: 2), miR-3679 (SEQ ID NO: 3) and miR-4521 (SEQ ID NO: 4).

In an embodiment of the present invention, in addition to a biomarker being one or more of the biomarkers selected from the group consisting of miR-4454 (SEQ ID NO: 1), miR-3138 (SEQ ID NO: 2), miR-3679 (SEQ ID NO: 3) and miR-4521 (SEQ ID NO: 4), an additional biomarker may be used. In an embodiment of the present invention, the aforementioned method for detecting liver disease may further include measuring a level of an additional biomarker in a biological sample from a subject, and comparing the measurement value of the additional biomarker with a reference value.

In one embodiment of the present invention,

provided is a method for assessing the presence or absence of liver disease or the risk or degree of progression thereof in a subject, and/or the degree of success of a procedure performed for treatment, the method including
measuring a level of a biomarker in a biological sample from the subject, and
comparing a measurement value of the biomarker with a reference value, where
the biomarker is one or more of the biomarkers selected from the group consisting of miR-4454 (SEQ ID NO: 1), miR-3138 (SEQ ID NO: 2), miR-3679 (SEQ ID NO: 3) and miR-4521 (SEQ ID NO: 4), and the method further includes
measuring a level of an additional biomarker in a biological sample from a subject, and
comparing the measurement value of the additional biomarker with a reference value.

In the aforementioned method, an additional biomarker may be selected from the group consisting of miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21) and miR-320b-2 (SEQ ID NO: 22); however, there are no limitations on this. In one embodiment of the present invention, provided is the aforementioned method utilizing an additional biomarker, where the additional biomarker is one or more of the biomarkers selected from the group consisting of miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21) and miR-320b-2 (SEQ ID NO: 22).

In another embodiment of the present invention, a biomarker used in the aforementioned method is one or more of the biomarkers selected from the group consisting of SEQ ID NOs: 23, 30, 35, 36, 43, 65, 73, 81, 83 to 125. In one embodiment of the present invention, provided is a method for assessing the presence or absence of liver disease or the risk or degree of progression thereof in a subject, and/or the degree of success of a procedure performed for treatment, the method including measuring a level of a biomarker in a biological sample from the subject, and comparing a measurement value of the biomarker with a reference value, where the biomarker is one or more of the biomarkers selected from the group consisting of SEQ ID NOs: 1 to 125.

The present invention moreover provides a method for detecting liver disease with high accuracy, which includes:

measuring a level of a biomarker 1 in a biological sample from a subject;
comparing a measurement value of the biomarker 1 with a reference value to obtain a parameter 1;
measuring a level of at least one additional biomarker N (where N is an integer of 2 or more) in the biological sample from the subject;
comparing a measurement value of the additional biomarker N with a reference value to obtain a parameter N; and
obtaining a result in the form of one set of at least two parameters made up of a combination of the parameter 1 and one or more of the parameters N; and

the result in the form of one set of at least two parameters indicating the presence or absence of liver disease or the risk or degree of progression thereof in the subject, and/or the degree of success of a procedure performed for treatment.

In the aforementioned method for detecting liver disease with high accuracy, the result in the form of one set of at least two parameters can be evaluated using a computer program. In one embodiment of the present invention, provided is a method for detecting liver disease with high accuracy, which includes:

measuring a level of a biomarker 1 in a biological sample from a subject;
comparing a measurement value of the biomarker 1 with a reference value to obtain a parameter 1;
measuring a level of at least one additional biomarker N (where N is an integer of 2 or more) in the biological sample from the subject;
comparing a measurement value of the additional biomarker N with a reference value to obtain a parameter N;
obtaining a result in the form of one set of at least two parameters made up of a combination of the parameter 1 and one or more of the parameters N; and
the result in the form of one set of at least two parameters indicating the presence or absence of liver disease or the risk or degree of progression thereof in the subject, and/or the degree of success of a procedure performed for treatment, where
the result in the form of one set of at least two parameters is evaluated using a computer program.

In the aforementioned method for detecting liver disease with high accuracy, the biomarker 1 and the at least one additional biomarker are one or more of the biomarkers selected from the group consisting of SEQ ID NOs: 1 to 125. In one embodiment of the present invention, provided is the aforementioned method for detecting liver disease with high accuracy, where the biomarker 1 and the at least one additional biomarker are one or more of the biomarkers selected from the group consisting of SEQ ID NOs: 1 to 125.

In the aforementioned method for detecting liver disease with high accuracy, the biomarker 1 and the at least one additional biomarker are one or more of the biomarkers selected from the group consisting of SEQ ID NOs: 1 to 82. In one embodiment of the present invention, provided is the aforementioned method for detecting liver disease with high accuracy, where the biomarker 1 and the at least one additional biomarker are one or more of the biomarkers selected from the group consisting of SEQ ID NOs: 1 to 82.

In the aforementioned method for detecting liver disease with high accuracy, the biomarker 1 and the at least one additional biomarker may be selected from the group consisting of miR-4454 (SEQ ID NO: 1), miR-3138 (SEQ ID NO: 2), miR-3679 (SEQ ID NO: 3), miR-4521 (SEQ ID NO: 4), miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21) and miR-320b-2 (SEQ ID NO: 22); however, there are no limitations on this. In one embodiment of the present invention, provided is the aforementioned method for detecting liver disease with high accuracy, where the biomarker 1 and the at least one additional biomarker are selected from the group consisting of miR-4454 (SEQ ID NO: 1), miR-3138 (SEQ ID NO: 2), miR-3679 (SEQ ID NO: 3), miR-4521 (SEQ ID NO: 4), miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21) and miR-320b-2 (SEQ ID NO: 22).

In another embodiment of the present invention, a biomarker used in the aforementioned method is one or more of the biomarkers selected from the group consisting of SEQ ID NOs: 23, 30, 35, 36, 43, 65, 73, 81, 83 to 125. In one embodiment of the present invention, provided is the aforementioned method for detecting liver disease with high accuracy, where the biomarker 1 and the at least one additional biomarker are one or more of the biomarkers selected from the group consisting of SEQ ID NOs: 23, 30, 35, 36, 43, 65, 73, 81, 83 to 125.

In one embodiment of the present invention, provided is software for implementing the aforementioned method for detecting liver disease with high accuracy, and a device in which this software was installed.

Each biomarker, biological sample, and liver disease are as mentioned above.

In the aforementioned method for detecting liver disease, the measurement value indicates a measurement value of a subject biomarker at the time this was measured, and the reference value indicates a value such as a value of this biomarker in subjects having no liver disease or a past measurement value in a subject having a liver disease.

According to what the present inventors found out, the expression of miR-320A and miR-483 was significantly raised in conjunction with the progression of fibrogenesis. A significant rise in expression can be seen for miR-122 (a similar pattern also for miR-99a and miR-194-1) and miR-125B-1 (also similarly miR-125B-2) when the fibrogenesis stage is 0. A significant reduction of expression level can be seen for miR-223-3P when compared with a normal subject, and a linear reduction of miRNA expression level can be seen for miR-4454. Accordingly, in an assessment in a method for detecting liver disease, when a biomarker level in a biological sample is higher than a subject value, it can be assessed to be highly likely that there is liver disease for significantly increased miRNA in conjunction with fibrogenesis (miR-34A, miR-194-1, miR-99a, miR-3679, miR-3138, miR-320c-1, miR-320b-1, miR-483, miR-192, miR-122, miR-378a, miR-148a, miR-100, miR-194-2, miR-125B-2, miR-125B-1, miR-320A). Alternatively, when a biomarker level in a biological sample is not high (namely, equal or low) compared to a subject value, it can be assessed to be highly likely that there is no liver disease. Conversely, in an assessment in a method for detecting liver disease, when a biomarker level in a biological sample is lower than a subject value, it can be assessed to be highly likely that there is liver disease for significantly decreased miRNA in conjunction with fibrogenesis (miR-144, miR-4521, miR-4454, miR-223-3p). Alternatively, when a biomarker level in a biological sample is not low (namely, equal or high) compared with a subject value, it can be assessed to be highly likely that there is no liver disease.

Moreover, when a biomarker level in a biological sample is higher than a subject value, it can be assessed to be highly likely that there is liver disease for significantly increased miRNA in conjunction with a rise in the score by Child-Pugh classification (miR-99a, miR-483, miR-122, miR-125B-2, miR-320b-2, miR-125B-1, miR-320A). Alternatively, when a biomarker level in a biological sample is not high (namely, equal or low) compared to a subject value, it can be assessed to be highly likely that there is no liver disease. Conversely, in an assessment in a method for detecting liver disease, when a biomarker level in a biological sample is lower than a subject value, it can be assessed to be highly likely that there is liver disease for significantly decreased miRNA in conjunction with fibrogenesis (miR-144, miR-194-1, miR-4454, miR-223-3p). Alternatively, when a biomarker level in a biological sample is not low (namely, equal or high) compared with a subject value, it can be assessed to be highly likely that there is no liver disease.

The present inventors have now discovered multiple biomarkers showing a clear correlational relationship between the staging of the pathology of liver disease, and have attained the development of a method for detecting liver disease by means of the measurement values of these biomarkers and the transitions thereof. The staging in relation to the degree of progression of liver disease is mentioned below.

< Method for screening a candidate compound of a therapeutic drug >

The present invention provides a method for screening a candidate compound of a therapeutic drug for liver disease in a subject, where the method includes measuring a level of a biomarker in a biological sample from the subject administered with a candidate compound of a therapeutic drug for liver disease, and comparing a measurement value of the biomarker with a reference value.

In one embodiment of the present invention, a biomarker used in the aforementioned method for screening a candidate compound of a therapeutic drug for liver disease is one or more of the biomarkers selected from the group consisting of SEQ ID NOs: 1 to 125. In one embodiment of the present invention, provided is a method for screening a candidate compound of a therapeutic drug for liver disease in a subject, the method including measuring a level of a biomarker in a biological sample from the subject administered with a candidate compound of a therapeutic drug for liver disease, and comparing a measurement value of the biomarker with a reference value, where the biomarker is one or more of the biomarkers selected from the group consisting of SEQ ID NOs: 1 to 125.

In one embodiment of the present invention, the biomarker is one or more of the biomarkers selected from the group consisting of SEQ ID NOs: 1 to 82. In one embodiment of the present invention, provided is a method for screening a candidate compound of a therapeutic drug for liver disease in a subject, the method including measuring a level of a biomarker in a biological sample from the subject administered with a candidate compound of a therapeutic drug for liver disease, and comparing a measurement value of the biomarker with a reference value, where the biomarker is one or more of the biomarkers selected from the group consisting of SEQ ID NOs: 1 to 82.

In one embodiment of the present invention, a biomarker used in the aforementioned method for screening a candidate compound of a therapeutic drug may be selected from the group consisting of miR-4454 (SEQ ID NO: 1), miR-3138 (SEQ ID NO: 2), miR-3679 (SEQ ID NO: 3) and miR-4521 (SEQ ID NO: 4); however, there are no limitations on this. In a preferred embodiment of the present invention, a biomarker used in the aforementioned method for screening a candidate compound of a therapeutic drug is one or more of the biomarkers selected from the group consisting of miR-4454 (SEQ ID NO: 1), miR-3138 (SEQ ID NO: 2), miR-3679 (SEQ ID NO: 3), and miR-4521 (SEQ ID NO: 4). In one embodiment of the present invention, a biomarker used in the aforementioned method for screening a candidate compound of a therapeutic drug is miR-4454 (SEQ ID NO: 1), miR-3138 (SEQ ID NO: 2), miR-3679 (SEQ ID NO: 3), or miR-4521 (SEQ ID NO: 4), or a combination thereof.

In one embodiment of the present invention,

provided is a method for screening a candidate compound of a therapeutic drug for liver disease in a subject, the method including
measuring a level of a biomarker in a biological sample from the subject administered with a candidate compound of a therapeutic drug for liver disease, and
comparing a measurement value of the biomarker with a reference value, where
the biomarker is one or more of the biomarkers selected from the group consisting of miR-4454 (SEQ ID NO: 1), miR-3138 (SEQ ID NO: 2), miR-3679 (SEQ ID NO: 3) and miR-4521 (SEQ ID NO: 4).

In another embodiment of the present invention, in addition to a biomarker being one or more of the biomarkers selected from the group consisting of miR-4454 (SEQ ID NO: 1), miR-3138 (SEQ ID NO: 2), miR-3679 (SEQ ID NO: 3) and miR-4521 (SEQ ID NO: 4), an additional biomarker may be used. In an embodiment of the present invention, the aforementioned method for screening a candidate compound of a therapeutic drug may further include measuring a level of an additional biomarker in a biological sample from a subject, and comparing the measurement value of the additional biomarker with a reference value.

In one embodiment of the present invention,

provided is a method for screening a candidate compound of a therapeutic drug for liver disease in a subject, the method including
measuring a level of a biomarker in a biological sample from the subject administered with a candidate compound of a therapeutic drug for liver disease, and
comparing a measurement value of the biomarker with a reference value, where
the biomarker is one or more of the biomarkers selected from the group consisting of miR-4454 (SEQ ID NO: 1), miR-3138 (SEQ ID NO: 2), miR-3679 (SEQ ID NO: 3), and miR-4521 (SEQ ID NO: 4) and the method further includes
measuring a level of an additional biomarker in a biological sample from a subject, and
comparing the measurement value of the additional biomarker with a reference value.

In the aforementioned method, an additional biomarker may be selected from the group consisting of miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21) and miR-320b-2 (SEQ ID NO: 22); however, there are no limitations on this. In one embodiment of the present invention, provided is the aforementioned method utilizing an additional biomarker, where the additional biomarker is one or more of the biomarkers selected from the group consisting of miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21) and miR-320b-2 (SEQ ID NO: 22).

In another embodiment of the present invention, a biomarker used in the aforementioned method is one or more of the biomarkers selected from the group consisting of SEQ ID NOs: 23, 30, 35, 36, 43, 65, 73, 81, 83 to 125. In one embodiment of the present invention, provided is a method for screening a candidate compound of a therapeutic drug for liver disease in a subject, the method including measuring a level of a biomarker in a biological sample from the subject administered with a candidate compound of a therapeutic drug for liver disease, and comparing a measurement value of the biomarker with a reference value, where the biomarker is one or more of the biomarkers selected from the group consisting of SEQ ID NOs: 23, 30, 35, 36, 43, 65, 73, 81, 83 to 125.

Each biomarker, biological sample, and liver disease are as mentioned above.

In the aforementioned method for screening a candidate compound of a therapeutic drug, the measurement value indicates a measurement value of a subject biomarker at the time when this was measured, and the reference value indicates a value of this biomarker in subjects having no liver disease or a past measurement value in a subject having a liver disease, etc.

The present inventors have now discovered multiple biomarkers showing a clear correlational relationship between the staging of the pathology of liver disease, and have attained the development of a method for screening a candidate compound of a therapeutic drug by observing and investigating the measurement values of these biomarkers and the transitions thereof. The staging in relation to the degree of progression of liver disease is mentioned below.

< Treatment method for liver disease >

The present invention provides a method for treating liver disease in a subject, where the method includes measuring a level of a biomarker in a biological sample from the subject, and comparing a measurement value of the biomarker with a reference value.

In one embodiment of the present invention, a biomarker used in the aforementioned method for treating liver disease is one or more of the biomarkers selected from the group consisting of SEQ ID NOs: 1 to 125. In one embodiment of the present invention, provided is a method for treating liver disease in a subject, the method including measuring a level of a biomarker in a biological sample from the subject, and comparing a measurement value of the biomarker with a reference value, where the biomarker is one or more of the biomarkers selected from the group consisting of SEQ ID NOs: 1 to 125.

In one embodiment of the present invention, the biomarker is one or more of the biomarkers selected from the group consisting of SEQ ID NOs: 1 to 82. In one embodiment of the present invention, provided is a method for treating liver disease in a subject, the method including measuring a level of a biomarker in a biological sample from the subject, and comparing a measurement value of the biomarker with a reference value, where the biomarker is one or more of the biomarkers selected from the group consisting of SEQ ID NOs: 1 to 125.

In one embodiment of the present invention, a biomarker used in the aforementioned treatment method for liver disease may be selected from the group consisting of miR-4454 (SEQ ID NO: 1), miR-3138 (SEQ ID NO: 2), miR-3679 (SEQ ID NO: 3) and miR-4521 (SEQ ID NO: 4); however, there are no limitations on this. In a preferred embodiment of the present invention, a biomarker used in the aforementioned treatment method for liver disease is one or more of the biomarkers selected from the group consisting of miR-4454 (SEQ ID NO: 1), miR-3138 (SEQ ID NO: 2), miR-3679 (SEQ ID NO: 3) and miR-4521 (SEQ ID NO: 4). In one embodiment of the present invention, a biomarker used in the aforementioned treatment method for liver disease is miR-4454 (SEQ ID NO: 1), miR-3138 (SEQ ID NO: 2), miR-3679 (SEQ ID NO: 3), or miR-4521 (SEQ ID NO: 4), or a combination thereof.

In one embodiment of the present invention, provided is a method for treating liver disease in a subject, the method including

measuring a level of a biomarker in a biological sample from the subject, and
comparing a measurement value of the biomarker with a reference value, where
the biomarker is one or more of the biomarkers selected from the group consisting of miR-4454 (SEQ ID NO: 1), miR3138 (SEQ ID NO: 2), miR3679 (SEQ ID NO: 3) and miR4521 (SEQ ID NO: 4).

In an embodiment of the present invention, in addition to a biomarker being one or more of the biomarkers selected from the group consisting of miR-4454 (SEQ ID NO: 1), miR-3138 (SEQ ID NO: 2), miR-3679 (SEQ ID NO: 3) and miR-4521 (SEQ ID NO: 4), an additional biomarker may be used. In an embodiment of the present invention, the aforementioned treatment method for liver disease may further include measuring a level of an additional biomarker in a biological sample from a subject, and comparing the measurement value of the additional biomarker with a reference value.

In one embodiment of the present invention, provided is a method for treating liver disease in a subject, the method including

measuring a level of a biomarker in a biological sample from the subject, and
comparing a measurement value of the biomarker with a reference value, where
the biomarker is one or more of the biomarkers selected from the group consisting of miR-4454 (SEQ ID NO: 1), miR3138 (SEQ ID NO: 2), miR3679 (SEQ ID NO: 3) and miR4521 (SEQ ID NO: 4), and the method further includes measuring a level of an additional biomarker in a biological sample from a subject, and

comparing the measurement value of the additional biomarker with a reference value.

In the aforementioned method, an additional biomarker may be selected from the group consisting of miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21) and miR-320b-2 (SEQ ID NO: 22); however, there are no limitations on this. In one embodiment of the present invention, provided is the aforementioned method utilizing an additional biomarker, where this additional biomarker is one or more of the biomarkers selected from the group consisting of miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21) and miR-320b-2 (SEQ ID NO: 22).

In another embodiment of the present invention, a biomarker used in the aforementioned method is one or more of the biomarkers selected from the group consisting of SEQ ID NOs: 23, 30, 35, 36, 43, 65, 73, 81, 83 to 125. In one embodiment of the present invention, provided is a method for treating liver disease in a subject, the method including measuring a level of a biomarker in a biological sample from the subject, and comparing a measurement value of the biomarker with a reference value, where the biomarker is one or more of the biomarkers selected from the group consisting of SEQ ID NOs: 23, 30, 35, 36, 43, 65, 73, 81, 83 to 125.

Each biomarker, biological sample, and liver disease are as mentioned above.

In the aforementioned treatment method for liver disease, the measurement value indicates a measurement value of a subject biomarker at the time this was measured, and the reference value indicates a value of this biomarker in subjects having no liver disease or a past measurement value in a subject having a liver disease, etc.

The aforementioned method may also include preparing an exosome from a biological sample as required, prior to measuring the level of the biomarker. In that case, the level of a biomarker in a biological sample is measured as a level of a biomarker in a prepared exosome. The collection of an exosome from a biological sample can be performed by means of an ultracentrifugation method (e.g. Thery C., Curr. Protoc. Cell Biol. (2006) Chapter 3: Unit 3.22.), a polymer precipitation method, an immunoprecipitation method, a FACS method, an ultra filtration method, a gel filtration method, an HPLC method, and methods of making an exosome adsorb onto a carrier such as beads by utilizing antibodies or lectins. Moreover, an exosome may also be collected by employing a commercially available kit for exosome isolation.

Checking that an exosome was collected or checking the physical properties of an exosome can be performed according to known methods, e.g. this may be visually checked by means of an electron microscope, or a particle diameter or the number of particles of an exosome may also be measured by means of an NTA (Nano Tracking Analysis) technique. Alternatively, by checking the expression of a protein and/or a gene which could become a marker of an exosome, the presence of an exosome can also be checked.

There is no particular limitation on the measurement of the level of a biomarker if it is a method which can measure an RNA level; however, the measurement is generally performed with a method utilizing a substance which binds specifically to an RNA biomarker such as a probe or primer. In the present specification, a "nucleic acid" includes a natural nucleic acid such as DNA and RNA, or an artificially created nucleic acid such as a crosslinking nucleic acid, such as PNA or Locked Nucleic Acid (2',4'-BNA), or a combination thereof. The substances binding specifically to RNA may also include at least a partially artificially designed sequence (e.g. a sequence for the purpose of labelling or tagging).

The measurement of a biomarker level can be performed by measuring the binding level of a substance binding specifically to a biomarker which is bound to a biomarker. The "binding level" can be configured as a binding amount, number of bindings, or binding ratio, or a numerical value which represents these (e.g. a measurement value itself such as a measured fluorescence intensity). In this case, a labelled substance binding specifically to a marker RNA may be used as a substance, or a marker RNA may be labelled and used. Moreover, a standard sample is generally measured at the same time, and a value calculated from the measurement value of a measurement sample by creating a standard curved line or a calibration line based on this standard sample, or a numerical value of which a standard sample level was standardised to an index, is determined as the binding level. Methods of labelling can include e.g. radioactive isotope labelling (RI) labelling, fluorescent labelling, and enzymatic labelling.

For example, the methods mentioned above may include the (a) to (c) below:

(a) bringing a biological sample into contact with at least one probe;
(b) measuring the binding level of a biomarker in the biological sample bound to the probe; and
(c) determining a biomarker level in this biological sample from the measured binding level.

Alternatively, the RNA level can be measured by means of a method which utilizes PCR, e.g. may also be measured using a primer to perform qPCR, ARMS (Amplification Refractory Mutation System), RT-PCR (Reverse transcriptase-PCR), or Nested PCR. Alternatively, the Invader (registered trademark) method may also be used. For example, a method employing the GenoExplorer™ miRNA qRT-PCR Kit (GenoSensor Corporation) together with a suitable primer can be used.

The amplification of all or a portion of a biomarker in a biological sample can be implemented by performing a PCR reaction etc. on this biological sample as a template. The level of the amplified nucleic acid can be measured by the dot-blot hybridization method, surface plasmon resonance (SPR method), PCR-RFLP method, In situ RT-PCR method, PCR-SSO (sequence specific Oligonucleotide) method, PCR-SSP method, AMPFLP (Amplifiable fragment length polymorphism) method, MVR-PCR method, and PCR-SSCP (single strand conformation polymorphism) method.

For example, the RNA level may also be measured by the steps below:

(a) amplifying all or a portion of the biomarker in the biological sample, with a biological sample as a template, and using a primer:
(b) measuring the level of the amplified nucleic acid molecule; and
(c) determining the biomarker level in the biological sample from the level of the amplified nucleic acid molecule.

**In** the present specification, "level" means an index relating to an amount present denoted by a numerical value, and includes e.g. a concentration, an amount, or an index (preferably a numerical value index) which can be used as alternatives thereof. Accordingly, the level may also be a measurement value itself, and may also be a value converted into a concentration or absolute amount. Moreover, the level may be an absolute numerical value (amount, amount per unit surface area or volume etc.), or may also be a relative numerical value compared with a comparative reference which was set as required (e.g. internal control).

The present inventors have now discovered multiple biomarkers showing a clear correlational relationship between the staging of the pathology of liver disease, and have attained the development of a method for treating liver disease by observing and investigating the measurement values of these biomarkers and the transitions thereof. The staging in relation to the degree of progression of liver disease is mentioned below.

< Diagnostic support system for liver disease >

The present invention provides a diagnostic support system for liver disease provided with:

a unit for measuring a level of one or more or at least two biomarkers in a biological sample from a subject;
a unit for comparing a measurement value of the one or more or at least two biomarkers with respective reference values thereof to obtain a plurality of parameters; and
a unit for calculating, from the plurality of parameters, the presence or absence of liver disease or the risk or degree of progression thereof in the subject, and/or the degree of success of a procedure performed for treatment.

In one embodiment of the present invention, a biomarker used in the aforementioned diagnostic support system for liver disease is one or more of the biomarkers selected from the group consisting of SEQ ID NOs: 1 to 125. In one embodiment of the present invention, provided is the aforementioned diagnostic support system for liver disease, where the biomarker is one or more of the biomarkers selected from the group consisting of SEQ ID NOs: 1 to 125.

In one embodiment of the present invention, a biomarker used in the aforementioned diagnostic support system for liver disease is one or more of the biomarkers selected from the group consisting of SEQ ID NOs: 1 to 82. In one embodiment of the present invention, provided is the aforementioned diagnostic support system for liver disease, where the biomarker is one or more of the biomarkers selected from the group consisting of SEQ ID NOs: 1 to 82.

In one embodiment of the present invention, the one or more or at least two biomarkers can be selected from the group consisting of miR-4454 (SEQ ID NO: 1), miR-3138 (SEQ ID NO: 2), miR-3679 (SEQ ID NO: 3), miR-4521 (SEQ ID NO: 4), miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21) and miR-320b-2 (SEQ ID NO: 22); however, there are no limitations on this. In one embodiment of the present invention, provided is the aforementioned diagnostic support system for liver disease, where the one or more or at least two biomarkers are selected from the group consisting of miR-4454 (SEQ ID NO: 1), miR-3138 (SEQ ID NO: 2), miR-3679 (SEQ ID NO: 3), miR-4521 (SEQ ID NO: 4), miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21) and miR-320b-2 (SEQ ID NO: 22).

In another embodiment of the present invention, a biomarker used in the aforementioned diagnostic support system for liver disease is one or more of the biomarkers selected from the group consisting of SEQ ID NOs: 23, 30, 35, 36, 43, 65, 73, 81, 83 to 125. In one embodiment of the present invention, provided is the aforementioned diagnostic support system for liver disease, where the biomarker is one or more of the biomarkers selected from the group consisting of SEQ ID NOs: 23, 30, 35, 36, 43, 65, 73, 81, 83 to 125.

Each biomarker, biological sample, and liver disease are as mentioned above.

In the aforementioned diagnostic support system, the measurement value indicates a measurement value of a subject biomarker at the time this was measured, and the reference value indicates a value of this biomarker in subjects having no liver disease or a past measurement value in a subject having a liver disease, etc.

The present inventors have now discovered multiple biomarkers showing a clear correlational relationship between the staging of the pathology of liver disease, and have attained the development of a diagnostic support system by observing and investigating the measurement values of these biomarkers and the transitions thereof. The staging in relation to the degree of progression of liver disease is mentioned below.

In one embodiment of the present invention, provided is a device of which the aforementioned diagnostic support system for liver disease is installed.

< Detection kit for liver disease >

In one embodiment of the present invention, provided is a detection kit for liver disease and a device for detection which includes a nucleic acid capable of binding specifically to a specific miRNA. In the present invention, a nucleic acid capable of binding specifically to this specific miRNA can be selected from a nucleic acid capable of binding specifically to a polynucleotide which is a biomarker for liver disease, and to a complementary strand of this polynucleotide.

In one embodiment of the present invention, a biomarker used in the aforementioned detection kit for liver disease is one or more of the biomarkers selected from the group consisting of SEQ ID NOs: 1 to 125. In one embodiment of the present invention, provided is the aforementioned detection kit for liver disease, where the biomarker is one or more of the biomarkers selected from the group consisting of SEQ ID NOs: 1 to 125.

In one embodiment of the present invention, a biomarker used in the aforementioned detection kit for liver disease is one or more of the biomarkers selected from the group consisting of SEQ ID NOs: 1 to 82. In one embodiment of the present invention, provided is the aforementioned detection kit for liver disease, where the biomarker is one or more of the biomarkers selected from the group consisting of SEQ ID NOs: 1 to 82.

The biomarker for liver disease used in the present invention is for example a polynucleotide selected from the group consisting of miR-4454 (SEQ ID NO: 1), miR-3138 (SEQ ID NO: 2), miR-3679 (SEQ ID NO: 3) and miR-4521 (SEQ ID NO: 4); however, there are no limitations on this. In one embodiment of the present invention, a biomarker for liver disease is a polynucleotide selected from the group consisting of miR-4454 (SEQ ID NO: 1), miR-3138 (SEQ ID NO: 2), miR-3679 (SEQ ID NO: 3) and miR-4521 (SEQ ID NO: 4).

In one embodiment of the present invention, provided is a detection kit for liver disease, which includes a nucleic acid capable of binding specifically to a polynucleotide, which is a biomarker, selected from the group consisting of miR-4454 (SEQ ID NO: 1), miR-3138 (SEQ ID NO: 2), miR-3679 (SEQ ID NO: 3) and miR-4521 (SEQ ID NO: 4), or to a complementary strand of these polynucleotides.

The aforementioned detection kit for liver disease can further include at least one nucleic acid capable of binding specifically to a polynucleotide which is another biomarker. In one embodiment of the present invention, provided is the aforementioned detection kit for liver disease which further includes at least one nucleic acid capable of binding specifically to a polynucleotide which is another biomarker.

In the aforementioned detection kit for liver disease, another biomarker may be selected from the group consisting of miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21) and miR-320b-2 (SEQ ID NO: 22); however, there are no limitations on this. In one embodiment of the present invention, provided is the aforementioned detection kit for liver disease utilizing another biomarker, where the other biomarker is one or more of the biomarkers selected from the group consisting of miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21) and miR-320b-2 (SEQ ID NO: 22).

In another embodiment of the present invention, a biomarker used in the aforementioned detection kit for liver disease is one or more of the biomarkers selected from the group consisting of SEQ ID NOs: 23, 30, 35, 36, 43, 65, 73, 81, 83 to 125. In one embodiment of the present invention, provided is the aforementioned detection kit for liver disease, where the biomarker is one or more of the biomarkers selected from the group consisting of SEQ ID NOs: 23, 30, 35, 36, 43, 65, 73, 81, 83 to 125.

In one embodiment of the present invention, provided is the aforementioned detection kit for liver disease, where the nucleic acid is selected from a polynucleotide or a fragment thereof listed in any of the (a) to (c) below.

(a)

(a-1) a polynucleotide or a fragment thereof containing 15 or more continuous bases, the polynucleotide consisting of a nucleotide sequence complementary to a nucleotide sequence of any of SEQ ID NOs: 1 to 125, or to a nucleotide sequence where u is t in this nucleotide sequence,
(a-2) a polynucleotide or a fragment thereof containing a deletion, substitution, addition or insertion of one or more bases in a nucleotide sequence of a polynucleotide or a fragment thereof in (a-1),
(a-3) a polynucleotide of (a-1) or (a-2) or a fragment thereof containing a modified nucleic acid and/or a modified nucleotide,

(b) a polynucleotide or a fragment thereof containing 15 or more continuous bases, the polynucleotide containing a nucleotide sequence complementary to a nucleotide sequence of any of SEQ ID NOs: 1 to 125, or to a nucleotide sequence where u is t in this nucleotide sequence, and
(c) a polynucleotide that hybridizes with a polynucleotide or a fragment thereof listed in any of the (c-1) to (c-4) below under a high stringent condition.

(c-1) a polynucleotide or a fragment thereof containing 15 or more continuous bases, the polynucleotide consisting of a nucleotide sequence of any of SEQ ID NOs: 1 to 125, or a nucleotide sequence where u is t in this nucleotide sequence,
(c-2) a polynucleotide or a fragment thereof containing a deletion, substitution, addition or insertion of one or more bases in a nucleotide sequence of a polynucleotide or a fragment thereof in (c-1),
(c-3) a polynucleotide of (c-1) or (c-2) or a fragment thereof containing a modified nucleic acid and/or a modified nucleotide,
(c-4) a polynucleotide or a fragment thereof containing 15 or more continuous bases, the polynucleotide containing a nucleotide sequence of any of SEQ ID NOs: 1 to 125, or a nucleotide sequence where u is t in this nucleotide sequence

In one embodiment of the present invention, the polynucleotide of any of the above (a) to (c) used in the aforementioned detection kit for liver disease is selected from polynucleotides of any of SEQ ID NOs: 1 to 82. In one embodiment of the present invention, provided is the aforementioned detection kit for liver disease, where the polynucleotide of any of the above (a) to (c) is selected from polynucleotides of any of SEQ ID NOs: 1 to 82.

In one embodiment of the present invention, provided is the aforementioned detection kit for liver disease, where the nucleic acid is selected from a polynucleotide or a fragment thereof listed in any of the (a) to (c) below.

(a)

(a-1) a polynucleotide or a fragment thereof containing 15 or more continuous bases, the polynucleotide consisting of a nucleotide sequence complementary to a nucleotide sequence of any of SEQ ID NOs: 1 to 4, or to a nucleotide sequence where u is t in this nucleotide sequence,
(a-2) a polynucleotide or a fragment thereof containing a deletion, substitution, addition or insertion of one or more bases in a nucleotide sequence of a polynucleotide or a fragment thereof in (a-1),
(a-3) a polynucleotide of (a-1) or (a-2) or a fragment thereof containing a modified nucleic acid and/or a modified nucleotide,

(b) a polynucleotide or a fragment thereof containing 15 or more continuous bases, the polynucleotide containing a nucleotide sequence complementary to a nucleotide sequence of any of SEQ ID NOs: 1 to 4, or to a nucleotide sequence where u is t in this nucleotide sequence, and
(c) a polynucleotide that hybridizes with a polynucleotide or a fragment thereof listed in any of the (c-1) to (c-4) below under a high stringent condition.

(c-1) a polynucleotide or a fragment thereof containing 15 or more continuous bases, the polynucleotide consisting of a nucleotide sequence of any of SEQ ID NOs: 1 to 4, or a nucleotide sequence where u is t in this nucleotide sequence,

(c-2) a polynucleotide or a fragment thereof containing a deletion, substitution, addition or insertion of one or more bases in a nucleotide sequence of a polynucleotide or a fragment thereof in (c-1),

(c-3) a polynucleotide of (c-1) or (c-2) or a fragment thereof containing a modified nucleic acid and/or a modified nucleotide,

(c-4) a polynucleotide or a fragment thereof containing 15 or more continuous bases, the polynucleotide containing a nucleotide sequence of any of SEQ ID NOs: 1 to 4, or a nucleotide sequence where u is t in this nucleotide sequence

In one embodiment of the present invention, provided is the aforementioned detection kit where the nucleic acid capable of binding specifically to a polynucleotide which is the other biomarker, is selected from a polynucleotide or a fragment thereof listed in any of the (d) to (f) below.

(d)

(d-1) a polynucleotide or a fragment thereof containing 15 or more continuous bases, the polynucleotide consisting of a nucleotide sequence complementary to a nucleotide sequence of any of SEQ ID NOs: 5 to 21, or to a nucleotide sequence where u is t in this nucleotide sequence,

(d-2) a polynucleotide or a fragment thereof containing a deletion, substitution, addition or insertion of one or more bases in a nucleotide sequence of a polynucleotide or a fragment thereof in (d-1),

(d-3) a polynucleotide of (d-1) or (d-2) or a fragment thereof containing a modified nucleic acid and/or a modified nucleotide,

(e) a polynucleotide or a fragment thereof containing 15 or more continuous bases, the polynucleotide containing a nucleotide sequence complementary to a nucleotide sequence of any of SEQ ID NOs: 5 to 21, or to a nucleotide sequence where u is t in this nucleotide sequence, and

(f) a polynucleotide that hybridizes with a polynucleotide or a fragment thereof listed in any of the (f-1) to (f-4) below under a high stringent condition.

(f-1) a polynucleotide or a fragment thereof containing 15 or more continuous bases, the polynucleotide consisting of a nucleotide sequence of any of SEQ ID NOs: 5 to 21, or a nucleotide sequence where u is t in this nucleotide sequence,

(f-2) a polynucleotide or a fragment thereof containing a deletion, substitution, addition or insertion of one or more bases in a nucleotide sequence of a polynucleotide or a fragment thereof in (f-1),

(f-3) a polynucleotide of (f-1) or (f-2) or a fragment thereof containing a modified nucleic acid and/or a modified nucleotide,

(f-4) a polynucleotide or a fragment thereof containing 15 or more continuous bases, the polynucleotide containing a nucleotide sequence of any of SEQ ID NOs: 5 to 21, or a nucleotide sequence where u is t in this nucleotide sequence

In another embodiment of the present invention, a polynucleotide shown in any of the above (a) to (c) used in the aforementioned detection kit for liver disease is one or more of the polynucleotides selected from the group consisting of SEQ ID NOs: 23, 30, 35, 36, 43, 65, 73, 81, 83 to 125. In one embodiment of the present invention, provided is the aforementioned detection kit for liver disease, where a polynucleotide shown in any of the above (a) to (c) is one or more of the polynucleotides selected from the group consisting of SEQ ID NOs: 23, 30, 35, 36, 43, 65, 73, 81, 83 to 125.

In the present specification, a "polynucleotide" may be used with respect to nucleic acids encompassing any of RNA, DNA, and RNA/DNA (chimera). Any of cDNA, genomic DNA, and synthetic DNA may be included in the aforementioned DNA. Moreover, any of total RNA, mRNA, rRNA, miRNA, siRNA, snoRNA, snRNA, non-coding RNA and synthetic RNA may also be included in the aforementioned RNA. In the present specification, "synthetic DNA" and "synthetic RNA" means artificially produced DNA and RNA using e.g. an automatic nucleic acid synthesiser, based on a predetermined nucleotide sequence (may be either a natural-type sequence or non-natural-type sequence). In the present specification, it is intended that a "non-natural-type sequence" be used with a broad meaning, which encompasses e.g. sequences containing 1 or more nucleotide substitutions, deletions, insertions and/or additions (namely, mutant sequences), and sequences containing 1 or more modified nucleotides (namely, modified sequences), etc., which differ to natural-type sequences. Moreover, in the present specification, a polynucleotide may be used interchangeably with a nucleic acid.

In the present specification, a "fragment" is a polynucleotide (including an oligonucleotide) having a nucleotide sequence of a continuous portion of a polynucleotide, where it is desirable that the fragment has a length of 15 or more bases, preferably 17 or more bases, and more preferably 19 or more bases.

In the present specification, unless otherwise specially mentioned, "micro RNA (miRNA)" is transcribed as an RNA precursor of a hairpin-like structure, cleaved by a dsRNA cleaving enzyme having RNase III cleaving activity, incorporated into a protein complex called a RISC, and intended to be used as a non-coding RNA of 15 to 25 bases which is involved in the translation suppression of mRNA. Moreover, the "miRNA" used in the present specification is not only the "miRNA" of specific nucleotide sequences (or SEQ ID NOs), but also encompasses precursors of this "miRNA" (pre-miRNA, pri-miRNA), miRNA whose biological function is equal to these precursors, e.g. homologues (or orthologues), mutants such as genetic polymorphisms etc., and derivatives. Such precursors, homologues, mutants or derivatives can be specifically identified by "miRBase release 20" (http://www.mirbase.org/), and can include an "miRNA" having a nucleotide sequence hybridizing with a complementary sequence of any of the specific nucleotide sequences of any of SEQ ID NOs: 1 to 22 under a stringent condition described later. Moreover, the "miRNA" used in the present specification may furthermore also be a gene product of miRgene, where such gene product encompasses mature miRNA (e.g. the aforementioned non-coding RNA of 15 to 25 bases, or 19 to 25 bases involved in the translation suppression of mRNA) or an miRNA precursor (e.g. the pre-miRNA or pri-miRNA as described above).

In the present specification, a "probe" encompasses a polynucleotide used for specifically detecting an RNA produced by gene expression or a polynucleotide derived therefrom, and/or a polynucleotide complementary thereto.

In the present specification, a "primer" encompasses a polynucleotide which specifically recognises and amplifies an RNA produced by gene expression or a polynucleotide derived therefrom, and/or a polynucleotide complementary thereto. Here, a "complementary polynucleotide (a complementary strand, reverse strand)" means a polynucleotide in a complementary relationship in terms of a base, on the basis of a base-pair relationship such as A:T(U), G:C with respect to a full-length or partial sequence (for convenience, this will be called a positive strand here) of a polynucleotide consisting of a nucleotide sequence defined by any of SEQ ID NOs: 1 to 22, or a nucleotide sequence where u is t in this nucleotide sequence. However, such a complementary strand is not limited to the case of forming a complementary sequence completely with a nucleotide sequence of a positive strand configured as a subject, but may also have a complementary relationship to the extent of being able to hybridize with a positive strand configured as a subject by a stringent condition.

In the present specification, a "stringent condition" refers to a condition of a nucleic acid probe hybridizing to a target sequence thereof, by an extent larger than that to other sequences (e.g. average of background measurement value + standard error of background measurement value × 2 or more measurement values). A stringent condition is sequence dependent, and differs according to the environment in which hybridization is performed. By controlling the stringency of a hybridization and/or wash condition, a target sequence which is 100% complementary to a nucleic acid probe can be identified.

In the present specification, a "nucleic acid" capable of binding specifically to a polynucleotide selected from an miRNA, which is the aforementioned biomarker, is a synthesised or prepared nucleic acid, and specifically includes a "nucleic acid probe" or a "primer". This "nucleic acid" is directly or indirectly used in order to: detect whether liver disease is present in a subject; or diagnose whether there is morbidity of liver disease, the extent of morbidity, whether there is improvement of liver disease and the extent of improvement, and sensitivity to treatment for liver disease; or screen a useful candidate substance for the prevention, improvement or treatment of liver disease. Encompassed in these are nucleotides, oligonucleotides and polynucleotides which can recognise and bind specifically to a transcriptional product of any of SEQ ID NOs: 1 to 22 or a cDNA synthesised nucleic acid thereof, either in-vivo or particularly in a specimen such as a bodily fluid such as blood and urine, in relation to the morbidity of liver disease. These nucleotides, oligonucleotides and polynucleotides can be effectively utilized as a probe for detecting the aforementioned genes expressed in-vivo, intra-tissue or intra-cellularly etc., or as a primer for amplifying the aforementioned genes expressed in-vivo, based on the aforementioned properties.

The phrase "capable of binding specifically to" in the present specification means that a nucleic acid probe or primer utilized in the present invention binds to a specific target nucleic acid, and cannot bind substantially to other nucleic acids.

The term "detection" used in the present specification can be substituted with terms such as test, measurement, detection or assessment support. Moreover, the term "evaluation" in the present specification can be used with meanings which include supporting a diagnosis or evaluation based on a test result or measuring result.

Each biomarker and liver disease are as mentioned above.

The present inventors have now discovered multiple biomarkers showing a clear correlational relationship between the staging of the pathology of liver disease, and have attained the development of a detection kit for liver disease and a device for detection by means of utilizing these biomarkers.

< Data processing method for staging the pathology of liver disease >

The present invention provides a method for performing data processing for staging the pathology of liver disease of subjects, the method executed by a computer, where the computer is provided with:

a computer processor; and

a computer readable medium storing software that gives instructions for staging the pathology of liver disease of the subject; and

where the method includes:

incorporating data of a plurality of parameters into a computer readable medium, the data of a plurality of parameters obtained by comparing a measurement value of one or more or at least two biomarkers in a biological sample obtained from the subject with respective reference values thereof; and

outputting an assessment result of a pathology of liver disease of the subject as information by the software that gives instructions for staging the pathology of liver disease of the subject, the assessment performed by the computer that executes the instructions using the computer processor to process the data of the plurality of parameters.

In one embodiment of the present invention,

provided is a method for performing data processing for staging the pathology of liver disease of a subject, the method executed by a computer, the computer provided with:

a computer processor; and

a computer readable medium storing software that gives instructions for staging the pathology of liver disease of the subject; and

where the method includes:

incorporating data of a plurality of parameters into a computer readable medium, the data of a plurality of parameters obtained by comparing a measurement value of one or more or at least two biomarkers in a biological sample obtained from the subject with respective reference values thereof; and

outputting an assessment result of a pathology of liver disease of the subject as information by the software that gives instructions for staging the pathology of liver disease of the subject, the assessment performed by the computer that executes the instructions using the computer processor to process the data of the plurality of parameters; and

where the staging includes one or more selected from the group consisting of a hepatic fibrogenesis stage, Child-Pugh classification, MELD score, MELD Na score, PELD score, ALBI score, mALBI (modified ALBI) score, FibroScan score, new Inuyama classification, and new European classification.

In the aforementioned method, an assessment result may be a combination of results respectively assessed by processing the above-mentioned data of a plurality of parameters. In one embodiment of the present invention, provided is the aforementioned method, where the assessment result is a combination of results respectively assessed by processing the data of the plurality of parameters.

In one embodiment of the present invention, the one or more or at least two biomarkers used in the aforementioned method for performing data processing are selected from the group consisting of SEQ ID NOs: 1 to 125. In one embodiment of the present invention, provided is the aforementioned method for performing data processing, where the one or more or at least two biomarkers are selected from the group consisting of SEQ ID NOs: 1 to 125.

In one embodiment of the present invention, the one or more or at least two biomarkers used in the aforementioned method for performing data processing are selected from the group consisting of SEQ ID NOs: 1 to 82. In one embodiment of the present invention, provided is the aforementioned method for performing data processing, where the one or more or at least two biomarkers are selected from the group consisting of SEQ ID NOs: 1 to 82.

In one embodiment of the present invention, the one or more or at least two biomarkers can be selected from the group consisting of miR-4454 (SEQ ID NO: 1), miR-3138 (SEQ ID NO: 2), miR-3679 (SEQ ID NO: 3), miR-4521 (SEQ ID NO: 4), miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21) and miR-320b-2 (SEQ ID NO: 22); however, there are no limitations on this. In one embodiment of the present invention, provided is the aforementioned method for performing data processing, where the one or more or at least two biomarkers are selected from the group consisting of miR-4454 (SEQ ID NO: 1), miR-3138 (SEQ ID NO: 2), miR-3679 (SEQ ID NO: 3), miR-4521 (SEQ ID NO: 4), miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO:

11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21) and miR-320b-2 (SEQ ID NO: 22).

In another embodiment of the present invention, the one or more or at least two biomarkers used in the aforementioned method for performing data processing, are one or more of the biomarkers selected from the group consisting of SEQ ID NOs: 23, 30, 35, 36, 43, 65, 73, 81, 83 to 125. In one embodiment of the present invention, provided is the aforementioned method for performing data processing, where the one or more or at least two biomarkers, are one or more of the biomarkers selected from the group consisting of SEQ ID NOs: 23, 30, 35, 36, 43, 65, 73, 81, 83 to 125.

Each biomarker, biological sample, and liver disease are as mentioned above.

In the aforementioned diagnostic support system, the measurement value indicates a measurement value of a subject biomarker at the time this was measured, and the reference value indicates a value of this biomarker in subjects having no liver disease or a past measurement value in a subject having a liver disease, etc.

The present inventors have now discovered multiple biomarkers showing a clear correlational relationship between the staging of the pathology of liver disease, and have attained the development of a method for performing data processing for staging the pathology of liver disease of a subject by observing and investigating the measurement values of these biomarkers and the transitions thereof. The staging in relation to the degree of progression of liver disease will be explained below.

Examples of staging of a pathology of liver disease include e.g. a hepatic fibrogenesis stage, Child-Pugh classification, MELD score, MELD Na score, PELD score, ALBI score, mALBI (modified ALBI) score, FibroScan score, new Inuyama classification, new European classification, Brunt classification etc.; however, there are no limitations on this. These stagings can be used alone, or a plurality of stagings can be used in combination. In one embodiment of the present invention, the staging includes one or more selected from the group consisting of a hepatic fibrogenesis stage, Child-Pugh classification, MELD score, MELD Na score, PELD score, ALBI score, mALBI (modified ALBI) score, FibroScan score, new Inuyama classification, new European classification, and Brunt classification.

- Hepatic fibrogenesis stage classification

The classifications progressively increase from no fibrogenesis seen, until the attainment of hepatic cirrhosis, and are divided into five stages of F0 (no fibrogenesis seen), F1 (mild), F2 (moderate), F3 (severe) and F4 (hepatic cirrhosis).

- Child-Pugh classification

This is an index showing the degree of damage of the liver. As shown in Table 1 below, five items of: the presence or absence of hepatic encephalopathy; presence or absence of ascites; serum bilirubin concentration (Bil); serum albumin concentration (Alb); and prothrombin activation value (PT-INR), are evaluated by 1 to 3 points, and the index is classified into three stages of A to C in accordance with the total of these points. The lowest degree of damage is A (compensated), and becomes B (decompensated) and C (decompensated) as the degree of damage becomes higher.

[Table 2-1]

| Item　　　　　　　　Points | 1 point | 2 points | 3 points |
|---|---|---|---|
| Encephalopathy | None | Mild | Occasional lethargy |
| Ascites | None | Small amount | Moderate amount |
| Serum bilirubin value (mg/dL) | Less than 2.0 | 2.0-3.0 | More than 3.0 |
| Serum albumin value (g/dL) | More than 3.5 | 2.8-3.5 | Less than 2.8 |
| Prothrombin activation value (%) | More than 70 | 40-70 | Less than 40 |

| Child-Pugh classification | A: 5 to 6 points |
|---|---|
| | B: 7 to 9 points |
| | C: 10 to 15 points |

- MELD score

This is a scoring system using the total bilirubin value (T-Bil), prothrombin activation value (PT-INR), creatinine value (Cr), and the presence or absence of dialysis treatment. The MELD score is an index used in the diagnosis of liver functional reserve in hepatic cirrhosis and liver transplant registrants who are 12 years of age or more.

- MELD Na score

This is a scoring system similar to that of the conventional MELD score, with the serum sodium concentration (Na) added to the blood biochemistry test data (T-Bil, PT-INR, Cr), and is an index used in the diagnosis of liver functional reserve in acute hepatitis / hepatic cirrhosis and liver transplant registrants who are 12 years of age or more.

- PELD score

This is a scoring system using blood biochemistry test data (Bil, PT-INR, Alb), age and growth rate, and is used in the diagnosis of liver functional reserve in hepatic cirrhosis and liver transplant registrants who are less than 12 years of age

- ALBI score

This is a scoring system using blood biochemistry test data (T-Bil, Alb), and is an index used when selecting a therapeutic method for hepatocellular carcinoma. Scoring is usually classified into three grades (1, 2, 3) called ALBI grades.

- mALBI (modified ALBI) score

This is a scoring system using blood biochemistry test data (T-Bil, Alb) similarly to that of the conventional ALBI score, and is an index used when selecting a therapeutic method for hepatocellular carcinoma. By sub-dividing into four categories (1, 2a, 2b, 3), a better evaluation ability can be expected.

- FibroScan score

This is a scoring system in which a special "probe" transmitting vibrations and ultrasound waves is applied to the surface of the right flank, and the hardness of the liver measured from the way such vibrations and ultrasound waves are transmitted is expressed as a numerical value. Because the vibrations transmit as quickly as the extent that fibrogenesis in the liver has occurred and thus becomes hardened, the extent of the fibrogenesis can be known with a numerical value. Moreover, the FibroScan score can be utilized not only in the evaluation of fibrogenesis, but also in the prediction of carcinogenesis as well as the evaluation of the extent of portal hypertension, etc.

- New Inuyama classification

This classifies the pathology of chronic hepatitis into four categories of the extent of inflammation of the portal region periphery, intralobular and portal regions, as well as the extent of fibrogenesis, which are semi-quantitatively expressed as scores.

- New European classification

This classifies the pathogenesis of hepatitis into viral, autoimmune, medicinal, and unknown etiology, and is described by division into four stages of grading and five stages of staging.

- Brunt classification

This is an index of evaluating and classifying a NASH pathological finding by the extent of grading and the extent of staging. Grading is evaluated in three stages, where the extent of (1) fat deposits, (2) ballooning of hepatocytes, and (3) inflammatory cellular infiltration of intralobular and intraportal regions, are comprehensively evaluated as evaluation items. Staging is evaluated in four stages, where configured are: Stage 1 as centrilobular fibrogenesis; Stage 2 as Stage 1 + fibrogenesis of the portal region; Stage 3 as the further addition of bridging fibrosis; and Stage 4 as hepatic cirrhosis.

[Table 2-2]

| Brunt classification | | | |
|---|---|---|---|
| Grading | Steatosis | Ballooning | Inflammation |
| Grade 1 (mild) | ~ 1/3 | Occasionally centrilobular | Mild |
| Grade 2 (moderate) | 1/3 ~ 2/3 | Clearly centrilobular | Moderate |
| Grade 3 (severe) | 2/3 ~ | Markedly present | Severe |
| Staging | | | |
| Stage 1 (B1) | Fibrogenesis of centrilobular part | | |
| Stage 2 (B2) | Stage 1 + Fibrogenesis of portal region | | |
| Stage 3 (B3) | Bridging fibrosis | | |
| Stage 4 (B4) | Hepatic cirrhosis | | |

In one embodiment of the present invention, provided is software for implementing the aforementioned method for performing data processing for staging the pathology of liver disease of subjects, and a device in which this software is installed.

In an embodiment of the present invention, a subject is a mammalian subject, and more specifically means a mammal such as a primate including a human and chimpanzee, a pet animal such as a dog and cat, a livestock animal such as a cow, horse, sheep and goat, and a rodent such as a mouse and rat. In a preferred embodiment of the present invention, a subject is a human subject.

< Program for executing an assessment of liver disease on a computer, and medium for recording the program >

In one embodiment of the present invention, provided is

a program for executing an assessment of the presence or absence of liver disease or the risk or degree of progression thereof in a subject and/or an assessment of the degree of success of a procedure performed for a treatment on a computer, the program executing:

a measurement value acquisition step of respectively acquiring a measurement value of a level of one or more or at least two biomarkers obtained from a biological sample from the subject;

a parameter data acquisition step of acquiring data of a plurality of parameters by comparing a measurement value of the one or more or at least two biomarkers with respective reference values; and

an assessment step of assessing, based on the data of the plurality of parameters, the presence or absence of liver disease or the risk or degree of progression thereof in the subject, and/or the degree of success of a procedure performed for treatment, and

the biomarker being selected from the group consisting of SEQ ID NOs: 1 to 125. In one embodiment of the present invention, this program can be recorded on a medium. In one embodiment of the present invention, provided is a recording medium on which this program was recorded.

In one embodiment of the present invention, the biomarker in the aforementioned program is selected from the group consisting of SEQ ID NOs: 1 to 82. In one embodiment of the present invention, provided is the aforementioned program, where the biomarker is selected from the group consisting of SEQ ID NOs: 1 to 82. In one embodiment of the present invention, this program can be recorded on a medium. In one embodiment of the present invention, provided is a recording medium on which this program was recorded.

In one embodiment of the present invention, the biomarker in the aforementioned program is selected from the group consisting of SEQ ID NOs: 23, 30, 35, 36, 43, 65, 73, 81, 83 to 125. In one embodiment of the present invention, provided is the aforementioned program, where the biomarker is selected from the group consisting of SEQ ID NOs: 23, 30, 35, 36, 43, 65, 73, 81, 83 to 125. In one embodiment of the present invention, this program can be recorded on a medium. In one embodiment of the present invention, provided is a recording medium on which this program was recorded.

Each biomarker, biological sample, and liver disease are as mentioned above.

< Data processing device to perform data processing for detecting liver disease >

In one embodiment of the present invention, provided is a data processing device to perform data processing for detecting liver disease, the data processing device provided with:

> a missing value supplement unit to obtain missing value supplemental data by supplementing a missing value in miRNA expression level data;
> a logarithmic transformation unit to obtain logarithmic transformation data by logarithmically transforming the missing value supplemental data;
> a regression transformation unit to obtain regression transformation data by substituting the logarithmic transformation data in a regression model and executing a regression transformation;
> a training data acquisition unit to acquire training data; and
> a learnt model creation unit to create a learnt model using the regression transformation data as the training data.

In one embodiment of the present invention, with a missing value supplement unit, missing value supplemental data is obtained for each miRNA by supplementing the missing value in the expression level data of each miRNA. In the present invention, a miRNA expression level data obtainable by an analytical method employing a highly accurate microarray DNA chip 3D-Gene (registered trademark) by Toray Industries Inc was used as miRNA expression level data. Three types of data of "Raw data", "BG subtraction" and "global normalization" are included in this miRNA expression level data. Amongst these, the "global normalization" was used in the present invention. However, since missing value is included in the "global normalization" data, the missing values were supplemented with the minimum value of each miRNA sample. Thereby, data of a predetermined number were included in the "global normalization" data. A missing value is understood as a general meaning, and indicates a value of a section where data has been deleted for whatever reason. Moreover, since the distribution of the aforementioned miRNA expression level data is a log-normal distribution, the missing value supplement mentioned above and the logarithmic transformation mentioned below are performed before the statistical analysis processing.

In one embodiment of the present invention, with a logarithmic transformation unit, logarithmic transformation data is obtained by logarithmically transforming a missing value supplemental data whose missing value was supplemented as in the aforementioned. A logarithmic transformation can be implemented by e.g. $\log_e$, $\log_2$, $\log_{10}$ etc. In the present invention, the logarithmic transformation was implemented configuring 2 as the base (logarithmic transformation by $\log_2$).

In one embodiment of the present invention, with a regression transformation unit, regression transformation data is obtained by substituting the aforementioned logarithmic transformation data in a regression model and executing a regression transformation. A regression transformation is understood as a general meaning, and by analysing the relationship of two quantitative variables x and y employing the regression analysis method, indicates a formulation of the relationship between two variables by the function of

$$y = f(x) \qquad (1).$$

In one embodiment of the present invention, the regression transformation includes:

> multiplying a regression coefficient;
> obtaining a logit value showing a predictive result of a fibrogenesis stage by adding an intercept value at each fibrogenesis stage;
> calculating a predictive probability of each fibrogenesis stage by performing an inverse transformation of a logistic transformation on the logit value and transforming it to a probability value of between 0 and 1; and
> configuring the fibrogenesis stage with the highest probability amongst the predictive probabilities of each fibrogenesis stage, to be a predictive value. **In** one embodiment of the present invention,
> provided is the aforementioned data processing device to perform data processing for detecting liver disease, where the regression transformation includes:

> > multiplying a regression coefficient;
> > obtaining a logit value showing a predictive result of a fibrogenesis stage by adding an intercept value at each fibrogenesis stage;
> > calculating a predictive probability of each fibrogenesis stage by performing an inverse transformation of a logistic transformation on the logit value and transforming it to a probability value of between 0 and 1; and
> > configuring the fibrogenesis stage with the highest probability amongst the predictive probabilities of each fibrogenesis stage, to be a predictive value.

The aforementioned regression coefficient is understood as a general meaning, and in a regression analysis, indicates the inclination of a straight line expressed by a regression formula (regression model) on a coordinate plane, and also indicates coefficient $\alpha$, when the average relationship of variable x (explanation variable) which serves as a cause and variable y (objective variable) serves as a result were expressed by linear formula $y = \alpha x + \beta$. $\beta$ indicates an intercept. In the present invention, the predictive value of a fibrogenesis stage is calculated using the regression coefficient produced by a regression model.

In the present invention, the aforementioned fibrogenesis stage indicates the fibrogenesis stages of the liver. Specifically, the aforementioned fibrogenesis stages are divided into five stages from no fibrogenesis seen until the attainment of hepatic cirrhosis, which are F0 (no fibrogenesis seen), F1 (mild), F2 (moderate), F3 (severe) and F4 (hepatic cirrhosis), as mentioned above for the hepatic fibrogenesis stage classification.

The aforementioned logit value is understood as a general meaning, and is a type of value tallied from probability p which takes the range from 0 to 1. The logit value is 0 when the probability is 0.5, and when the probability is greater than 0.5 it changes towards the infinitely large direction, and when the probability is less than 0.5 it changes towards the negative infinitely large direction. The logit value logit (p) based on probability p is defined by Formula (2) mentioned below. The below Formula (2) is also called a logit function.

$$\text{logit (p)} = \ln\,(p/(1\text{-}p)) \qquad (2)$$

In the formula, p is a probability variable, and ln is a natural logarithmic function. p/(1-p) is called odds. Namely, the p/(1-p) which took the logarithm of the odds is a logit value.

The logistic transformation is understood as a general meaning, and indicates a process of obtaining a logit from probability p by the aforementioned Formula (2). Also, an inverse transformation of a logistic transformation is understood as a general meaning, and indicates a process of obtaining a probability value from logit value a, which is expressed by Formula (3) mentioned below. The below Formula (3) is also called a logistic function.

$$\text{expit (a)} = 1/(1\text{+}e^{\text{-a}}) \qquad (3)$$

In the formula, a is a logit value, and expit (a) is a probability value. In the present invention, this probability value was configured as the predictive probability of each fibrogenesis stage. Also, the fibrogenesis stage with the highest probability amongst the predictive probabilities of each fibrogenesis stage was configured to be a predictive value.

The aforementioned regression model is also called a regression formula, and indicates, from amongst formulae expressing a relationship of certain two variables, a formula estimated by a statistical method. In one embodiment of the present invention, the aforementioned regression model may be selected from the group consisting of a multinominal LASSO model, gaussian LASSO (Least Absolute Shrinkage and Selection Operator) model, and ordinal logistic regression model. In one embodiment of the present invention, provided is the aforementioned data processing device to perform data processing for detecting liver disease, where the aforementioned regression model is selected from the group consisting of a multinominal LASSO model, gaussian LASSO (Least Absolute Shrinkage and Selection Operator) model, and ordinal logistic regression model.

The multinominal LASSO model indicates a polynomial logistic regression model which predicts three or more types of categories whilst performing variable selection. The polynomial logistic regression model combines a binomial logistic regression model, of which one amongst the three or more types of categories was configured as a reference, and creates only the number thereof. A predictive probability of each category is calculated from the created binomial logistic regression models, and the category with the highest predictive probability is configured as the predictive value. In the present invention, the five stages of fibrogenesis mentioned above were analysed as a category when employing the multinominal LASSO model. Cross-Validation by the Leave One Out method (hereinunder described as LOO-CV) was performed for variable selection. The flow of statistical analysis processing by means of multinomial LASSO is shown below.

[Table 3]

Flow of statistical analysis processing by means of multinomial LASSO
▽ START
├ Configure an analysis matrix into a target, variable selection by means of LOO-CV LASSO
├ Drawing of residual curve and solution path by the model created by LOO-CV
├ Save the regression coefficient of lambda min of the model created by LOO-CV
├ Predict fibrogenesis stage from the model created with LOO-CV
△ END

The gaussian LASSO (Least Absolute Shrinkage and Selection Operator) model indicates a linear regression model which predicts an objective variable whilst performing variable selection. The linear regression model is expressed by the below Formula (4).

$$y = \alpha_1 x_1 + \alpha_2 x_2 + ... + \beta \qquad (4)$$

This formula expressed the average relationship of variable x (explanation variable) which serves as a cause and quantitative variable y (objective variable) which serves as a result, where $\alpha$ indicates a coefficient and $\beta$ indicates an intercept. In this case, a linear score is calculated. In the present invention, when employing the gaussian LASSO model, the five stages of fibrogenesis mentioned above were analysed as objective variables, and a fibrogenesis stage was predicted using a cutoff value from the calculated predicted score. The flow of statistical analysis processing by means of gaussian LASSO is shown below.

[Table 4]

Flow of statistical analysis processing by means of gaussian LASSO
▽ START
├ Configure an analysis matrix into a target, variable selection by means of LOO-CV LASSO
├ Drawing of residual curve and solution path by the model created by LOO-CV
├ Save the regression coefficient of lambda min of the model created by LOO-CV
├ Predict the linear score from the model created by LOO-CV
├ Calculate cut-off value of the linear score from the ROC curved line
├ Predict the fibrogenesis stage by using the cut-off value calculated
△ END

The ordinal logistic regression model indicates a regression model which predicts a category with three or more types of ordinal relationships. The ordinal logistic regression model performs a comparison on the entirety of category groups, and configures the category with the highest predictive probability as a predictive value. **In** the present invention, when employing the ordinal logistic regression model, the five stages of fibrogenesis mentioned above were analysed as categories with an ordinal relationship, and a miRNA selected by multinomial LASSO and gaussian LASSO was utilized as an explanation variable. The flow of statistical analysis processing by means of ordinal logistic regression is shown below.

[Table 5]

Flow of statistical analysis processing by means of ordinal logistic regression
▽ START
├ Configure an analysis matrix into a target, and create a model with ordinal logistic regression
├ Save regression coefficient of the created model
├ Predict fibrogenesis stage from the model created
△ END

In the present invention, when employing the "ordinal logistic regression model 1", a miRNA which was selected in common by both multinomial LASSO and gaussian LASSO was utilized as the explanation variable. Moreover, in the present invention, when employing the "ordinal logistic regression model 2", a miRNA which was selected by multinomial LASSO and/or gaussian LASSO was utilized as the explanation variable.

In one embodiment of the present invention, training data is acquired with a training data acquisition unit. In the present invention, the training data are the training data for showing a relationship between a blood miRNA concentration for learning, and a fibrogenesis stage corresponding to the blood miRNA concentration for learning. In one embodiment of the present invention, provided is the aforementioned data processing device to perform data processing for detecting liver disease, where the training data are the training data for showing a relationship between a blood miRNA concentration for learning, and a fibrogenesis stage corresponding to the blood miRNA concentration for learning.

In one embodiment of the present invention, a learnt model is created with a learnt model creation unit, using the aforementioned regression transformation data as training data. In the present invention, the training data are the training data for showing a relationship between a blood miRNA concentration for learning, and a fibrogenesis stage corresponding to the blood miRNA concentration for learning. With the data processing device to perform data processing for detecting liver disease of the present invention, training data processing was implemented using the learnt model thus obtained.

In one embodiment of the present invention, the data processing for detecting liver disease further includes a training data acquisition step for acquiring training data, and a learning data creation step for creating learning data utilizing the training data. In this embodiment, the training data are training data for showing a relationship between a blood miRNA concentration for learning, and a fibrogenesis stage corresponding to the blood miRNA concentration for learning. In one embodiment of the present invention, provided is the aforementioned data processing device to perform data processing for detecting liver disease, where the data processing for detecting liver disease further includes a training data acquisition step for acquiring training data, and a learning data creation step for creating learning data utilizing the training data, where the training data are training data for showing a relationship between a blood miRNA concentration for learning, and a fibrogenesis stage corresponding to the blood miRNA concentration for learning.

In one embodiment of the present invention,
the data processing for detecting liver disease further includes a pre-processing step for obtaining data for analysis, and the pre-processing step includes:

obtaining a low expression miRNA list by listing low expression miRNA;
obtaining a missing value supplemental data by supplementing a missing value in the data of the miRNA list;
obtaining data for transformation by excluding low expression miRNA included in the low expression miRNA list from an analysis target; and
logarithmically transforming the data for transformation. In one embodiment of the present invention,
provided is the aforementioned data processing device to perform data processing for detecting liver disease, where the data processing for detecting liver disease further includes a pre-processing step for obtaining data for analysis, and the pre-processing step includes:

obtaining a low expression miRNA list by listing low expression miRNA;
obtaining a missing value supplemental data by supplementing a missing value in the data of the miRNA list;
obtaining data for transformation by excluding low expression miRNA included in the low expression miRNA list from an analysis target; and
logarithmically transforming the data for transformation.

The aforementioned low expression miRNA indicates miRNA with little expression levels. By listing low expression miRNA to obtain a low expression miRNA list, low expression miRNA contained in the low expression miRNA list can be excluded from the data for transformation and can be removed from an analysis target.

The logarithmic transformation is as mentioned above.

In one embodiment of the present invention, the miRNA in the aforementioned data processing device to perform data processing for detecting liver disease is selected from the group consisting of SEQ ID NOs: 1 to 125. In one embodiment of the present invention, provided is the aforementioned data processing device to perform data processing for detecting liver disease, where the miRNA is selected from the group consisting of SEQ ID NOs: 1 to 125.

In one embodiment of the present invention, the miRNA in the aforementioned data processing device to perform data processing for detecting liver disease is selected from the group consisting of SEQ ID NOs: 1 to 82. In one embodiment of the present invention, provided is the aforementioned data processing device to perform data processing for detecting liver disease, where the miRNA is selected from the group consisting of SEQ ID NOs: 1 to 82.

In one embodiment of the present invention, the miRNA in the aforementioned data processing device to perform data processing for detecting liver disease is selected from the group consisting of SEQ ID NOs: 23, 30, 35, 36, 43, 65, 73, 81, 83 to 125. In one embodiment of the present invention, provided is the aforementioned data processing device to perform data processing for detecting liver disease, where the miRNA is selected from the group consisting of SEQ ID NOs: 23, 30, 35, 36, 43, 65, 73, 81, 83 to 125.

Each miRNA, biological sample, and liver disease are as mentioned above.

Examples

The present invention is specifically explained below by the examples; however, this does not restrict the scope of the present invention.

Example 1. Analysis of miRNA using the RNA seq method

Method:

(1) Preparation of an exosome fraction

17 specimens of serum and 3 specimens of normal serum (Table 3) respectively obtained from patients having differing fibrogenesis stages and Child-Pugh scores underwent ultracentrifugation, and the exosome fractions were purified. Firstly, the serum underwent centrifugal treatment at 2,000 g for 10 minutes, and the obtained supernatant further underwent centrifugal treatment at 12,000 g for 30 minutes. Afterwards, the supernatant thereby obtained underwent centrifugal treatment at 110,000 g for 70 minutes, and the precipitation fractions ultimately obtained were configured as the exosome fractions.

[Table 6]

| No. | Sample | Amount(uL) | Cause | Fibrogenesis stage | Child Pugh Score | Age |
|---|---|---|---|---|---|---|
| 1 | Normal serum 1 | 300 | | | | 71 |
| 2 | Normal serum 2 | 300 | | | | 45 |
| 3 | Normal serum 3 | 300 | | | | 36 |
| 4 | YK-05 | 300 | HCV | 4 | 6 | 61.4 |
| 5 | YK-01 | 300 | HCV | 4 | 7 | 71.2 |
| 6 | YK-02 | 300 | HCV | 4 | 7 | 61.6 |
| 7 | YK-03 | 300 | HBV | 4 | 6 | 72.6 |
| 8 | YK-04 | 300 | HBV | 4 | 6 | 61.0 |
| 9 | YK-06 | 300 | HCV | 4 | 6 | 71.0 |
| 10 | YK-07 | 300 | HCV | 4 | 9 | 57.7 |
| 11 | YK-08 | 300 | HBV | 4 | 8 | 79.2 |
| 12 | YK-09 | 300 | HCV | 4 | 9 | 53.8 |
| 13 | YK-10 | 300 | NBNC | 4 | 11 | 68.7 |
| 14 | YK-11 | 300 | NBNC | 4 | 10 | 62.6 |
| 15 | YK-12 | 300 | HBV | 0 | 0 | 50.6 |
| 16 | YK-13 | 300 | HBV | 0 | 0 | 30.4 |
| 17 | YK-14 | 300 | HBV | 0 | 0 | 41.0 |
| 18 | YK-15 | 300 | HBV | 0 | 0 | 52.8 |
| 19 | YK-16 | 300 | HCV | 0 | 0 | 77.2 |
| 20 | YK-17 | 300 | HCV | 1 | 0 | 61.9 |

(2) Analysis of miRNA

RNA was purified and extracted from the aforementioned exosome fractions, and analysis of miRNA by means of RNA sequence determination (RNA-seq) was performed. Then, for these miRNAs, analysis was implemented for the correlational relationship of changes between non-infection, and pathologies such as HBV infection, HCV infection etc. as well as changes in the fibrogenesis stage and Child-Pugh score, and changes in miRNA expression levels.

Result:

(1) Comparison of miRNA expression level in subjects whose fibrogenesis stage is 0, and those whose stages are 1 to 4

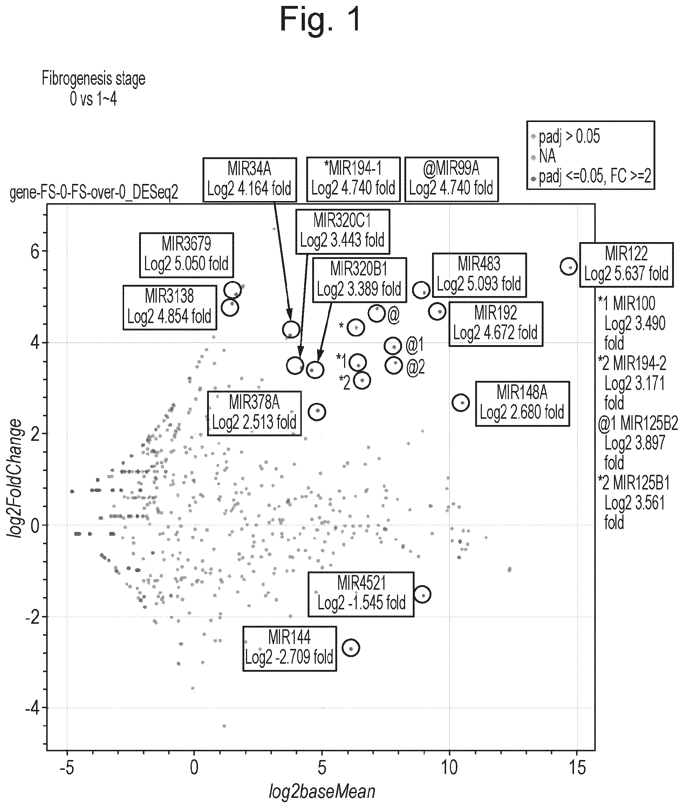

For subjects whose fibrogenesis stage is 0, and subjects whose fibrogenesis stages are 1 to 4, the relationship between a variety of miRNA expression levels and fibrogenesis stages were analysed and shown in Fig. 1. Of the spots shown in Fig. 1, those circled in black correspond to miRNA having shown significant expression changes. Moreover, those surrounded by square black bolded lines as illustrated in the upper half of Fig. 1 have significantly raised expressions, and those surrounded by square black thin lines as illustrated in the lower half of Fig. 1 have significantly reduced expressions. In those with significantly raised expressions, MIR122 (log2 5.637 fold), MIR483 (Log2 5.093 fold) are the highest, and following this are MIR3679 (log2 5.050 fold), MIR3138 (log2 4.854 fold), MIR99A (log2 4.740 fold), MIR194-1 (log2 4.740 fold), MIR192 (log2 4.672 fold), MIR34A (log2 4.164 fold), MIR125B2 (log2 3.897 fold), MIR125B1 (log2 3.561 fold), MIR100 (log2 3.490 fold), MIR320C1 (log2 3.443 fold), MIR320B1 (log2 3.389 fold), MIR194-2 (log2 3.171 fold), MIR148A (log2 2.680 fold), MIR378A (log2 2.513 fold). Those with significantly reduced expressions are MIR4521 (log2 -1.545 fold) and MIR144 (log2 -2.709 fold).

(2) Relevance of extent of fibrogenesis and miRNA expression level

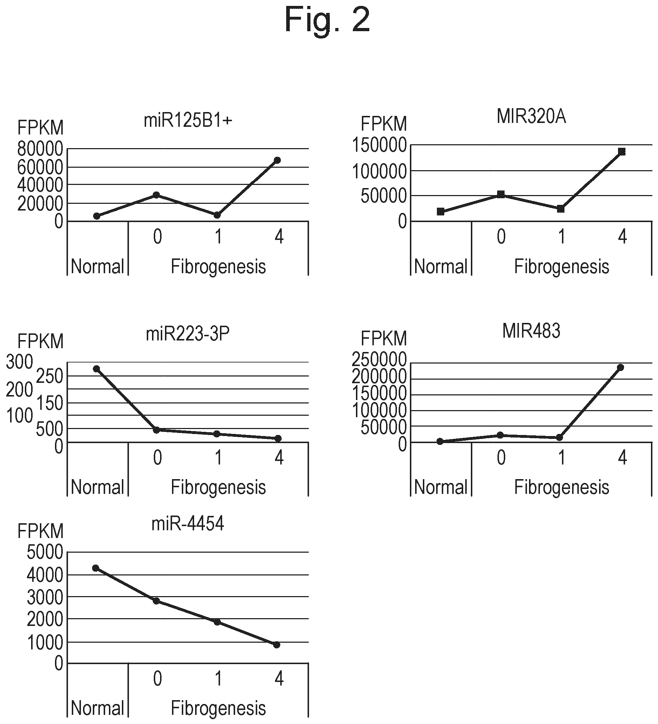

The relationship between a variety of miRNA expression levels and fibrogenesis stages (extent of fibrogenesis) was analysed for normal subjects, subjects whose fibrogenesis stage is 0, subjects whose fibrogenesis stage is 1, and subjects whose fibrogenesis stage is 4, as shown in Fig. 2. The expressions of MIR320A and MIR483 were significantly raised in conjunction with the progression of fibrogenesis, as shown in Fig. 2. For MIR122 (and a similar pattern also for MIR99A and MIR194-1) and MIR125B1 (similarly also for MIR125B2), significant expression rises could be seen when the fibrogenesis stage is 0. A significant reduction of expression level could be seen for MIR223-3P when compared with a normal subject, and a linear reduction of miRNA expression level could be seen for MIR4454. Accordingly, miRNA which can serve as biomarkers could be selected by means of comprehensive analysis using serum samples with differing fibrogenesis stages.

(3) Comparison of miRNA expression level in subjects with a Child-Pugh score 0 and 6 or more

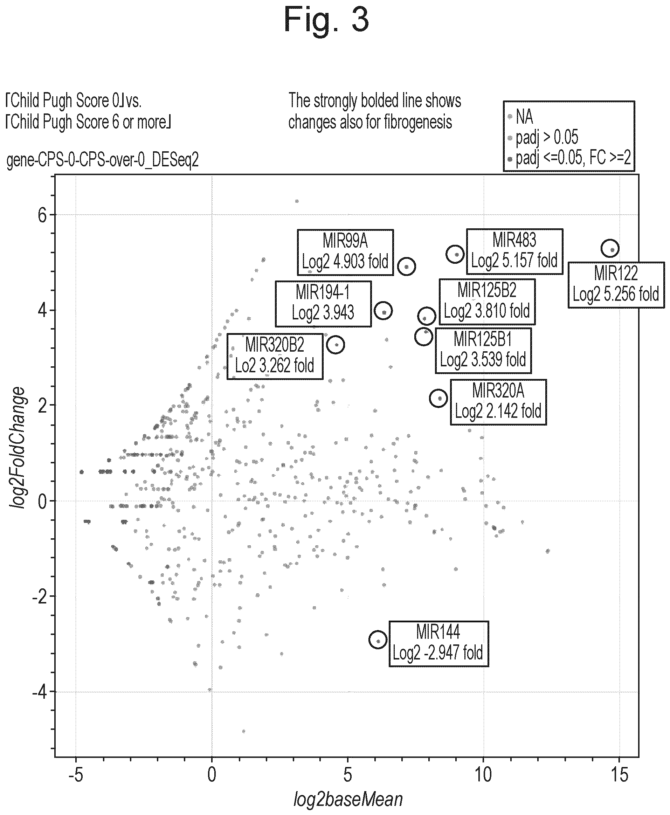

For subjects whose Child-Pugh score (also referred to as "CP score", "CP" or "CPF") is 0 and those whose CP score is 6 or more, the relationship of a variety of miRNA and CP scores were analysed and shown in Fig. 3. Of the spots shown in Fig. 3, those circled in black correspond to miRNA having shown significant expression changes. Moreover, those surrounded by square black bolded lines have significantly raised expressions, and that surrounded by a square black thin line has a significantly reduced expression. As shown in Fig. 3, those with significantly raised expressions were MIR122 (log2 5.256 fold), MIR483 (log2 5.157 fold), MIR99A (log2 4.903 fold), MIR194-1 (log2 3.943 fold), MIR125B2 (log2 3.810 fold), MIR125B1 (log2 3.539 fold), MIR320B2 (log2 3.262 fold), MIR320A (log2 2.142 fold); and that with a significantly reduced expression was MIR144.

(4) Relevance with Child-Pugh score

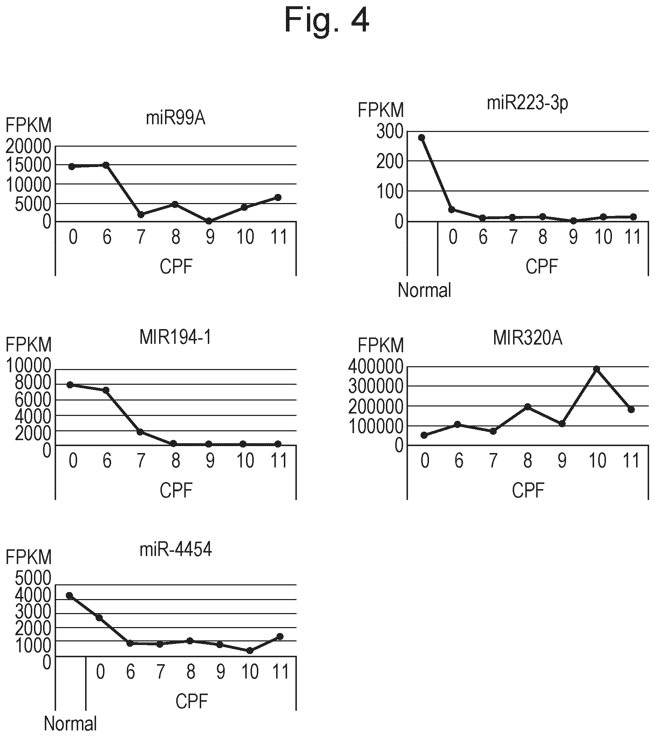

For subjects whose CP score is 0 and subjects whose CP score is 6 to 11, the relationship of a variety of miRNA expression levels and CP scores were analysed and shown in Fig. 4. For MIR194-1, a reduction of miRNA expression could be seen together with a reduction of CP score, as shown in Fig. 4. Although not quite a straight-line pattern could be seen for MIR320A, a tendency towards a rise of miRNA expression level could be seen until CP score 10. A tendency towards a reduction of miRNA expression level could be seen for MIR99A, and a significant reduction of expression level when compared with a normal subject could be seen for MIR4454 and MIR223-3P. Accordingly, miRNA which can serve as biomarkers could be selected by means of comprehensive analysis using serum samples with differing CP scores.

Example 2. Analysis of miRNA by means of 3D-Gene (registered trademark)

Method:

(1) Preparation of an exosome fraction

Same as the aforementioned, for 300 μL of 17 specimens of serum and 2 specimens of normal serum respectively obtained from patients having differing fibrogenesis stages and CP scores, exosome fractions were purified using the same ultracentrifugation technique as that of Example 1.

[Table 7]

| No. | Sample | Amount(uL) | Cause | Fibrogenesis stage | Child Pugh Score | Age |
|---|---|---|---|---|---|---|
| 1 | Normal serum 1 | 300 | | | | 71 |
| 2 | Normal serum 2 | 300 | | | | 45 |
| 3 | YK-05 | 300 | HCV | 4 | 6 | 61.4 |
| 4 | YK-01 | 300 | HCV | 4 | 7 | 71.2 |
| 5 | YK-02 | 300 | HCV | 4 | 7 | 61.6 |
| 6 | YK-03 | 300 | HBV | 4 | 6 | 72.6 |
| 7 | YK-04 | 300 | HBV | 4 | 6 | 61.0 |
| 8 | YK-06 | 300 | HCV | 4 | 6 | 71.0 |
| 9 | YK-07 | 300 | HCV | 4 | 9 | 57.7 |
| 10 | YK-08 | 300 | HBV | 4 | 8 | 79.2 |
| 11 | YK-09 | 300 | HCV | 4 | 9 | 53.8 |
| 12 | YK-10 | 300 | NBNC | 4 | 11 | 68.7 |
| 13 | YK-11 | 300 | NBNC | 4 | 10 | 62.6 |
| 14 | YK-12 | 300 | HBV | 0 | 0 | 50.6 |
| 15 | YK-13 | 300 | HBV | 0 | 0 | 30.4 |
| 16 | YK-14 | 300 | HBV | 0 | 0 | 41.0 |
| 17 | YK-15 | 300 | HBV | 0 | 0 | 52.8 |
| 18 | YK-16 | 300 | HCV | 0 | 0 | 77.2 |
| 19 | YK-17 | 300 | HCV | 1 | 0 | 61.9 |

(2) Analysis of miRNA

From the aforementioned exosome fractions obtained as precipitation fractions, miRNA was purified and the quality of RNA was checked by Bioanalyzer (manufactured by Agilent, 2100), and hybridization was implemented by means of the 3D-Gene (registered trademark) chip by Toray Industries Inc. Analysis was performed for expression level changes of miRNA by means of the 3D-Gene (registered trademark) analytical method. Then, for these miRNAs, analysis was implemented for the correlational relationship of changes between non-infection, and pathologies such as HBV infection, HCV infection etc. as well as changes in the fibrogenesis stage and CP scores, and changes in miRNA expression levels.

Result:

Although many miRNAs which change in conjunction with fibrogenesis stage and CP score were obtained, examples of representative miRNAs include miR-223 and miR-4454 etc. whose RNA expression levels reduce in conjunction with the progression of fibrogenesis stage and increase of CP scores.

Example 3. Correlational analysis between liver pathology and miRNA

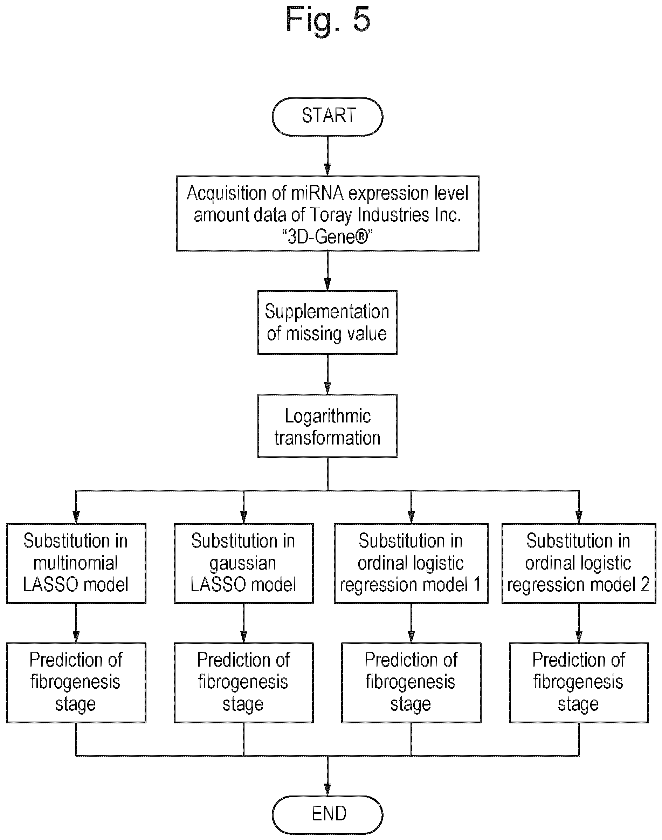

The miRNA samples of viral liver disease (HCV and HBV) patients were used; the miRNA expression level data measured from the aforementioned highly accurate microarray DNA chip "3D-Gene" (registered trademark) developed by Toray Industries Inc was utilized; missing value supplemental data was obtained by supplementing the missing value of this miRNA expression level data by the minimum value of each sample; logarithmic transformation data was obtained by performing a logarithmic transformation, configuring 2 as the base, on this missing value supplemental data; regression coefficients calculated with four regression models on this logarithmic transformation data were used to calculate predictive values of the fibrogenesis stages; and a correlational analysis between liver pathology and miRNA was thereby implemented (Fig. 5).

(1) Correlational analysis using the multinominal LASSO model

For each Stage, the logarithmic transformation data corresponding to each miRNA, and the regression coefficients and intercepts shown in the below Table 3-1 are applied to the below Formula (5), and logit values are thereby obtained.

$$\eta = \alpha_1 x_1 + \alpha_2 x_2 + \ldots + \beta \qquad (5)$$

In the formula, $\alpha$ indicates a coefficient, $\beta$ indicates an intercept, x indicates each miRNA, and $\eta$ indicates a logit value. Performing an inverse transformation of a logistic transformation on this logit value transformed it to a probability value of between 0 and 1, a predictive probability was calculated, and the fibrogenesis stage with the highest probability was configured to be a predictive value.

[Table 8]

Table 3-1 Regression coefficient of multinomial LASSO model

| Variable | Regression coefficient | Stage |
|---|---|---|
| (Intercept) | -1.2405875 | 0 |
| hsa-miR-451a | 1.0690234 | 0 |
| hsa-miR-4258 | 0.251741 | 0 |
| hsa-miR-4648 | 0.2239835 | 0 |
| hsa-miR-204-3p | 0.3352471 | 0 |
| hsa-miR-6126 | 0.1427806 | 0 |
| hsa-miR-6131 | -0.457728 | 0 |
| (Intercept) | 0.20814387 | 1 |
| hsa-miR-1228-3p | 0.47096028 | 1 |
| hsa-miR-1231 | -0.79502634 | 1 |
| hsa-miR-1470 | 0.27690784 | 1 |
| hsa-miR-4286 | 0.21651974 | 1 |
| hsa-miR-3935 | 1.10001108 | 1 |
| hsa-miR-2467-3p | 0.31806064 | 1 |
| hsa-miR-4787-3p | -0.45035088 | 1 |
| hsa-miR-1185-2-3p | 0.1947493 | 1 |
| hsa-miR-6729-3p | 0.01083458 | 1 |
| hsa-miR-6798-3p | 0.17286946 | 1 |
| (Intercept) | 4.55581114 | 2 |
| hsa-miR-379-5p | -0.03277763 | 2 |
| hsa-miR-323a-5p | -0.24120036 | 2 |
| hsa-miR-665 | 0.32473167 | 2 |
| hsa-miR-4279 | -0.01215092 | 2 |
| hsa-miR-3605-5p | -0.01680554 | 2 |
| hsa-miR-4684-3p | 0.15854038 | 2 |
| hsa-miR-365b-5p | -0.01748131 | 2 |
| hsa-miR-6126 | -0.0368392 | 2 |
| hsa-miR-6782-5p | -0.1482362 | 2 |
| hsa-miR-6880-3p | 0.80670205 | 2 |
| hsa-miR-6887-3p | -0.16438501 | 2 |
| hsa-miR-4433b-5p | -0.39232472 | 2 |
| hsa-miR-10396a-3p | 0.04512949 | 2 |
| (Intercept) | 5.2203749 | 3 |
| hsa-miR-373-5p | -0.06275179 | 3 |
| hsa-miR-323a-5p | 0.3099925 | 3 |
| hsa-miR-4539 | 0.15674047 | 3 |
| hsa-miR-4648 | -0.0274626 | 3 |
| hsa-miR-4687-3p | 0.14486852 | 3 |
| hsa-miR-4763-5p | -0.13307243 | 3 |
| hsa-miR-4482-3p | 0.15233675 | 3 |
| hsa-miR-6721-5p | 0.33436027 | 3 |
| hsa-miR-6812-3p | -0.75029949 | 3 |
| hsa-miR-6815-5p | 0.03463929 | 3 |
| hsa-miR-6871-5p | 0.10573189 | 3 |
| hsa-miR-7975 | -0.20824564 | 3 |
| hsa-miR-11181-3p | -0.21093839 | 3 |
| (Intercept) | -8.74374245 | 4 |
| hsa-miR-548d-3p | 0.29016737 | 4 |
| hsa-miR-320b | 0.78443194 | 4 |
| hsa-miR-2278 | 0.76486639 | 4 |
| hsa-miR-3192-5p | 0.0752127 | 4 |
| hsa-miR-4673 | 0.27230154 | 4 |
| hsa-miR-4722-3p | 0.08336756 | 4 |
| hsa-miR-4748 | 0.58964917 | 4 |
| hsa-miR-4750-3p | 0.05766574 | 4 |
| hsa-miR-6752-3p | 0.40515 | 4 |
| hsa-miR-6804-5p | 0.3721713 | 4 |
| hsa-miR-6828-5p | 0.3034243 | 4 |

(2) Correlational analysis using the gaussian LASSO model

The logarithmic transformation data corresponding to each miRNA, and the regression coefficients and intercepts shown in the below Table 3-2 are applied to the below Formula (6), and logit values are thereby obtained.

$$\eta = \alpha_1 x_1 + \alpha_2 x_2 + ... + \beta \qquad (6)$$

In the formula, $\alpha$ indicates a coefficient, $\beta$ indicates an intercept, x indicates each miRNA, and $\eta$ indicates a logit value. These logit values and the cutoff values for each Stage of Table 3-3 were applied to the below conditions, and a fibrogenesis stage which fitted a condition was configured as the predictive value.

- When a logit value is less than the cutoff value of Stage 0|1, the fibrogenesis stage is configured as 0.
- When a logit value is at, or more than, the cutoff value of Stage 0|1, and less than the cutoff value of Stage 1|2, the fibrogenesis stage is configured as 1.
- When a logit value is at, or more than, the cutoff value of Stage 1|2, and less than the cutoff value of Stage 2|3, the fibrogenesis stage is configured as 2.
- When a logit value is at, or more than, the cutoff value of Stage 2|3, and less than the cutoff value of Stage 3|4, the fibrogenesis stage is configured as 3.
- When a logit value is at, or more than, the cutoff value of Stage 3|4, the fibrogenesis stage is configured as 4.

[Table 9]

Table 3-2 Regression coefficient of gaussian LASSO model

| Variable | Regression coefficient |
|---|---|
| (Intercept) | -1.350710378 |
| hsa-miR-451a | -0.235183864 |
| hsa-miR-614 | 0.02051743 |
| hsa-miR-548d-3p | 0.01050913 |
| hsa-miR-320b | 0.220372967 |
| hsa-miR-320c | 0.152649117 |
| hsa-miR-1470 | -0.311829063 |
| hsa-miR-2110 | 0.206137539 |
| hsa-miR-3177-3p | 0.005135416 |
| hsa-miR-4258 | -0.180826728 |
| hsa-miR-4497 | -0.060975626 |
| hsa-miR-4673 | 0.06993303 |
| hsa-miR-4748 | 0.341870552 |
| hsa-miR-4787-3p | 0.016203323 |
| hsa-miR-204-3p | -0.084339806 |
| hsa-miR-6131 | 0.147520971 |
| hsa-miR-210-5p | 0.004433798 |
| hsa-miR-6752-3p | 0.13730913 |
| hsa-miR-6778-5p | -0.185987967 |
| hsa-miR-6780a-5p | 0.011306514 |
| hsa-miR-6801-3p | 0.034059745 |
| hsa-miR-8059 | 0.314218824 |

[Table 10]

Table 3-3 Cut-off value of gaussian LASSO

| Stage | Cutoff |
|---|---|
| 0|1 | 1.716915 |
| 1|2 | 1.983237 |
| 2|3 | 2.288713 |
| 3|4 | 2.389503 |

(3) Correlational analysis using the ordinal logistic regression model 1

For each Stage, the logarithmic transformation data corresponding to each miRNA, and the regression coefficients shown in the below Table 3-4 and intercepts shown in the below Table 3-5 are applied to the below Formula (7), and logit values are thereby obtained.

$$\eta = -\alpha_1 x_1 - \alpha_2 x_2 - \ldots + \beta \qquad (7)$$

In the formula, $\alpha$ indicates a coefficient, $\beta$ indicates an intercept, x indicates each miRNA, and $\eta$ indicates a logit value. Performing an inverse transformation of a logistic transformation on this logit value transformed it to a probability value of between 0 and 1, and a predictive probability was calculated. Furthermore, the difference of predictive probability was calculated for each Stage, and the fibrogenesis stage with the highest probability is configured to be a predictive value. The calculation method of the difference of predictive probability for each Stage is as follows.

- Difference of predictive probability of Stage 0 = 1 - Predictive probability of Stage 0|1
- Difference of predictive probability of Stage 1 = Predictive probability of Stage 0|1 - Predictive probability of Stage 1|2
- Difference of predictive probability of Stage 2 = Predictive probability of Stage 1|2 - Predictive probability of Stage 2|3
- Difference of predictive probability of Stage 3 = Predictive probability of Stage 2|3 - Predictive probability of Stage 3|4
- Difference of predictive probability of Stage 4 = Predictive probability of Stage 3|4

[Table 11]

Table 3-4 Regression coefficient of ordinal logistic regression model 1

| Variable | Regression coefficient |
|---|---|
| hsa-miR-451a | -1.29452 |
| hsa-miR-548d-3p | 1.414791 |
| hsa-miR-320b | 1.153853 |
| hsa-miR-1470 | -2.13089 |
| hsa-miR-4258 | -1.08465 |
| hsa-miR-4673 | 1.279323 |
| hsa-miR-4748 | 1.326245 |
| hsa-miR-4787-3p | 0.844023 |
| hsa-miR-204-3p | -0.87251 |
| hsa-miR-6131 | 0.768582 |
| hsa-miR-6752-3p | 0.857326 |

[Table 12]

Table 3-5 Intercept value of ordinal logistic regression model 1

| Stage | Intercept |
|---|---|
| 0|1 | 3.04316 |
| 1|2 | 5.9857033 |
| 2|3 | 7.589967 |
| 3|4 | 9.1880967 |

(4) Correlational analysis using the ordinal logistic regression model 2

For each Stage, the logarithmic transformation data corresponding to each miRNA, and the regression coefficients shown in the below Table 3-6 and intercepts shown in the below Table 3-7 are applied to the below Formula (8), and logit values are thereby obtained.

$$\eta = -\alpha_1 x_1 - \alpha_2 x_2 - \ldots + \beta \qquad (8)$$

In the formula, $\alpha$ indicates a coefficient, $\beta$ indicates an intercept, x indicates each miRNA, and $\eta$ indicates a logit value.

Performing an inverse transformation of a logistic transformation on this logit value transformed it to a probability value of between 0 and 1, and a predictive probability was calculated. Furthermore, the difference of predictive probability was calculated for each Stage, and the fibrogenesis stage with the highest probability is configured to be a predictive value. The calculation method of the difference of predictive probability for each Stage is as follows.

- Difference of predictive probability of Stage 0 = 1 - Predictive probability of Stage 0|1
- Difference of predictive probability of Stage 1 = Predictive probability of Stage 0|1 - Predictive probability of Stage 1|2
- Difference of predictive probability of Stage 2 = Predictive probability of Stage 1|2 - Predictive probability of Stage 2|3
- Difference of predictive probability of Stage 3 = Predictive probability of Stage 2|3 - Predictive probability of Stage 3|4
- Difference of predictive probability of Stage 4 = Predictive probability of Stage 3|4

[Table 13]

Table 3-6 Regression coefficient of ordinal logistic regression model 2

| Variable | Regression coefficient |
|---|---|
| hsa-miR-451a | -2.341765686 |
| hsa-miR-4258 | -0.142536275 |
| hsa-miR-4648 | 0.874074072 |
| hsa-miR-204-3p | -6.64773221 |
| hsa-miR-6126 | 1.896558345 |
| hsa-miR-6131 | 3.86788476 |
| hsa-miR-1228-3p | -0.128324158 |
| hsa-miR-1231 | 1.429371333 |
| hsa-miR-1470 | -3.155836528 |
| hsa-miR-4286 | 2.719043594 |
| hsa-miR-3935 | -1.10483117 |
| hsa-miR-2467-3p | -5.195466249 |
| hsa-miR-4787-3p | 0.005777377 |
| hsa-miR-1185-2-3p | 2.839101857 |
| hsa-miR-6729-3p | -2.88701171 |
| hsa-miR-6798-3p | -3.909598739 |
| hsa-miR-379-5p | -5.02732803 |
| hsa-miR-323a-5p | -8.323764721 |
| hsa-miR-665 | -5.349681278 |
| hsa-miR-4279 | 2.411340172 |
| hsa-miR-3605-5p | 2.337002272 |
| hsa-miR-4684-3p | 0.70140275 |
| hsa-miR-365b-5p | 2.590978638 |
| hsa-miR-6782-5p | 2.425622138 |
| hsa-miR-6880-3p | -4.463936523 |
| hsa-miR-6887-3p | -0.047208928 |
| hsa-miR-4433b-5p | 2.830282701 |
| hsa-miR-10396a-3p | -0.122288945 |
| hsa-miR-373-5p | -0.856881166 |
| hsa-miR-4539 | -0.677072594 |
| hsa-miR-4687-3p | 4.361757748 |
| hsa-miR-4763-5p | -1.275832702 |
| hsa-miR-4482-3p | 0.794946778 |
| hsa-miR-6721-5p | 1.367070086 |
| hsa-miR-6812-3p | -3.739323328 |
| hsa-miR-6815-5p | -2.05493619 |
| hsa-miR-6871-5p | -0.833563193 |
| hsa-miR-7975 | -2.619386175 |
| hsa-miR-11181-3p | -0.299829175 |
| hsa-miR-548d-3p | 6.898976299 |
| hsa-miR-320b | -1.457827364 |
| hsa-miR-2278 | 8.221423189 |
| hsa-miR-3192-5p | 2.515055782 |
| hsa-miR-4673 | 2.969952745 |
| hsa-miR-4722-3p | 3.249125773 |
| hsa-miR-4748 | 1.897269028 |
| hsa-miR-4750-3p | 1.943587873 |
| hsa-miR-6752-3p | -0.68153125 |
| hsa-miR-6804-5p | 0.680166684 |
| hsa-miR-6828-5p | 4.632258179 |
| hsa-miR-614 | 10.88977045 |
| hsa-miR-320c | 0.844350135 |
| hsa-miR-2110 | -0.106800226 |
| hsa-miR-3177-3p | 2.383208119 |
| hsa-miR-4497 | -3.617908646 |
| hsa-miR-210-5p | -1.635329214 |
| hsa-miR-6778-5p | -2.758146029 |
| hsa-miR-6780a-5p | 1.205833836 |
| hsa-miR-6801-3p | 2.732807417 |
| hsa-miR-8059 | -10.69243304 |

[Table 14]

Table 3-7 Intercept value of ordinal logistic regression model 2

| Stage | Intercept |
|---|---|
| 0\|1 | 6.319394996 |
| 1\|2 | 13.83958127 |
| 2\|3 | 17.82339537 |
| 3\|4 | 21.75057032 |

(5) Discrimination performance

(5-1) Accuracy rate

A contingency table was created with actual groups (Normal, F1: stage 1, F2: stage 2, F3: stage 3, F4: stage 4) and predicted groups, and the concordance rates of the diagonal components thereof were calculated. Furthermore, the accuracy rates of each group from the contingency table were calculated. The accuracy rates when the aforementioned four regression models were used are shown below. It was obvious that sufficiently high accuracy rates are shown for any of the models.

[Table 15]

| stage \ group | multinomial LASSO model | gaussian LASSO model | ordinal.intersect (ordinal logistic regression model 1) | ordinal.union (ordinal logistic regression model 2) |
|---|---|---|---|---|
| 1 Normal | 1.00 | 1.00 | 0.90 | 0.95 |
| 2 F1 | 0.70 | 0.45 | 0.50 | 0.75 |
| 3 F2 | 0.80 | 0.30 | 0.50 | 0.65 |
| 4 F3 | 0.90 | 0.25 | 0.35 | 0.60 |
| 5 F4 | 0.85 | 0.90 | 0.60 | 0.85 |

(5-2) Kappa coefficient

Furthermore, the kappa coefficient (kappa statistic) was calculated. The kappa coefficient is an index of evaluating the matching rate of a category variable with an ordinal relationship, and this coefficient is also called the Cohen's coefficient of agreement. The kappa coefficient generally takes a value of 0 to 1, where the value becomes higher to the extent that the matching rate is large. The kappa coefficient can generally be said to show a sufficiently high performance if it is more than 0.6. Moreover, a kappa coefficient which takes into consideration the weight of an ordinal is called a weighted kappa coefficient. When investigating the matching rate of an ordinal scale such as the extent of a medical symptom, it is suitable to use the weighted kappa coefficient and not the kappa coefficient. In the present invention, the weighted kappa coefficient is used. The weighted kappa coefficients when using the aforementioned four regression models are shown in the table below. It was shown that any of the models have a sufficiently high performance.

[Table 16]

| | model | kappa statistic |
|---|---|---|
| 1 | multinomial LASSO model | 0.87 |
| 2 | gaussian LASSO model | 0.78 |
| 3 | ordinal.intersect (ordinal logistic regression model 1) | 0.82 |
| 4 | ordinal.union (ordinal logistic regression model 2) | 0.94 |

The present inventors found out that by using the miRNA of viral liver disease patients and performing a backwards analysis, a fibrogenesis stage can be non-invasively predicted and assessed by using the miRNA. As mentioned above, according to the present invention, it was shown that by measuring the miRNA of viral liver disease patients and substituting this in a prediction model formula, a fibrogenesis stage can be predicted. Although the present inventors constructed four prediction models, highly accurate predictive results can be obtained by separately using these prediction models as required.

Example 4. Correlational analysis (1) between NASH and miRNA

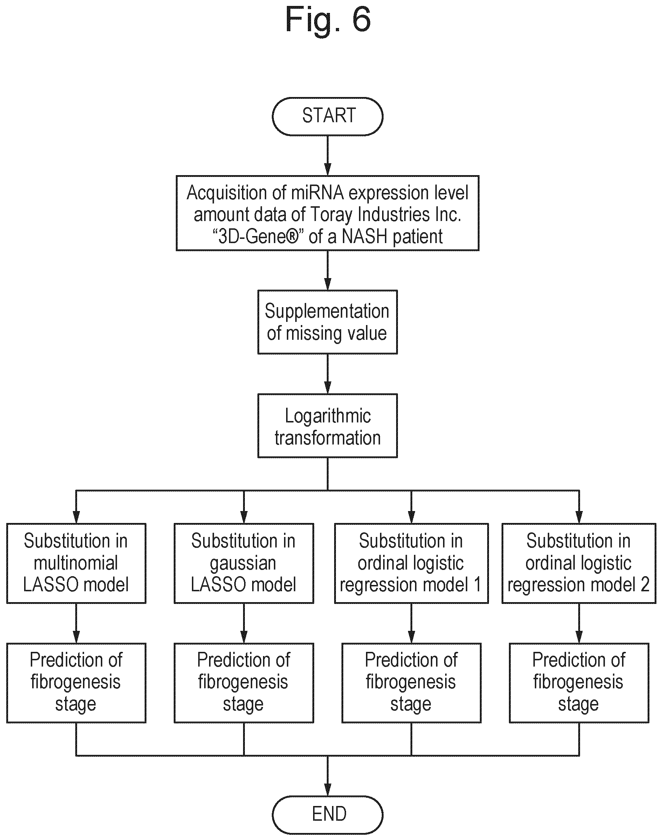

The miRNA samples of NASH patients were used; the miRNA expression level data measured from the aforementioned highly accurate microarray DNA chip "3D-Gene" (registered trademark) developed by Toray Industries Inc was utilized; missing value supplemental data was obtained by supplementing the missing value of this miRNA expression level data by the minimum value of each sample; logarithmic transformation data was obtained by performing a logarithmic transformation, configuring 2 as the base, on this missing value supplemental data; and regression coefficients calculated with four regression models on this logarithmic transformation data were used to calculate predictive values of the fibrogenesis stages of NASH were (Fig. 6).

(1) Correlational analysis using the multinominal LASSO model

For each Stage, the logarithmic transformation data corresponding to each miRNA, and the regression coefficients and intercepts shown in the below Table 4-1 are applied to the below Formula (9), and logit values are thereby obtained.

$$\eta = \alpha_1 x_1 + \alpha_2 x_2 + ... + \beta \qquad (9)$$

In the formula, $\alpha$ indicates a coefficient, $\beta$ indicates an intercept, x indicates each miRNA, and $\eta$ indicates a logit value. Performing an inverse transformation of a logistic transformation on this logit value transformed it to a probability value of between 0 and 1, a predictive probability is calculated, and the fibrogenesis stage with the highest probability was configured to be a predictive value.

[Table 17]

Table 4-1 Regression coefficient of multinomial LASSO model

| Variable | Regression coefficient | Stage |
|---|---|---|
| (Intercept) | 11.6405744 | 0 |
| hsa-miR-614 | -0.8668745 | 0 |
| hsa-miR-3619-3p | -0.4290646 | 0 |
| hsa-miR-6798-3p | -0.2953853 | 0 |
| (Intercept) | -3.871562 | 1 |
| hsa-miR-518d-3p | -0.1174561 | 1 |
| hsa-miR-4458 | 0.08150468 | 1 |
| hsa-miR-4746-3p | 0.29954501 | 1 |
| hsa-miR-8083 | 0.40253772 | 1 |
| (Intercept) | -2,0184714 | 2 |
| hsa-miR-145-5p | 0.00353749 | 2 |
| (Intercept) | -3.1814602 | 3 |
| hsa-miR-6801-5p | 0.4307845 | 3 |
| (Intercept) | -2.5690808 | 4 |
| hsa-miR-127-3p | 0.06108857 | 4 |
| hsa-miR-6748-3p | 0.20842344 | 4 |

(2) Correlational analysis using the gaussian LASSO model

The logarithmic transformation data corresponding to each miRNA, and the regression coefficients and intercepts shown in the below Table 4-2 are applied to the below Formula (10), and logit values are thereby obtained.

$$\eta = \alpha_1 x_1 + \alpha_2 x_2 + ... + \beta \qquad (10)$$

In the formula, α indicates a coefficient, β indicates an intercept, x indicates each miRNA, and η indicates a logit value. These logit values and the cutoff values for each Stage of Table 4-3 were applied to the below conditions, and a fibrogenesis stage which fitted a condition was configured as the predictive value.

- When a logit value is less than the cutoff value of Stage 011, the fibrogenesis stage is configured as 0.
- When a logit value is at, or more than, the cutoff value of Stage 0|1, and less than the cutoff value of Stage 112, the fibrogenesis stage is configured as 1.
- When a logit value is at, or more than, the cutoff value of Stage 112, and less than the cutoff value of Stage 2|3, the fibrogenesis stage is configured as 2.
- When a logit value is at, or more than, the cutoff value of Stage 2|3, and less than the cutoff value of Stage 3|4, the fibrogenesis stage is configured as 3.
- When a logit value is at, or more than, the cutoff value of Stage 3|4, the fibrogenesis stage is configured as 4.

[Table 18]

Table 4-2 Regression coefficient of gaussian LASSO model

| Variable | Regression coefficient |
|---|---|
| (Intercept) | -5.35389548 |
| hsa-miR-4731-3p | 0.15665695 |
| hsa-miR-6798-3p | 0.22425088 |
| hsa-miR-7112-5p | 0.03487231 |
| hsa-miR-12116 | 0.72030644 |

[Table 19]

Table 4-3 Cut-off value of gaussian LASSO

| Stage | Cutoff |
|---|---|
| 0|1 | 1.063741 |
| 1|2 | 1.968709 |
| 2|3 | 1.987465 |
| 3|4 | 2.170295 |

(3) Correlational analysis using the ordinal logistic regression model 1

For each Stage, the logarithmic transformation data corresponding to each miRNA, and the regression coefficient shown in the below Table 4-4 and intercepts shown in the below Table 4-5 are applied to the below Formula (11), and logit values are thereby obtained.

$$\eta = - \alpha_1 x_1 - \alpha_2 x_2 - \ldots + \beta \qquad (11)$$

In the formula, α indicates a coefficient, β indicates an intercept, x indicates each miRNA, and η indicates a logit value. Performing an inverse transformation of a logistic transformation on this logit value transformed it to a probability value of between 0 and 1, and a predictive probability was calculated. Furthermore, the difference of predictive probability was calculated for each Stage, and the fibrogenesis stage with the highest probability is configured to be a predictive value. The calculation method of the difference of predictive probability for each Stage is as follows.

- Difference of predictive probability of Stage 0 = 1 - Predictive probability of Stage 0|1
- Difference of predictive probability of Stage 1 = Predictive probability of Stage 0|1 - Predictive probability of Stage 1|2
- Difference of predictive probability of Stage 2 = Predictive probability of Stage 1|2 - Predictive probability of Stage 2|3
- Difference of predictive probability of Stage 3 = Predictive probability of Stage 2|3 - Predictive probability of Stage 3|4
- Difference of predictive probability of Stage 4 = Predictive probability of Stage 3|4

[Table 20]

Table 4-4 Regression coefficient of ordinal logistic regression model 1

| Variable | Regression coefficient |
|---|---|
| hsa-miR-6798-3p | 4.36204 |

[Table 21]

Table 4-5 Intercept value of ordinal logistic regression model 1

| Stage | Intercept |
|---|---|
| 0\|1 | 19.69343 |
| 1\|2 | 22.533077 |
| 2\|3 | 23.60854 |
| 3\|4 | 25.12942 |

(4) Correlational analysis using the ordinal logistic regression model 2

For each Stage, the logarithmic transformation data corresponding to each miRNA, and the regression coefficients shown in the below Table 4-6 and intercepts shown in the below Table 4-7 are applied to the below Formula (12), and logit values are thereby obtained.

$$\eta = -\alpha_1 x_1 - \alpha_2 x_2 - \ldots + \beta \qquad (12)$$

In the formula, $\alpha$ indicates a coefficient, $\beta$ indicates an intercept, x indicates each miRNA, and $\eta$ indicates a logit value. Performing an inverse transformation of a logistic transformation on this logit value transformed it to a probability value of between 0 and 1, and a predictive probability was calculated. Furthermore, the difference of predictive probability was calculated for each Stage, and the fibrogenesis stage with the highest probability is configured to be a predictive value. The calculation method of the difference of predictive probability for each Stage is as follows.

- Difference of predictive probability of Stage 0 = 1 - Predictive probability of Stage 0\|1
- Difference of predictive probability of Stage 1 = Predictive probability of Stage 0\|1 - Predictive probability of Stage 1\|2
- Difference of predictive probability of Stage 2 = Predictive probability of Stage 1\|2 - Predictive probability of Stage 2\|3
- Difference of predictive probability of Stage 3 = Predictive probability of Stage 2\|3 - Predictive probability of Stage 3\|4
- Difference of predictive probability of Stage 4 = Predictive probability of Stage 3\|4

[Table 22]

Table 4-6 Regression coefficient of ordinal logistic regression model 2

| Variable | Regression coefficient |
|---|---|
| hsa-miR-614 | 0.729658803 |
| hsa-miR-3619-3p | 0.560642932 |
| hsa-miR-6798-3p | 3.089674317 |
| hsa-miR-518d-3p | -1.10305039 |
| hsa-miR-4458 | -1.089558078 |
| hsa-miR-4746-3p | -1.227759471 |
| hsa-miR-8083 | 0.11360011 |
| hsa-miR-145-5p | -0.661597286 |
| hsa-miR-6801-5p | -0.595120758 |
| hsa-miR-127-3p | 0.414387738 |
| hsa-miR-6748-3p | 1.941972456 |
| hsa-miR-4731-3p | 0.705803215 |
| hsa-miR-7112-5p | 2.642584241 |
| hsa-miR-12116 | -0.657528884 |

[Table 23]

Table 4-7 Intercept value of ordinal logistic regression model 2

| Stage | Intercept |
|---|---|
| 0\|1 | 25.37917954 |
| 1\|2 | 29.89401258 |
| 2\|3 | 31.32460877 |
| 3\|4 | 33.47041722 |

(5) Discrimination performance

(5-1) Accuracy rate

A contingency table was created with actual groups (0: normal, 1: stage 1, 2: stage 2, 3: stage 3, 4: stage 4) and predicted groups, and the concordance rates of the diagonal components thereof were calculated. Furthermore, the accuracy rates of each group from the contingency table were calculated. The accuracy rates when the aforementioned four regression models were used are shown below. It was obvious that sufficiently high accuracy rates are shown for any of the models.

[Table 24]

| group<br><br>stage | multinomial LASSO model | gaussian LASSO model | ordinal.intersect (ordinal logistic regression model 1) | ordinal.union (ordinal logistic regression model 2) |
|---|---|---|---|---|
| 1 Normal | 1.00 | 1.00 | 1.00 | 1.00 |
| 2 F1 | 0.80 | 0.80 | 1.00 | 1.00 |
| 3 F2 | 0.00 | 0.17 | 0.00 | 0.33 |
| 4 F3 | 0.80 | 0.30 | 0.40 | 0.40 |
| 5 F4 | 0.25 | 0.88 | 0.38 | 0.75 |

(5-2) Kappa coefficient

The weighted kappa coefficients when using the aforementioned four regression models are shown in the table below. It was shown that any of the models have a sufficiently high performance.

[Table 25]

| | model | kappa statistic |
|---|---|---|
| 1 | multinomial LASSO model | 0.68 |
| 2 | gaussian LASSO model | 0.85 |
| 3 | ordinal.intersect (ordinal logistic regression model 1) | 0.73 |
| 4 | ordinal.union (ordinal logistic regression model 2) | 0.87 |

The present inventors found out that by using the miRNA of NASH patients and performing a backwards analysis, a fibrogenesis stage can be non-invasively predicted and assessed by using the miRNA. As mentioned above, according to the present invention, it was shown that by measuring the miRNA of NASH patients and substituting this in a prediction model formula, a fibrogenesis stage can be predicted. Although the present inventors constructed four prediction models, highly accurate predictive results can be obtained by separately using these prediction models as required.

Example 5. Correlational analysis (2) between NASH and miRNA

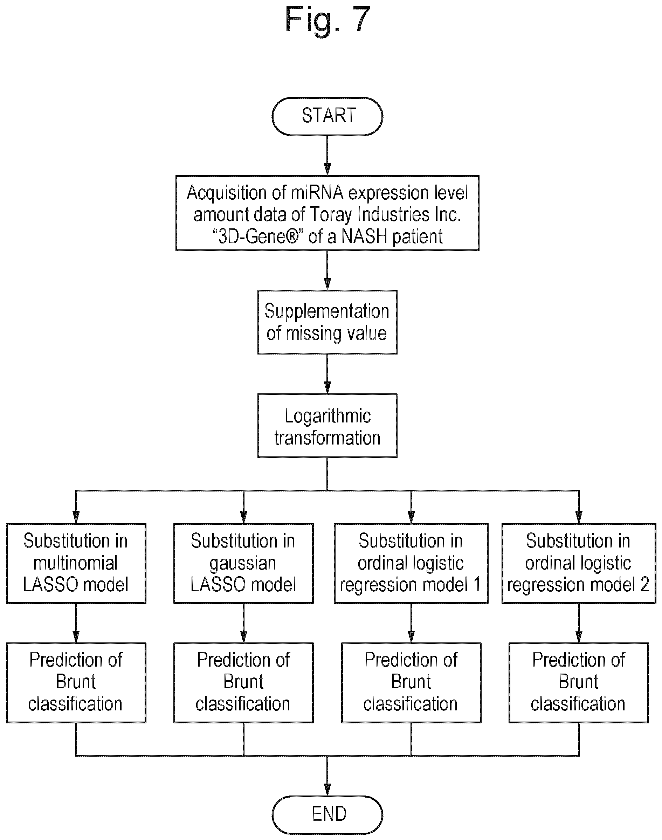

The miRNA samples of NASH patients were used; the miRNA expression level data measured from the aforementioned highly accurate microarray DNA chip "3D-Gene" (registered trademark) developed by Toray Industries Inc was utilized; missing value supplemental data was obtained by supplementing the missing value of this miRNA expression level data by the minimum value of each sample; logarithmic transformation data was obtained by performing a logarithmic transformation, configuring 2 as the base, on this missing value supplemental data; and regression coefficients calculated with four regression models on this logarithmic transformation data were used to calculate predictive values of the Brunt classifications of NASH were (Fig. 7).

(1) Correlational analysis using the multinominal LASSO model

For each Stage, the logarithmic transformation data corresponding to each miRNA, and the regression coefficients and intercepts shown in the below Table 5-1 are applied to the below Formula (13), and logit values are thereby obtained.

$$\eta = \alpha_1 x_1 + \alpha_2 x_2 + ... + \beta \qquad (13)$$

In the formula, $\alpha$ indicates a coefficient, $\beta$ indicates an intercept, x indicates each miRNA, and $\eta$ indicates a logit value. Performing an inverse transformation of a logistic transformation on this logit value transformed it to a probability value of between 0 and 1, a predictive probability was calculated, and the Brunt classification with the highest probability was configured to be a predictive value.

[Table 26]

Table 5-1 Regression coefficient of multinomial LASSO model

| Variable | Regression coefficient | Stage |
|---|---|---|
| (Intercept) | 15.5612526 | 0 |
| hsa-miR-614 | -0.9554465 | 0 |
| hsa-miR-3619-3p | -0.8563155 | 0 |
| hsa-miR-4646-5p | -0.0420774 | 0 |
| hsa-miR-6798-3p | -0.2783515 | 0 |
| (Intercept) | -3.7813318 | 1 |
| hsa-miR-525-5p | 0.06160089 | 1 |
| hsa-miR-657 | -0.3474242 | 1 |
| hsa-miR-921 | 0.11560258 | 1 |
| hsa-miR-4444 | 0.1087084 | 1 |
| hsa-miR-4458 | 0.21362495 | 1 |
| hsa-miR-4746-3p | 0.04526107 | 1 |
| hsa-miR-1292-3p | 0.00890983 | 1 |
| hsa-miR-6133 | 0.24458344 | 1 |
| (Intercept) | -4.5502661 | 2 |
| hsa-miR-6754-3p | 0.3541885 | 2 |
| hsa-miR-7975 | 0.1517134 | 2 |
| (Intercept) | -0.6425153 | 3 |
| hsa-miR-365a-3p,hsa-miR-365b-3p | 0.36075146 | 3 |
| hsa-miR-451a | -0.0307741 | 3 |
| hsa-miR-6748-3p | -0.2625393 | 3 |
| hsa-miR-6892-3p | 0.17014593 | 3 |
| hsa-miR-8083 | -0.6083296 | 3 |
| (Intercept) | -6.5871393 | 4 |
| hsa-miR-525-5p | -0.2465414 | 4 |
| hsa-miR-4451 | 0.01508251 | 4 |
| hsa-miR-4724-5p | 0.82138378 | 4 |
| hsa-miR-6801-3p | -0.091803 | 4 |
| hsa-miR-6884-5p | 0.45997636 | 4 |
| hsa-miR-7156-3p | 0.26980121 | 4 |

(continued)

Table 5-1 Regression coefficient of multinomial LASSO model

| Variable | Regression coefficient | Stage |
|---|---|---|
| hsa-miR-3059-3p | 0.26717762 | 4 |

(2) Correlational analysis using the gaussian LASSO model

The logarithmic transformation data corresponding to each miRNA, and the regression coefficients and intercepts shown in the below Table 5-2 are applied to the below Formula (14), and logit values are thereby obtained.

$$\eta = \alpha_1 x_1 + \alpha_2 x_2 + ... + \beta \qquad (14)$$

In the formula, $\alpha$ indicates a coefficient, $\beta$ indicates an intercept, x indicates each miRNA, and $\eta$ indicates a logit value. These logit values and the cutoff values for each Stage of Table 5-3 were applied to the below conditions, and a Brunt classification which fitted a condition was configured as the predictive value.

- When a logit value is less than the cutoff value of Stage 0|1, the Brunt classification is configured as 0.
- When a logit value is at, or more than, the cutoff value of Stage 0|1, and less than the cutoff value of Stage 1|2, the Brunt classification is configured as 1.
- When a logit value is at, or more than, the cutoff value of Stage 1|2, and less than the cutoff value of Stage 2|3, the Brunt classification is configured as 2.
- When a logit value is at, or more than, the cutoff value of Stage 2|3, and less than the cutoff value of Stage 3|4, the Brunt classification is configured as 3.
- When a logit value is at, or more than, the cutoff value of Stage 3|4, the Brunt classification is configured as 4.

[Table 27]

Table 5-2 Regression coefficient of gaussian LASSO model

| Variable | Regression coefficient |
|---|---|
| (Intercept) | -4.779808523 |
| hsa-miR-19b-3p | -0.000265714 |
| hsa-miR-518e-3p | 0.011816696 |
| hsa-miR-646 | 0.040751163 |
| hsa-miR-657 | 0.266816476 |
| hsa-miR-298 | -0.048239892 |
| hsa-miR-921 | -0.02169432 |
| hsa-miR-1228-3p | 0.081508562 |
| hsa-miR-1234-3p | -0.179207017 |
| hsa-miR-2115-5p | 0.09745127 |
| hsa-miR-3142 | 0.051290355 |
| hsa-miR-3179 | -0.160447176 |
| hsa-miR-3 190-5p | 0.04655078 |
| hsa-miR-3675-3p | 0.037102567 |
| hsa-miR-4441 | -0.19383846 |
| hsa-miR-4451 | 0.009226911 |
| hsa-miR-4475 | 0.035283961 |
| hsa-miR-4787-3p | -0.136441088 |
| hsa-miR-6126 | -0.202554499 |
| hsa-miR-6718-5p | -0.219094751 |
| hsa-miR-6743-3p | -0.042356173 |
| hsa-miR-6798-3p | 0.198351564 |
| hsa-miR-6801-3p | -0.062033004 |
| hsa-miR-6894-3p | -0.181906101 |

(continued)

Table 5-2 Regression coefficient of gaussian LASSO model

| Variable | Regression coefficient |
|---|---|
| hsa-miR-7112-5p | 0.2129558 |
| hsa-miR-3059-3p | 0.616784778 |
| hsa-miR-12116 | 0.80832969 |

[Table 28]

Table 5-3 Cut-off value of gaussian LASSO

| Stage | Cutoff |
|---|---|
| 0\|1 | 0.750386 |
| 1\|2 | 1.819986 |
| 2\|3 | 2.238632 |
| 3\|4 | 3.10229 |

(3) Correlational analysis using the ordinal logistic regression model 1

For each Stage, the logarithmic transformation data corresponding to each miRNA, and the regression coefficients shown in the below Table 5-4 and intercepts shown in the below Table 5-5 are applied to the below Formula (15), and logit values are thereby obtained.

$$\eta = -\alpha_1 x_1 - \alpha_2 x_2 - \ldots + \beta \qquad (15)$$

In the formula, $\alpha$ indicates a coefficient, $\beta$ indicates an intercept, x indicates each miRNA, and $\eta$ indicates a logit value. Performing an inverse transformation of a logistic transformation on this logit value transformed it to a probability value of between 0 and 1, and a predictive probability was calculated. Furthermore, the difference of predictive probability was calculated for each Stage, and the Brunt classification with the highest probability is configured to be a predictive value. The calculation method of the difference of predictive probability for each Stage is as follows.

- Difference of predictive probability of Stage 0 = 1 - Predictive probability of Stage 0|1
- Difference of predictive probability of Stage 1 = Predictive probability of Stage 0|1 - Predictive probability of Stage 1|2
- Difference of predictive probability of Stage 2 = Predictive probability of Stage 1|2 - Predictive probability of Stage 2|3
- Difference of predictive probability of Stage 3 = Predictive probability of Stage 2|3 - Predictive probability of Stage 3|4
- Difference of predictive probability of Stage 4 = Predictive probability of Stage 3|4

[Table 29]

Table 5-4 Regression coefficient of ordinal logistic regression model 1

| Variable | Regression coefficient |
|---|---|
| hsa-miR-6798-3p | 5.951227 |
| hsa-miR-657 | 1.267299 |
| hsa-miR-921 | -1.6656 |
| hsa-miR-4451 | 0.96452 |
| hsa-miR-6801-3p | -0.73976 |
| hsa-miR-3059-3p | 2.888619 |

[Table 30]

Table 5-5 Intercept value of ordinal logistic regression model 1

| Stage | Intercept |
|---|---|
| 0\|1 | 33.512154 |
| 1\|2 | 36.723854 |
| 2\|3 | 38.567314 |

(continued)

Table 5-5 Intercept value of ordinal logistic regression model 1

| Stage | Intercept |
|---|---|
| 3\|4 | 42.926782 |

(4) Correlational analysis using the ordinal logistic regression model 2

For each Stage, the logarithmic transformation data corresponding to each miRNA, and the regression coefficients shown in the below Table 5-6 and intercepts shown in the below Table 5-7 are applied to the below Formula (16), and logit values are thereby obtained.

$$\eta = -\alpha_1 x_1 - \alpha_2 x_2 - ... + \beta \qquad (16)$$

In the formula, $\alpha$ indicates a coefficient, $\beta$ indicates an intercept, x indicates each miRNA, and $\eta$ indicates a logit value. Performing an inverse transformation of a logistic transformation on this logit value transformed it to a probability value of between 0 and 1, and a predictive probability was calculated. Furthermore, the difference of predictive probability was calculated for each Stage, and the fibrogenesis stage with the highest probability is configured to be a predictive value. The calculation method of the difference of predictive probability for each Stage is as follows.

- Difference of predictive probability of Stage 0 = 1 - Predictive probability of Stage 0|1
- Difference of predictive probability of Stage 1 = Predictive probability of Stage 0|1 - Predictive probability of Stage 1|2
- Difference of predictive probability of Stage 2 = Predictive probability of Stage 1|2 - Predictive probability of Stage 2|3
- Difference of predictive probability of Stage 3 = Predictive probability of Stage 2|3 - Predictive probability of Stage 3|4
- Difference of predictive probability of Stage 4 = Predictive probability of Stage 3|4

[Table 31]

Table 5-6 Regression coefficient of ordinal logistic regression model 2

| Variable | Regression coefficient |
|---|---|
| hsa-miR-614 | 36.96750762 |
| hsa-miR-3619-3p | 42.07030404 |
| hsa-miR-4646-5p | -47.25626817 |
| hsa-miR-6798-3p | 16.81916796 |
| hsa-miR-525-5p | 14.97674423 |
| hsa-miR-657 | -25.37300444 |
| hsa-miR-921 | 19.05116874 |
| hsa-miR-4444 | -10.6971939 |
| hsa-miR-4458 | 6.31801176 |
| hsa-miR-4746-3p | 23.37514001 |
| hsa-miR-1292-3p | -4.026335848 |
| hsa-miR-6133 | -27.42937096 |
| hsa-miR-6754-3p | 27.70913305 |
| hsa-miR-7975 | -31.60886779 |
| hsa-miR-365a-3p, hsa-miR-365b-3p | 3.999566697 |
| hsa-miR-451a | -5.951445744 |
| hsa-miR-6748-3p | -20.94808486 |
| hsa-miR-6892-3p | -10.71566305 |
| hsa-miR-8083 | -10.77927167 |
| hsa-miR-4451 | 7.781400953 |
| hsa-miR-4724-5p | 10.03889938 |
| hsa-miR-6801-3p | 19.44002139 |
| hsa-miR-6884-5p | 29.5235185 |
| hsa-miR-7156-3p | -8.438710102 |

(continued)

Table 5-6 Regression coefficient of ordinal logistic regression model 2

| Variable | Regression coefficient |
| --- | --- |
| hsa-miR-3059-3p | 32.18549237 |
| hsa-miR-19b-3p | -3.2882255 |
| hsa-miR-518e-3p | 40.83942009 |
| hsa-miR-646 | 16.7582579 |
| hsa-miR-298 | -27.58734069 |
| hsa-miR-1228-3p | -27.92289611 |
| hsa-miR-1234-3p | 3.648618806 |
| hsa-miR-2115-5p | 0.356618099 |
| hsa-miR-3142 | -28.17125021 |
| hsa-miR-3179 | -27.87479854 |
| hsa-miR-3190-5p | 22.54356098 |
| hsa-miR-3675-3p | -49.45068905 |
| hsa-miR-4441 | 15.81520137 |
| hsa-miR-4475 | 38.63649031 |
| hsa-miR-4787-3p | -24.16372259 |
| hsa-miR-6126 | -19.13709078 |
| hsa-miR-6718-5p | -37.74985513 |
| hsa-miR-6743-3p | -8.515187399 |
| hsa-miR-6894-3p | 2.53760787 |
| hsa-miR-7112-5p | 32.15072722 |
| hsa-miR-12116 | 71.9783582 |

[Table 32]

Table 5-7 Intercept value of ordinal logistic regression model 2

| Stage | Intercept |
| --- | --- |
| 0|1 | 693.1914718 |
| 1|2 | 736.5056963 |
| 2|3 | 779.093749 |
| 3|4 | 822.2316654 |

(5) Discrimination performance

(5-1) Accuracy rate

A contingency table was created with actual groups (0: normal, 1: stage 1, 2: stage 2, 3: stage 3, 4: stage 4) and predicted groups, and the concordance rates of the diagonal components thereof were calculated. Furthermore, the accuracy rates of each group from the contingency table were calculated. The accuracy rates when the aforementioned four regression models were used are shown below. It was obvious that sufficiently high accuracy rates are shown for any of the models.

[Table 33]

| stage / group | multinomial LASSO model | gaussian LASSO model | ordinal.intersect (ordinal logistic regression model 1) | ordinal.union (ordinal logistic regression model 2) |
|---|---|---|---|---|
| 1 Normal | 1.00 | 1.00 | 1.00 | 1.00 |
| 2 B1 | 0.90 | 1.00 | 0.60 | 1.00 |
| 3 B2 | 0.17 | 1.00 | 0.33 | 1.00 |
| 4 B3 | 1.00 | 0.80 | 0.73 | 1.00 |
| 5 B4 | 0.78 | 0.89 | 0.67 | 1.00 |

(5-2) Kappa coefficient

The weighted kappa coefficients when using the aforementioned four regression models are shown in the table below. It was shown that any of the models have a sufficiently high performance.

[Table 34]

| | model | kappa statistic |
|---|---|---|
| 1 | multinomial LASSO model | 0.91 |
| 2 | gaussian LASSO model | 0.99 |
| 3 | ordinal.intersect (ordinal logistic regression model 1) | 0.80 |
| 4 | ordinal.union (ordinal logistic regression model 2) | 1.00 |

The present inventors found out that by using the miRNA of NASH patients and performing a backwards analysis, a Brunt classification can be non-invasively predicted and assessed by using the miRNA. As mentioned above, according to the present invention, it was shown that by measuring the miRNA of NASH patients and substituting this in a prediction model formula, a Brunt classification can be predicted. Although the present inventors constructed four prediction models, highly accurate predictive results can be obtained by separately using these prediction models as required.

Industrial Applicability

Liver disease can be detected non-invasively or minimally invasively with high accuracy by using: the liver disease non-invasive biomarker; the kit, method and program using this biomarker; the data processing method for detecting liver disease with high accuracy using this liver disease non-invasive marker; and the data processing device for use in this data processing, of the present invention.

**Claims**

1. A detection kit for liver disease, comprising a nucleic acid capable of binding specifically to one or more polynucleotides, which are biomarkers, selected from the group consisting of miR-4454 (SEQ ID NO: 1), miR-3138 (SEQ ID NO: 2), miR-3679 (SEQ ID NO: 3), miR-4521 (SEQ ID NO: 4), miR-320A (SEQ ID NO: 5), miR-483 (SEQ ID NO: 6), miR-122 (SEQ ID NO: 7), miR-125B-1 (SEQ ID NO: 8), miR-125B-2 (SEQ ID NO: 9), miR-194-1 (SEQ ID NO: 10), miR-194-2 (SEQ ID NO: 11), miR-99a (SEQ ID NO: 12), miR-223 (SEQ ID NO: 13), miR-378a (SEQ ID NO: 14), miR-34a (SEQ ID NO: 15), miR-320c-1 (SEQ ID NO: 16), miR-320b-1 (SEQ ID NO: 17), miR-192 (SEQ ID NO: 18), miR-148a (SEQ ID NO: 19), miR-100 (SEQ ID NO: 20), miR-144 (SEQ ID NO: 21), miR-320b-2 (SEQ ID NO: 22), miR-451a (SEQ ID NO: 23), miR-548d-3p (SEQ ID NO: 24), miR-320b (SEQ ID NO: 25), miR-1470 (SEQ ID NO: 26), miR-4258 (SEQ ID NO: 27), miR-4673 (SEQ ID NO: 28), miR-4748 (SEQ ID NO: 29), miR-4787-3p (SEQ ID NO: 30), miR-204-3p (SEQ ID NO: 31), miR-6131 (SEQ ID NO: 32), miR-6752-3p (SEQ ID NO: 33), miR-4648 (SEQ ID NO: 34), miR-6126 (SEQ ID NO: 35), miR-1228-3p (SEQ ID NO: 36), miR-1231 (SEQ ID NO: 37), miR-4286 (SEQ ID NO: 38), miR-3935 (SEQ ID NO: 39), miR-2467-3p (SEQ ID NO: 40), miR-1185-2-3p (SEQ ID NO: 41), miR-6729-3p (SEQ ID NO: 42), miR-6798-3p (SEQ ID NO: 43), miR-379-5p (SEQ ID NO: 44), miR-323a-5p (SEQ ID NO: 45), miR-665 (SEQ ID NO: 46), miR-4279 (SEQ ID NO: 47), miR-3605-5p (SEQ ID NO: 48), miR-4684-3p (SEQ ID NO: 49), miR-365b-5p (SEQ ID NO: 50), miR-6782-5p (SEQ ID NO: 51), miR-6880-3p (SEQ ID NO: 52), miR-6887-3p (SEQ ID NO: 53), miR-4433b-5p (SEQ ID NO: 54), miR-10396a-3p (SEQ ID NO: 55), miR-373-5p (SEQ ID NO: 56), miR-4539 (SEQ ID NO: 57), miR-4687-3p (SEQ ID NO: 58), miR-4763-5p (SEQ ID NO: 59), miR-4482-3p (SEQ ID NO: 60), miR-6721-5p (SEQ ID NO: 61), miR-6812-3p (SEQ ID NO: 62), miR-6815-5p (SEQ ID NO: 63), miR-6871-5p (SEQ ID NO: 64), miR-7975 (SEQ ID NO: 65), miR-11181-3p (SEQ ID NO: 66), miR-2278 (SEQ ID NO: 67), miR-3192-5p (SEQ ID NO: 68), miR-4722-3p (SEQ ID NO: 69), miR-4750-3p (SEQ ID NO: 70), miR-6804-5p (SEQ ID NO: 71), miR-6828-5p (SEQ ID NO: 72), miR-614 (SEQ ID NO: 73), miR-320c (SEQ ID NO: 74), miR-2110 (SEQ ID NO: 75), miR-3177-3p (SEQ ID NO: 76), miR-4497 (SEQ ID NO: 77), miR-210-5p (SEQ ID NO: 78), miR-6778-5p (SEQ ID NO: 79), miR-6780a-5p (SEQ ID NO: 80), miR-6801-3p (SEQ ID NO: 81), miR-8059 (SEQ ID NO: 82), miR-657 (SEQ ID NO: 83), miR-921 (SEQ ID NO: 84), miR-4451 (SEQ ID NO: 85), miR-3059-3p (SEQ ID NO: 86), miR-3619-3p (SEQ ID NO: 87), miR-4646-5p (SEQ ID NO: 88), miR-525-5p (SEQ ID NO: 89), miR-4444 (SEQ ID NO: 90), miR-4458 (SEQ ID NO: 91), miR-4746-3p (SEQ ID NO: 92), miR-1292-3p (SEQ ID NO: 93), miR-6133 (SEQ ID NO: 94), miR-6754-3p (SEQ ID NO: 95), miR-365a-3p (SEQ ID NO: 96), miR-365b-3p (SEQ ID NO: 97), miR-6748-3p (SEQ ID NO: 98), miR-6892-3p (SEQ ID NO: 99), miR-8083 (SEQ ID NO: 100), miR-4724-5p (SEQ ID NO: 101), miR-6884-5p (SEQ ID NO: 102), miR-7156-3p (SEQ ID NO: 103), miR-19b-3p (SEQ ID NO: 104), miR-518e-3p (SEQ ID NO: 105), miR-646 (SEQ ID NO: 106), miR-298 (SEQ ID NO: 107), miR-1234-3p (SEQ ID NO: 108), miR-2115-5p (SEQ ID NO: 109), miR-3142 (SEQ ID NO: 110), miR-3179 (SEQ ID NO: 111), miR-3190-5p (SEQ ID NO: 112), miR-3675-3p (SEQ ID NO: 113), miR-4441 (SEQ ID NO: 114), miR-4475 (SEQ ID NO: 115), miR-6718-5p (SEQ ID NO: 116), miR-6743-3p (SEQ ID NO: 117), miR-6894-3p (SEQ ID NO: 118), miR-7112-5p (SEQ ID NO: 119), miR-12116 (SEQ ID NO: 120), miR-518d-3p (SEQ ID NO: 121), miR-145-5p (SEQ ID NO: 122), miR-6801-5p (SEQ ID NO: 123), miR-127-3p (SEQ ID NO: 124), and miR-4731-3p (SEQ ID NO: 125), or to a complementary strand of these polynucleotides.

2.  The detection kit for liver disease according to Claim 1, comprising a nucleic acid capable of binding specifically to one or more polynucleotides, which are biomarkers, selected from the group consisting of polynucleotides of SEQ ID NOs: 1 to 82, or to a complementary strand of these polynucleotides.

3.  The detection kit for liver disease according to Claim 2, comprising a nucleic acid capable of binding specifically to a polynucleotide, which is a biomarker, selected from the group consisting of polynucleotides of SEQ ID NOs: 1 to 4, or to a complementary strand of these polynucleotides.

4.  The detection kit according to Claim 3, wherein the kit further comprises at least one nucleic acid capable of binding specifically to a polynucleotide which is another biomarker.

5.  The detection kit according to Claim 4, wherein the other biomarker is one or more of the biomarkers selected from the group consisting of polynucleotides of SEQ ID NOs: 5 to 22.

6.  The detection kit for liver disease according to Claim 1, comprising a nucleic acid capable of binding specifically to one or more polynucleotides, which are biomarkers, selected from the group consisting of polynucleotides of SEQ ID NOs: 23, 30, 35, 36, 43, 65, 73, 81, 83 to 125, or to a complementary strand of these polynucleotides.

7.  The detection kit for liver disease according to Claim 1, wherein the liver disease is selected from the group consisting of hepatitis, hepatic fibrosis, fatty liver, hepatic cirrhosis and liver cancer.

8.  The detection kit for liver disease according to Claim 2, wherein the liver disease is a viral liver disease.

9.  The detection kit for liver disease according to Claim 6, wherein the liver disease is non-alcoholic steatohepatitis (NASH).

10. The detection kit for liver disease according to Claim 1, wherein the nucleic acid is selected from a polynucleotide or a fragment thereof listed in any of the (a) to (c) below:

    (a)

        (a-1) a polynucleotide or a fragment thereof containing 15 or more continuous bases, the polynucleotide consisting of a nucleotide sequence complementary to a nucleotide sequence of any of SEQ ID NOs: 1 to 125, or to a nucleotide sequence where u is t in this nucleotide sequence,

(a-2) a polynucleotide or a fragment thereof containing a deletion, substitution, addition or insertion of one or more bases in a nucleotide sequence of a polynucleotide or a fragment thereof in (a-1),
(a-3) a polynucleotide of (a-1) or (a-2) or a fragment thereof containing a modified nucleic acid and/or a modified nucleotide,

(b) a polynucleotide or a fragment thereof containing 15 or more continuous bases, the polynucleotide containing a nucleotide sequence complementary to a nucleotide sequence of any of SEQ ID NOs: 1 to 125, or to a nucleotide sequence where u is t in this nucleotide sequence, and
(c) a polynucleotide that hybridizes with a polynucleotide or a fragment thereof listed in any of the (c-1) to (c-4) below under a high stringent condition:

(c-1) a polynucleotide or a fragment thereof containing 15 or more continuous bases, the polynucleotide consisting of a nucleotide sequence of any of SEQ ID NOs: 1 to 125, or a nucleotide sequence where u is t in this nucleotide sequence,
(c-2) a polynucleotide or a fragment thereof containing a deletion, substitution, addition or insertion of one or more bases in a nucleotide sequence of a polynucleotide or a fragment thereof in (c-1),
(c-3) a polynucleotide of (c-1) or (c-2) or a fragment thereof containing a modified nucleic acid and/or a modified nucleotide,
(c-4) a polynucleotide or a fragment thereof containing 15 or more continuous bases, the polynucleotide containing a nucleotide sequence of any of SEQ ID NOs: 1 to 125, or a nucleotide sequence where u is t in this nucleotide sequence.

11. The detection kit for liver disease according to Claim 10, wherein the polynucleotide of any of the above (a) to (c) is selected from polynucleotides of any of SEQ ID NOs: 1 to 82.

12. The detection kit for liver disease according to Claim 10, wherein the polynucleotide of any of the above (a) to (c) is selected from polynucleotides of any of SEQ ID NOs: 23, 30, 35, 36, 43, 65, 73, 81, 83 to 125.

13. The detection kit for liver disease according to Claim 10, wherein the liver disease is selected from the group consisting of hepatitis, hepatic fibrosis, fatty liver, hepatic cirrhosis and liver cancer.

14. The detection kit for liver disease according to Claim 11, wherein the liver disease is a viral liver disease.

15. The detection kit for liver disease according to Claim 12, wherein the liver disease is non-alcoholic steatohepatitis (NASH).

16. A biomarker selected from the group consisting of polynucleotides of SEQ ID NOs: 1 to 125.

17. The biomarker according to Claim 16, selected from the group consisting of polynucleotides of SEQ ID NOs: 1 to 82.

18. The biomarker according to Claim 17, selected from the group consisting of polynucleotides of SEQ ID NOs: 1 to 4.

19. The biomarker according to Claim 16, selected from the group consisting of polynucleotides of SEQ ID NOs: 23, 30, 35, 36, 43, 65, 73, 81, 83 to 125.

20. The biomarker according to Claim 16, wherein the biomarker is obtainable from a biological sample selected from the group consisting of blood, serum, plasma, saliva, sweat, tears, faeces, urine and organ specimens.

21. The biomarker according to Claim 16, for use in diagnosing the presence or absence of liver disease or the risk thereof.

22. The biomarker according to Claim 16, for use in assessing the degree of progression of liver disease.

23. The biomarker according to Claim 16, wherein the liver disease is selected from the group consisting of hepatitis, hepatic fibrosis, fatty liver, hepatic cirrhosis and liver cancer.

24. The biomarker according to Claim 17, wherein the liver disease is a viral liver disease.

25. The biomarker according to Claim 19, wherein the liver disease is non-alcoholic steatohepatitis (NASH).

26. A method for assessing the presence or absence of liver disease or the risk or degree of progression thereof in a subject, and/or the degree of success of a procedure performed for treatment, the method comprising

measuring a level of a biomarker in a biological sample from the subject, and
comparing a measurement value of the biomarker with a reference value,
the biomarker being one or more of the biomarkers selected from the group consisting of polynucleotides of SEQ ID NOs: 1 to 125.

27. The method according to Claim 26, wherein the biomarker is one or more of the biomarkers selected from the group consisting of polynucleotides of SEQ ID NOs: 1 to 82.

28. The method according to Claim 27, wherein the biomarker is selected from the group consisting of polynucleotides of SEQ ID NOs: 1 to 4.

29. The method according to Claim 28, further comprising

measuring a level of an additional biomarker in a biological sample from a subject, and
comparing a measurement value of the additional biomarker with a reference value.

30. The method according to Claim 29, wherein the additional biomarker is one or more of the biomarkers selected from the group consisting of polynucleotides of SEQ ID NOs: 5 to 22.

31. The method according to Claim 26, wherein the biomarker is selected from the group consisting of polynucleotides of SEQ ID NOs: 23, 30, 35, 36, 43, 65, 73, 81 and 83 to 125.

32. The method according to Claim 26, wherein the biological sample is selected from the group consisting of blood, serum, plasma, saliva, sweat, tears, faeces, urine and organ specimens.

33. The method according to Claim 26, wherein the liver disease is selected from the group consisting of hepatitis, hepatic fibrosis, fatty liver, hepatic cirrhosis and liver cancer.

34. The method according to Claim 27, wherein the liver disease is a viral liver disease.

35. The method according to Claim 31, wherein the liver disease is non-alcoholic steatohepatitis (NASH).

36. A method for screening a candidate compound of a therapeutic drug for liver disease in a subject, comprising

measuring a level of a biomarker in a biological sample from the subject administered with a candidate compound of a therapeutic drug for liver disease, and
comparing a measurement value of the biomarker with a reference value,
the biomarker being one or more of the biomarkers selected from the group consisting of polynucleotides of SEQ ID NOs: 1 to 125.

37. The method according to Claim 36, wherein the biomarker is one or more of the biomarkers selected from the group consisting of polynucleotides of SEQ ID NOs: 1 to 82.

38. The method according to Claim 37, wherein the biomarker is selected from the group consisting of polynucleotides of SEQ ID NOs: 1 to 4.

39. The method according to Claim 38, further comprising

measuring a level of an additional biomarker in a biological sample from a subject, and
comparing a measurement value of the additional biomarker with a reference value.

40. The method according to Claim 39, wherein the additional biomarker is one or more of the biomarkers selected from the group consisting of polynucleotides of SEQ ID NOs: 5 to 22.

41. The method according to Claim 36, wherein the biomarker is selected from the group consisting of polynucleotides of SEQ ID NOs: 23, 30, 35, 36, 43, 65, 73, 81 and 83 to 125.

42. The method according to Claim 36, wherein the biological sample is selected from the group consisting of blood, serum, plasma, saliva, sweat, tears, faeces, urine and organ specimens.

43. The method according to Claim 36, wherein the liver disease is selected from the group consisting of hepatitis, hepatic fibrosis, fatty liver, hepatic cirrhosis and liver cancer.

44. The method according to Claim 37, wherein the liver disease is a viral liver disease.

45. The method according to Claim 41, wherein the liver disease is non-alcoholic steatohepatitis (NASH).

46. A method for treating liver disease in a subject, comprising

measuring a level of a biomarker in a biological sample from the subject, and
comparing a measurement value of the biomarker with a reference value,
the biomarker being one or more of the biomarkers selected from the group consisting of polynucleotides of SEQ ID NOs: 1 to 125.

47. The method according to Claim 46, wherein the biomarker is one or more of the biomarkers selected from the group consisting of polynucleotide of SEQ ID NOs: 1 to 82.

48. The method according to Claim 47, wherein the biomarker is selected from the group consisting of polynucleotides of SEQ ID NOs: 1 to 4.

49. The method according to Claim 48, further comprising

measuring a level of an additional biomarker in a biological sample from a subject, and
comparing a measurement value of the additional biomarker with a reference value.

50. The method according to Claim 49, wherein the additional biomarker is one or more of the biomarkers selected from the group consisting of polynucleotides of SEQ ID NOs: 5 to 22.

51. The method according to Claim 46, wherein the biomarker is selected from the group consisting of polynucleotides of SEQ ID NO: 23, 30, 35, 36, 43, 65, 73, 81 and 83 to 125.

52. The method according to Claim 46, wherein the biological sample is selected from the group consisting of blood, serum, plasma, saliva, sweat, tears, faeces, urine and organ specimens.

53. The method according to Claim 46, wherein the liver disease is selected from the group consisting of hepatitis, hepatic fibrosis, fatty liver, hepatic cirrhosis and liver cancer.

54. The method according to Claim 47, wherein the liver disease is a viral liver disease.

55. The method according to Claim 51, wherein the liver disease is non-alcoholic steatohepatitis (NASH).

56. A method for detecting liver disease with high accuracy, comprising:

measuring a level of a biomarker 1 in a biological sample from a subject;
comparing a measurement value of the biomarker 1 with a reference value to obtain a parameter 1;
measuring a level of at least one additional biomarker N (where N is an integer of 2 or more) in the biological sample from the subject;
comparing a measurement value of the additional biomarker N with a reference value to obtain a parameter N; and
obtaining a result in the form of one set of at least two parameters made up of a combination of the parameter 1 and one or more of the parameters N,
the result in the form of one set of at least two parameters indicating the presence or absence of liver disease or the risk or degree of progression thereof in the subject, and/or the degree of success of a procedure performed for treatment.

57. The method according to Claim 56, wherein the result in the form of one set of at least two parameters is evaluated using a computer program.

58. The method according to Claim 56, wherein the biomarker 1 and the at least one additional biomarker are selected from the group consisting of polynucleotides of SEQ ID NOs: 1 to 125.

59. The method according to Claim 58, wherein the biomarker 1 and the at least one additional biomarker are one or more of the biomarkers selected from the group consisting of polynucleotides of SEQ ID NOs: 1 to 82.

60. The method according to Claim 58, wherein the biomarker 1 and the at least one additional biomarker are selected from the group consisting of polynucleotides of SEQ ID NOs: 23, 30, 35, 36, 43, 65, 73, 81 and 83 to 125.

61. The method according to Claim 56, wherein the biological sample is selected from the group consisting of blood, serum, plasma, saliva, sweat, tears, faeces, urine and organ specimens.

62. The method according to Claim 56, wherein the liver disease is selected from the group consisting of hepatitis, hepatic fibrosis, fatty liver, hepatic cirrhosis and liver cancer.

63. The method according to Claim 59, wherein the liver disease is a viral liver disease.

64. The method according to Claim 60, wherein the liver disease is non-alcoholic steatohepatitis (NASH).

65. A diagnostic support system for liver disease, comprising

a unit for measuring a level of one or more biomarkers in a biological sample from a subject;
a unit for comparing a measurement value of the one or more biomarkers with respective reference values thereof to obtain a plurality of parameters; and
a unit for calculating, from the plurality of parameters, the presence or absence of liver disease or the risk or degree of progression thereof in the subject, and/or the degree of success of a procedure performed for treatment.

66. The diagnostic support system for liver disease according to Claim 65, wherein the one or more biomarkers are selected from the group consisting of polynucleotides of SEQ ID NOs: 1 to 125.

67. The diagnostic support system for liver disease according to Claim 66, wherein the biomarker is one or more of the biomarkers selected from the group consisting of polynucleotides of SEQ ID NOs: 1 to 82.

68. The diagnostic support system for liver disease according to Claim 66, wherein the biomarker is selected from the group consisting of polynucleotides of SEQ ID NOs: 23, 30, 35, 36, 43, 65, 73, 81, 83 to 125.

69. The diagnostic support system for liver disease according to Claim 65, wherein the biological sample is selected from the group consisting of blood, serum, plasma, saliva, sweat, tears, faeces, urine and organ specimens.

70. The diagnostic support system for liver disease according to Claim 65, wherein the liver disease is selected from the group consisting of hepatitis, hepatic fibrosis, fatty liver, hepatic cirrhosis and liver cancer.

71. The diagnostic support system for liver disease according to Claim 67, wherein the liver disease is a viral liver disease.

72. The diagnostic support system for liver disease according to Claim 68, wherein the liver disease is non-alcoholic steatohepatitis (NASH).

73. A method for performing data processing for staging the pathology of liver disease of a subject, the method executed by a computer, the computer comprising:

a computer processor; and
a computer readable medium storing software that gives instructions for staging the pathology of liver disease of the subject,

the method comprising:

comparing a measurement value of one or more biomarkers in a biological sample obtained from the subject with respective reference values thereof to obtain data of a plurality of parameters; and

outputting an assessment result of a pathology of liver disease of the subject as information by the software that gives instructions for staging the pathology of liver disease of the subject, the assessment performed by the computer that executes the instructions using the computer processor to process the data of the plurality of parameters, and

the staging comprising one or more selected from the group consisting of a hepatic fibrogenesis stage, Child-Pugh classification, MELD score, MELD Na score, PELD score, ALBI score, mALBI (modified ALBI) score, FibroScan score, new Inuyama classification, new European classification, and Brunt classification.

74. The method according to Claim 73, wherein the assessment result is a combination of results respectively assessed by processing the data of the plurality of parameters.

75. The method according to Claim 73, wherein the one or more biomarkers are selected from the group consisting of polynucleotides of SEQ ID NOs: 1 to 125.

76. The method according to Claim 75, wherein the biomarker is one or more of the biomarkers selected from the group consisting of polynucleotides of SEQ ID NOs: 1 to 82.

77. The method according to Claim 75, wherein the biomarker is selected from the group consisting of polynucleotides of SEQ ID NOs: 23, 30, 35, 36, 43, 65, 73, 81 and 83 to 125.

78. The method according to Claim 73, wherein the biological sample is selected from the group consisting of blood, serum, plasma, saliva, sweat, tears, faeces, urine and organ specimens.

79. The method according to Claim 73, wherein the liver disease is selected from the group consisting of hepatitis, hepatic fibrosis, fatty liver, hepatic cirrhosis and liver cancer.

80. The method according to Claim 76, wherein the liver disease is a viral liver disease.

81. The method according to Claim 77, wherein the liver disease is non-alcoholic steatohepatitis (NASH).

82. The method according to Claim 73, wherein the subject is a mammalian subject.

83. The method according to Claim 82, wherein the subject is a human subject.

84. A program for executing an assessment of the presence or absence of liver disease or the risk or degree of progression thereof in a subject and/or an assessment of the degree of success of a procedure performed for a treatment on a computer, the program executing:

a measurement value acquisition step of respectively acquiring a measurement value of a level of one or more biomarkers obtained from a biological sample from the subject;

a parameter data acquisition step of acquiring data of a plurality of parameters by comparing a measurement value of the one or more biomarkers with respective reference values; and

an assessment step of assessing, based on the data of the plurality of parameters, the presence or absence of liver disease or the risk or degree of progression thereof in the subject, and/or the degree of success of a procedure performed for the treatment,

the biomarker being selected from the group consisting of polynucleotides of SEQ ID NOs: 1 to 125.

85. A data processing device to perform data processing for detecting liver disease, comprising:

a missing value supplement unit to obtain missing value supplemental data by supplementing a missing value in miRNA expression level data;

a logarithmic transformation unit to obtain logarithmic transformation data by logarithmically transforming the missing value supplemental data;

a regression transformation unit to obtain regression transformation data by substituting the logarithmic transformation data in a regression model and executing a regression transformation;
a training data acquisition unit to acquire training data; and
a learnt model creation unit to create a learnt model using the regression transformation data as the training data, the miRNA being one or more selected from the group consisting of polynucleotides of SEQ ID NOs: 1 to 125.

86. The data processing device to perform data processing for detecting liver disease according to Claim 85, wherein the regression transformation comprises:

multiplying a regression coefficient;
obtaining a logit value showing a predictive result of a fibrogenesis stage by adding an intercept value at each fibrogenesis stage;
calculating a predictive probability of each fibrogenesis stage by performing an inverse transformation of a logistic transformation on the logit value and transforming it to a probability value of between 0 and 1; and
configuring the fibrogenesis stage with the highest probability amongst the predictive probabilities of each fibrogenesis stage, to be a predictive value.

87. The data processing device to perform data processing for detecting liver disease according to Claim 86, wherein the regression model is selected from the group consisting of the multinominal LASSO model, gaussian LASSO (Least Absolute Shrinkage and Selection Operator) model, and ordinal logistic regression model.

88. The data processing device to perform data processing for detecting liver disease according to Claim 85, wherein the training data are the training data for showing a relationship between a blood miRNA concentration for learning, and a fibrogenesis stage corresponding to the blood miRNA concentration for learning.

89. The data processing device to perform data processing for detecting liver disease according to Claim 85, wherein the data processing for detecting liver disease further comprises a training data acquisition step for acquiring training data, and a learning data creation step for creating learning data utilizing the training data, and wherein the training data are training data for showing a relationship between a blood miRNA concentration for learning and a fibrogenesis stage corresponding to the blood miRNA concentration for learning.

90. The data processing device to perform data processing for detecting liver disease according to Claim 85, wherein the data processing for detecting liver disease further comprises a pre-processing step for obtaining data for analysis, and the pre-processing step comprises:

obtaining a low expression miRNA list by listing low expression miRNA;
obtaining a missing value supplemental data by supplementing a missing value in the data of the miRNA list;
obtaining data for transformation by excluding low expression miRNA included in the low expression miRNA list from an analysis target; and
logarithmically transforming the data for transformation.

91. The data processing device to perform data processing for detecting liver disease according to Claim 85, wherein the miRNA is one or more selected from the group consisting of polynucleotides of SEQ ID NOs: 1 to 82.

92. The data processing device to perform data processing for detecting liver disease according to Claim 85, wherein the miRNA is selected from the group consisting of polynucleotides of SEQ ID NOs: 23, 30, 35, 36, 43, 65, 73, 81, 83 to 125.

93. The data processing device to perform data processing for detecting liver disease according to Claim 85, wherein the biological sample is selected from the group consisting of blood, serum, plasma, saliva, sweat, tears, faeces, urine and organ specimens.

94. The data processing device to perform data processing for detecting liver disease according to Claim 85, wherein the liver disease is selected from the group consisting of hepatitis, hepatic fibrosis, fatty liver, hepatic cirrhosis and liver cancer.

95. The data processing device to perform data processing for detecting liver disease according to Claim 91, wherein the liver disease is a viral liver disease.

**96.** The data processing device to perform data processing for detecting liver disease according to Claim 92, wherein the liver disease is non-alcoholic steatohepatitis (NASH).

# Fig. 1

# Fig. 2

FPKM  miR125B1+
80000
60000
40000
20000
0
        |Normal|    0    1    4
                Fibrogenesis

FPKM  MIR320A
150000
100000
50000
0
        |Normal|    0    1    4
                Fibrogenesis

FPKM  miR223-3P
300
    250
200
    150
100
    500
        0
        |Normal|    0    1    4
                Fibrogenesis

FPKM  MIR483
250000
200000
150000
100000
50000
0
        |Normal|    0    1    4
                Fibrogenesis

FPKM  miR-4454
5000
4000
3000
2000
1000
0
        |Normal|    0    1    4
                Fibrogenesis

# Fig. 3

「Child Pugh Score 0」vs.
「Child Pugh Score 6 or more」

gene-CPS-0-CPS-over-0_DESeq2

The strongly bolded line shows
changes also for fibrogenesis

- NA
- padj > 0.05
- padj <=0.05, FC >=2

# Fig. 4

# Fig. 5

```
                    ┌─────────────┐
                    │    START    │
                    └──────┬──────┘
                           │
                           ▼
        ┌──────────────────────────────────────┐
        │  Acquisition of miRNA expression level │
        │   amount data of Toray Industries Inc. │
        │            "3D-Gene®"                  │
        └──────────────────┬───────────────────┘
                           │
                           ▼
                 ┌──────────────────┐
                 │  Supplementation │
                 │  of missing value│
                 └─────────┬────────┘
                           │
                           ▼
                 ┌──────────────────┐
                 │   Logarithmic    │
                 │  transformation  │
                 └─────────┬────────┘
                           │
```

| Substitution in multinomial LASSO model | Substitution in gaussian LASSO model | Substitution in ordinal logistic regression model 1 | Substitution in ordinal logistic regression model 2 |
|---|---|---|---|
| Prediction of fibrogenesis stage | Prediction of fibrogenesis stage | Prediction of fibrogenesis stage | Prediction of fibrogenesis stage |

```
                    ┌─────────────┐
                    │     END     │
                    └─────────────┘
```

# Fig. 6

# Fig. 7

```
                    ┌──────────────┐
                    │    START     │
                    └──────┬───────┘
                           │
                           ▼
        ┌──────────────────────────────────────┐
        │ Acquisition of miRNA expression level │
        │ amount data of Toray Industries Inc.  │
        │   "3D-Gene®" of a NASH patient        │
        └──────────────────┬───────────────────┘
                           │
                           ▼
                 ┌──────────────────┐
                 │  Supplementation │
                 │  of missing value│
                 └────────┬─────────┘
                          │
                          ▼
                 ┌──────────────────┐
                 │    Logarithmic   │
                 │  transformation  │
                 └────────┬─────────┘
                          │
```

| Substitution in multinomial LASSO model | Substitution in gaussian LASSO model | Substitution in ordinal logistic regression model 1 | Substitution in ordinal logistic regression model 2 |
|---|---|---|---|
| Prediction of Brunt classification | Prediction of Brunt classification | Prediction of Brunt classification | Prediction of Brunt classification |

```
                    ┌──────────────┐
                    │     END      │
                    └──────────────┘
```

## INTERNATIONAL SEARCH REPORT

| International application No. |
| --- |
| PCT/JP2023/029768 |

### A. CLASSIFICATION OF SUBJECT MATTER

Int.Cl. C12N 15/113(2010. 01)i; C12Q 1/6883(2018. 01)i; G01N 33/53(2006. 01)i

FI: C12N15/113 Z ZNA; G01N33/53 M; C12Q1/6883 Z

According to International Patent Classification (IPC) or to both national classification and IPC

### B. FIELDS SEARCHED

Minimum documentation searched (classification system followed by classification symbols)

Int.Cl. C12N15/113; C12Q1/6883; G01N33/53

Documentation searched other than minimum documentation to the extent that such documents are included in the fields searched

| | |
| --- | --- |
| Published examined utility model applications of Japan | 1922-1996 |
| Published unexamined utility model applications of Japan | 1971-2023 |
| Registered utility model specifications of Japan | 1996-2023 |
| Published registered utility model applications of Japan | 1994-2023 |

Electronic data base consulted during the international search (name of data base and, where practicable, search terms used)

JSTPlus/JMEDPlus/JST7580(JDreamIII)

### C. DOCUMENTS CONSIDERED TO BE RELEVANT

| Category* | Citation of document, with indication, where appropriate, of the relevant passages | Relevant to claim No. |
| --- | --- | --- |
| X | WO 2013/125691 A1 (SUMITOMO BAKELITE CO., LTD.) 29 August 2013 (2013-08-29) claims, examples & JP 2013-198483 A claims, examples & US 2015/0031574 A1 claims, examples & EP 2818549 A1 | 1,2,5-17, 19-27, 29-37, 39-47, 49-72 |
| Y | | 1-96 |

☒ Further documents are listed in the continuation of Box C. ☐ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 04 October 2023 | 17 October 2023 |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

| | International application No. |
|---|---|
| | PCT/JP2023/029768 |

C (Continuation).　DOCUMENTS CONSIDERED TO BE RELEVANT

| Category* | Citation of document, with indication, where appropriate, of the relevant passages | Relevant to claim No. |
|---|---|---|
| X | JP 2011-217617 A (KYOTO UNIV.) 04 November 2011 (2011-11-04)<br>claims, examples, paragraph [0032], table 1<br>(Family: none) | 1, 2, 5, 7-11, 13-17, 20-27, 29, 30, 32-37, 39, 40, 42-47, 49, 50, 52-59, 61-67, 69-72 |
| Y | | 1-96 |
| X | WO 2016/196945 A1 (REGULUS THERAPEUTICS INC.) 08 December 2016 (2016-12-08)<br>claims, examples, paragraph [0062]<br>& JP 2018-518169 A<br>claims, examples, paragraph [0062]<br>& US 2018/0155787 A1<br>& EP 3303629 A1 | 1, 2, 5, 7, 9-11, 13, 15-17, 20-23, 25-27, 29, 30, 32, 33, 35-37, 39, 40, 42, 43, 45-47, 49, 50, 52, 53, 55-59, 61, 62, 64-67, 69, 70, 72 |
| Y | | 1-96 |
| X | WO 2017/046181 A1 (GENFIT) 23 March 2017 (2017-03-23)<br>claims, examples<br>& JP 2018-534542 A<br>claims, examples<br>& US 2018/0265924 A1<br>& EP 3350341 A1<br>& KR 10-2018-0049098 A<br>& CN 108138232 A | 1, 2, 5, 7, 9-11, 13, 15-17, 20-23, 25-27, 29, 30, 32, 33, 35-37, 39, 40, 42, 43, 45-47, 49, 50, 52, 53, 55-59, 61, 62, 64-67, 69, 70, 72 |
| Y | | 1-96 |
| A | 小森敦正 外, C型肝炎ウイルス駆除後に発現量が変化する血清miRNAの探索, 肝臓, vol. 59, suppl. 1, 2018, A432, O-245, non-official translation (KOMORI, Atsumasa et al. Search for serum miRNAs whose expression levels change after eradication of hepatitis C virus. Liver.)<br>entire text | 1-96 |

Form PCT/ISA/210 (continuation of second sheet) (January 2015)

## INTERNATIONAL SEARCH REPORT

International application No.

PCT/JP2023/029768

**Box No.I    Nucleotide and/or amino acid sequence(s) (Continuation of item 1.c of the first sheet)**

1.    With regard to any nucleotide and/or amino acid sequence disclosed in the international application, the international search was carried out on the basis of a sequence listing:

   a.   forming part of the international application as filed:

   ☒    in the form of an Annex C/ST.25 text file.

   ☐    on paper or in the form of an image file.

   b.   furnished together with the international application under PCT Rule 13*ter*.1(a) for the purpose of international search only in the form of an Annex C/ST.25 text file.

   c.   furnished subsequent to the international filing date for the purposes of international search only:

   ☐    in the form of an Annex C/ST.25 text file (Rule 13*ter*.1(a)).

   ☐    on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:
"In the form of an Annex C/ST.25 text file" above should be understood as "in ST.26 format".

Form PCT/ISA/210 (continuation of first sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **H NISHIKAWA**. Serum Wisteria floribunda agglutinin-positive Mac-2-binding protein for patients with chronic hepatitis B and C: a comparative study. *J Viral Hepat.*, 31 July 2016, vol. 23 (12), 977-984

- **THERY C.** Curr. Protoc. Cell Biol.. 2006